# EUROPÄISCHE PATENTSCHRIFT

(11) **EP 0 736 001 B1**
(45) Veröffentlichungstag und Bekanntmachung des Hinweises auf die Patenterteilung: **09.06.1999**
(21) Anmeldenummer: 95904446.2
(22) Anmeldetag: 09.12.1994
(51) Int. Cl.: C07C 251/52, C07C 235/34, C07C 255/64, C07C 251/60, A01N 37/50, A01N 37/36, A01N 43/00, C07D 417/04, C07D 213/54, C07D 333/24, C07D 213/53, C07D 239/34

(54) **ARYLESSIGSÄUREDERIVATE UND IHRE VERWENDUNG ALS FUNGIZIDE**
ARYL ACETIC ACID DERIVATIVES AND THEIR USE AS FUNGICIDES
DERIVES DE L'ACIDE ARYLACETIQUE ET LEUR UTILISATION COMME FONGICIDES

(30) Priorität: 22.12.1993 DE 4343829; 14.07.1994 DE 4424788
(43) Veröffentlichungstag der Anmeldung: 09.10.1996
(73) Patentinhaber: BAYER AG, 51368 Leverkusen (DE)
(72) Erfinder: KLEEFELD, Gerd, D-41468 Neuss-Üdesheim (DE); TIEMANN, Ralf, D-51375 Leverkusen (DE); GERDES, Peter, D-52080 Aachen (DE); HEINEMANN, Ulrich, D-42799 Leichlingen (DE); DEHNE, Heinz-Wilhelm, D-53125 Bonn (DE); DUTZMANN, Stefan, D-40721 Hilden (DE); STENZEL, Klaus, D-40595 Düsseldorf (DE)
(86) Internationale Anmeldenummer: EP9404103
(87) Internationale Veröffentlichungsnummer: WO9517376

(56) Entgegenhaltungen:
- EP-A- 0 400 417

## Beschreibung

Die Erfindung betrifft neue Arylessigsäurederivate, Verfahren zu ihrer Herstellung und ihre Verwendung als Fungizide.

Aus der EP-400 417 sind 2-Phenoxymethyl-phenylglyoxylsäuremethyl ester Oximether zur Verwendung als Fungizid bekannt.

Es wurden neue Arylessigsäurederivate der allgemeinen Formel (I) gefunden, 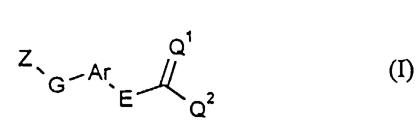 in welcher
- Ar: für jeweils gegebenenfalls substituiertes Arylen oder Heteroarylen steht,
- E: für eine 1-Alken-1,1-diyl-Gruppierung oder für eine 2-Aza-1-alken-1,1-diyl-Gruppierung steht, die jeweils in 2-Position eine Halogenalkoxy-Gruppe enthalten,
- G: für Sauerstoff, für eine direkte Bindung, für jeweils gegebenenfalls durch Halogen, Hydroxy, Alkyl, Halogenalkyl oder Cycloalkyl substituiertes Alkandiyl, Alkendiyl, Alkindiyl oder eine der nachstehenden Gruppierungen
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R¹)=N-O-, -C(R¹)=N-O-CH₂-, -N(R²)-, -CQ-N(R²)-, -N(R²)-CQ-, -Q-CQ-N(R²)-, -N=C(R¹)-Q-CH₂-, -CH₂-O-N=C(R¹)-, -N(R²)-CQ-Q-, -CQ-N(R²)-CQ-Q- oder -N(R²)-CQ-Q-CH₂- steht,
wobei
n für die Zahlen 0, 1 oder 2 steht,
Q für Sauerstoff oder Schwefel steht,
R¹ für Wasserstoff, Cyano oder jeweils gegebenenfalls substituiertes Alkyl, Alkoxy, Alkylthio, Alkylamino, Dialkylamino oder Cycloalkyl steht, und
R² für Wasserstoff, Hydroxy, Cyano oder jeweils gegebenenfalls substituiertes Alkyl, Alkoxy oder Cycloalkyl steht,
- Q¹: für Sauerstoff oder Schwefel steht,
- Q²: für Hydroxy, Mercapto, Amino oder für jeweils gegebenenfalls substituiertes Alkoxy, Alkylthio, Alkylamino, Alkoxyamino, Dialkylamino oder N-Alkoxy-N-alkyl-amino steht, sowie
- Z: für jeweils gegebenenfalls substituiertes Alkyl, Alkenyl, Alkinyl, Cycloalkyl, Aryl oder Heteroaryl steht.

Weiter wurde gefunden, daß man die neuen Arylessigsäurederivate der allgemeinen Formel (I) erhält, wenn man
(a) Arylglyoxylsäurederivate der allgemeinen Formel (II) 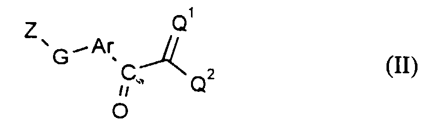 in welcher
   Ar, G, Q¹, Q² und Z die oben angegebene Bedeutung haben,
   mit O-Halogenalkyl-hydroxylaminen der allgemeinen Formel (III)

   H₂N-O-R (III)

   in welcher
   - R: für Halogenalkyl steht,

   - oder mit Hydrogenhalogenid-Addukten von Verbindungen der Formel (III) -
   gegebenenfalls in Gegenwart eines Reaktionshilfsmittels und gegebenenfalls in Gegenwart eines Verdünnungsmittels umsetzt, oder wenn man
(b) 2-Hydroximino-arylessigsäurederivate der allgemeinen Formel (IV) 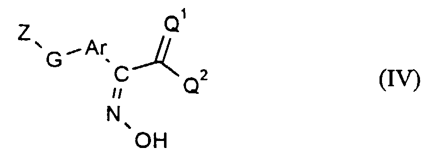 in welcher
   Ar, G, Q¹, Q² und Z die oben angegebene Bedeutung haben,
   mit Halogenalkanen der allgemeinen Formel (V)

   X-R (V)

   in welcher
   - R: für Halogenalkyl steht und
   - X: für Halogen steht,
   gegebenenfalls in Gegenwart eines Reaktionshilfsmittels und gegebenenfalls in Gegenwart eines Verdünnungsmittels umsetzt.

Die Verbindungen der Formel (I), bei denen E für eine 1-Alken-1,1-diyl-Gruppierung steht, die in 2-Position eine Halogenalkoxy-Gruppe enthält, können analog zu Verfahren (b) aus Verbindungen der Formel (I), bei denen E für eine entsprechende 1-Alken-1,1-diyl-Gruppierung steht, die in 2-Position eine Hydroxy-Gruppe enthält, hergestellt werden.

Schließlich wurde gefunden, daß die neuen Arylessigsäurederivate der allgemeinen Formel (I) sehr starke fungizide Wirksamkeit zeigen.

Die erfindungsgemäßen Verbindungen können gegebenenfalls als Mischungen verschiedener möglicher isomerer Formen, insbesondere von E- und Z-Isomeren, vorliegen. Es werden sowohl die E- als auch die Z-Isomeren wie auch beliebige Mischungen dieser Isomeren beansprucht.

Gegenstand der Erfindung sind vorzugsweise Verbindungen der Formel (I), in welcher
- Ar: für jeweils gegebenenfalls substituiertes Phenylen oder Naphthylen oder für Heteroarylen mit 5 oder 6 Ringgliedern, von denen mindestens eines für Sauerstoff, Schwefel oder Stickstoff steht und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
- E: für eine der nachstehenden Gruppierungen steht 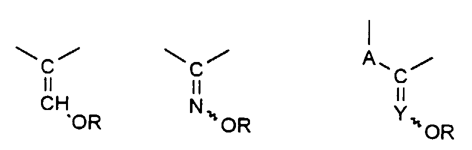 worin
R für Halogenalkyl mit 1 bis 6 Kohlenstoffatomen steht,
A für Sauerstoff, Schwefel, Imino (NH) oder Alkylimino (N-Alkyl - letzteres mit 1 bis 4 Kohlenstoffatomen) steht, und
Y für Stickstoff oder eine CH-Gruppierung steht,
- G: für Sauerstoff für eine direkte Bindung, für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Alkindiyl mit jeweils bis zu 4 Kohlenstoffatomen oder eine der nachstehenden Gruppierungen
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R¹)=N-O-, -C(R¹)=N-O-CH₂-, -N(R²)-, -CQ-N(R²)-, -N(R²)-CQ-, -Q-CQ-N(R²)-, -N=C(R¹)-Q-CH₂-, -CH₂-O-N=C(R¹)-, -N(R²)-CQ-Q-, -CQ-N(R²)-CQ-Q- oder -N(R²)-CQ-Q-CH₂- steht,
wobei
n für die Zahlen 0, 1 oder 2 steht,
Q für Sauerstoff oder Schwefel steht,
R¹ für Wasserstoff, Cyano, für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht, und
R² für Wasserstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht,
- Q¹: für Sauerstoff oder Schwefel steht,
- Q²: für Hydroxy, Mercapto, Amino oder für jeweils gegebenenfalls durch Halogen oder C₁-C₄-Alkoxy substituiertes C₁-C₆-Alkoxy, C₁-C₆-Alkylthio, C₁-C₆-Alkylamino, C₁-C₆-Alkoxyamino, Di-(C₁-C₄-alkyl)-amino oder N-(C₁-C₄-Alkoxy)-N-(C₁-C₄-alkyl)-amino steht, sowie
- Z: für gegebenenfalls durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sind) substituiertes Alkyl mit 1 bis 8 Kohlenstoffatomen, für jeweils gegebenenfalls durch Halogen substituiertes Alkenyl oder Alkinyl mit jeweils bis zu 8 Kohlenstoffatomen, für gegebenenfalls durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxycarbonyl substiuiertes Cycloalkyl mit 3 bis 6 Kohlenstoffatomen, für jeweils gegebenenfalls substituiertes Phenyl, Naphthyl oder Heteroaryl mit 5 oder 6 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, Cycloalkyl mit 3 bis 6 Kohlenstoffatomen, gegebenenfalls subsituiertes Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -, sowie jeweils gegebenenfalls im Phenylteil einfach oder mehrfach, gleich oder verschieden durch Halogen, Cyano und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen substituiertes Phenyl, Phenoxy, Phenoxymethyl, Benzyl, Benzyloxy, Benzylthio, Phenylethyl oder Phenylethyloxy.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I), in welcher
- Ar: für jeweils gegebenenfalls substituiertes ortho-, meta- oder para-Phenylen, für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl (insbesondere Pyridin-2,3-diyl), Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind: Fluor, Chlor, Cyano, Methyl, Ethyl, Trifluormethyl, Methoxy, Ethoxy, Methylthio, Methylsulfinyl oder Methylsulfonyl,
- E: für eine der nachstehenden Gruppierungen steht 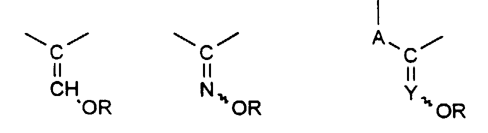 worin
R für jeweils durch Fluor und/oder Chlor substituiertes Methyl, Ethyl, n- oder i-Propyl, n-, i-, s- oder t-Butyl steht,
A für Sauerstoff, Schwefel, Imino (NH) oder Methylimino (N-Methyl) steht, und
Y für Stickstoff oder eine CH-Gruppierung steht,
- G: für Sauerstoff für eine direkte Bindung, für jeweils gegebenenfalls durch Fluor, Chlor, Hydroxy, Methyl, Ethyl, n- oder i-Propyl, Trifluormethyl, Cyclopropyl, Cyclobutyl, Cyclopentyl oder Cyclohexyl substituiertes Methylen, Dimethylen (Ethan-1,2-diyl), Ethen-1,2-diyl, Ethin-1,2-diyl oder eine der nachstehenden Gruppierungen
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R¹)=N-O-, -C(R¹)=N-O-CH₂-, -N(R²)-, -CQ-N(R²)-, -N(R²)-CQ-, -Q-CQ-N(R²)-, -N=C(R¹)-Q-CH₂-, -CH₂-O-N=C(R¹)-, -N(R²)-CQ-Q-, -CQ-N(R²)-CQ-Q- oder -N(R²)-CQ-Q-CH₂- steht,
wobei
n für die Zahlen 0, 1 oder 2 steht,
Q für Sauerstoff oder Schwefel steht,
R¹ für Wasserstoff, Cyano, für jeweils gegebenenfalls durch Fluor, Chlor, Cyano, Methoxy oder Ethoxy substituiertes Methyl, Ethyl, n- oder i-Propyl, n-, i- oder s-Butyl, Methoxy, Ethoxy, Propoxy, Butoxy, Methylthio, Ethylthio, Propylthio, Butylthio, Methylamino, Ethylamino, Propylamino, Dimethylamino oder Diethylamino oder für jeweils gegebenenfalls durch Fluor, Chlor, Cyano, Carboxy, Methyl, Ethyl, n- oder i-Propyl, Methoxycarbonyl oder Ethoxy-carbonyl substituiertes Cyclopropyl, Cyclobutyl, Cyclopentyl oder Cyclohexyl steht, und
R² für Wasserstoff, Hydroxy, Cyano oder für jeweils gegebenenfalls durch Fluor, Chlor, Cyano, Methoxy oder Ethoxy substituiertes Methyl, Ethyl, n- oder i-Propyl, n-, i-, s- oder t-Butyl oder für jeweils gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht,
- Q¹: für Sauerstoff oder Schwefel steht,
- Q²: für Hydroxy, Mercapto, Amino oder für jeweils gegebenenfalls durch Fluor, Chlor, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i- s- oder t-Butylamino, Methoxyamino, Ethoxyamino, n- oder i-Propoxyamino, n-, i-, s- oder t-Butoxyamino, Dimethylamino, Diethylamino, Dipropylamino, Dibutylamino oder N-Methoxy-N-methyl-amino steht, sowie
- Z: für jeweils gegebenenfalls durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl, für jeweils gegebenenfalls durch Fluor, Chlor oder Brom substituiertes Allyl, Crotonyl, 1-Methyl-allyl, Propargyl oder 1-Methyl-propargyl , für jeweils gegebenenfalls durch Fluor, Chlor, Brom, Cyano, Carboxy, Phenyl (welches gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, n- oderi-Propoxy, Difluormethoxy oder Trifluormethoxy substituiert ist), Methyl, Ethyl, n- oder i-Propyl, Methoxy-carbonyl oder Ethoxy-carbonyl substiuiertes Cyclopropyl, Cyclobutyl, Cyclopentyl oder Cyclohexyl , für jeweils gegebenenfalls substituiertes Phenyl, Naphthyl, Furyl, Thienyl, Oxazolyl, Isoxazolyl, Thiazolyl, Isothiazolyl, Oxadiazolyl, Thiadiazolyl, Pyridinyl, Pyrimidinyl, Pyridazinyl, Pyrazinyl oder Triazinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl , Trifluormethyl, Difluormethoxy, Trifluormethoxy, Difluormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Ethyl, n- oder i-Propyl substituiertes Trimethylen (Propan-1,3-diyl), Methylendioxy oder Ethylendioxy, Cyclopropyl, Cyclobutyl, Cyclopentyl oder Cyclohexyl, sowie jeweils gegebenenfalls im Phenylteil bzw. im Heteroarylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy, Benzylthio, Phenoxymethyl bzw. Furyl, Thienyl, Oxazolyl, Isoxazolyl, Thiazolyl, Isothiazolyl, Oxadiazolyl, Thiadiazolyl, Pyridinyl, Pyrimidinyl, Pyridazinyl, Pyrazinyl oder Triazinyl.

Eine besonders bevorzugte Gruppe erfindungsgemäßer Verbindungen sind diejenigen Verbindungen der Formel (I), in welcher
- Ar: für ortho-Phenylen, Pyridin-2,3-diyl oder Thiophen-2,3-diyl steht,
- E: für eine der nachstehenden Gruppierungen steht 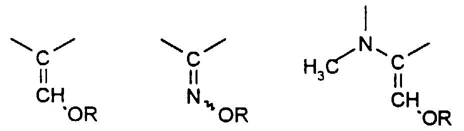 worin
R für Fluormethyl oder Difluormethyl steht,
- G: für Sauerstoff für eine direkte Bindung, Methylen, Ethen-1,2-diyl oder eine der nachstehenden Gruppierungen
-O-CO-, -CO-O-, -CH₂-O-, -O-CH₂-, -S(O)ₙ-, -CH₂-S(O)ₙ-, -S(O)ₙ-CH₂-, -C(R¹)=N-O-, -O-N=C(R¹)-, -C(R¹)=N-O-CH₂-, -N(R²)- oder -CH₂-O-N=C(R¹)- steht,
wobei
n für die Zahlen 0, 1 oder 2 steht,
R¹ für Wasserstoff, Methyl oder Ethyl steht und
R² für Wasserstoff, Methyl oder Ethyl steht,
- Q¹: für Sauerstoff steht,
- Q²: für Methoxy, Ethoxy, Methylamino oder Ethylamino steht, sowie
- Z: für jeweils gegebenenfalls substituiertes Phenyl, Thiazolyl, Thiadiazolyl, Pyridinyl, Pyrimidinyl, Pyridazinyl, Pyrazinyl oder Triazinyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxy-carbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy, Benzylthio oder Phenoxymethyl, sowie ferner jeweils gegebenenfalls einfach oder mehrfach, gleich oder verschieden durch Fluor, Chlor, Brom, Methyl, Methoxy oder Trifluormethyl substituiertes Furyl, Thienyl, Pyridinyl oder Pyrimidinyl.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Verwendet man beispielsweise α-(2-Phenoxy-phenyl)-glyoxylsäure-methylester und O-Fluormethyl-hydroxylamin als Ausgangsstoffe, so kann der Reaktionsablauf beim erfindungsgemäßen Verfahren (a) durch das folgende Formelschema skizziert werden:

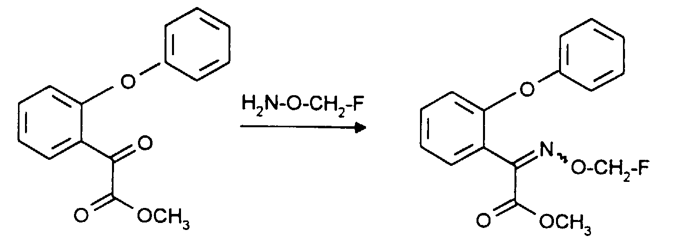

Verwendet man beispielsweise α-Hydroximino-(2-(2-methyl-phenoxy)-methyl)-phenyl-essigsäure-methylester und Chlordifluormethan als Ausgangsstoffe, so kann der Reaktionsablauf beim erfindungsgemäßen Verfahren (b) durch das folgende Formelschema skizziert werden:

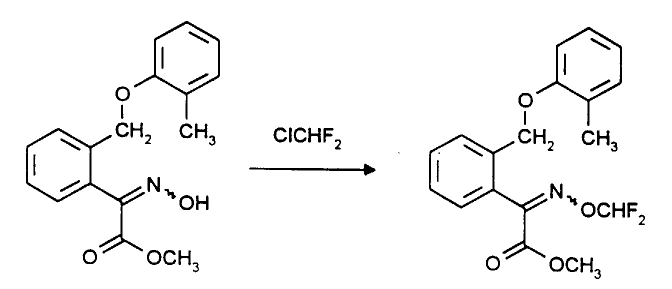

Die beim erfindungsgemäßen Verfahren (a) zur Herstellung der Verbindungen der allgemeinen Formel (I) als Ausgangsstoffe zu verwendenden Arylglyoxylsäurederivate sind durch die Formel (II) allgemein definiert. In der Formel (II) haben Ar, G, Q¹, Q² und Z vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Ar, G, Q¹, Q² und Z angegeben wurden.

Die Ausgangsstoffe der Formel (II) sind bekannt und/oder können nach an sich bekannten Verfahren hergestellt werden (vgl. EP-A 253213).

Die beim erfindungsgemäßen Verfahren (a) weiter als Ausgangsstoffe zu verwendenden O-Halogenalkyl-hydroxylamine sind durch die Formel (III) allgemein definiert. In der Formel (In) hat R vorzugsweise bzw. insbesondere diejenige Bedeutung, die bereits oben im Zusammenhang mit der Beschreibung der Verbindungen der Formel (I) als bevorzugt bzw. als insbesondere bevorzugt für R angegeben wurde.

Die Ausgangsstoffe der Formel (III) sind bekannt und/oder können nach an sich bekannten Verfahren hergestellt werden (vgl. EP-A 333154).

Die beim erfindungsgemäßen Verfahren (b) zur Herstellung der Verbindungen der allgemeinen Formel (I) als Ausgangsstoffe zu verwendenden 2-Hydroximino-aryl-essigsäurederivate sind durch die Formel (IV) allgemein definiert. In der Formel (IV) haben Ar, G, Q¹, Q² und Z vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Ar, G, Q¹, Q² und Z angegeben wurden.

Die beim erfindungsgemäßen Verfahren (b) weiter als Ausgangsstoffe zu verwendenden Halogenalkane sind durch die Formel (V) allgemein definiert. In der Formel (V) hat R vorzugsweise bzw. insbesondere diejenige Bedeutung, die bereits oben im Zusammenhang mit der Beschreibung der Verbindungen der Formel (I) als bevorzugt bzw. als insbesondere bevorzugt für R angegeben wurde; X steht vorzugsweise für Fluor, Chlor, Brom oder Iod, insbesondere für Chlor oder Brom.

Die Ausgangsstoffe der Formel (V) sind bekannt und/oder können nach an sich bekannten Verfahren hergestellt werden (vgl. DE 3906273).

Als Verdünnungsmittel zur Durchführung der erfindungsgemäßen Verfahren (a) und (b) kommen die üblichen organischen Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether.

Die erfindungsgemäßen Verfahren (a) und (b) werden vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie basische organische Stickstoffverbindungen, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Als weitere Reaktionshilfsmittel können gegebenenfalls auch Reaktionsbeschleuniger, wie z.B. Kaliumiodid oder Natriumiodid eingesetzt werden.

Die Reaktionstemperaturen können bei der Durchführung der erfindungsgemäßen Verfahren (a) oder (b) in einem größeren Bereich variiert werden. Im allgemeinen arbeitet man bei Temperaturen zwischen -20°C und +100°C, vorzugsweise bei Temperaturen zwischen 0°C und 80°C.

Die erfindungsgemäßen Verfahren (a) und (b) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung der erfindungsgemäßen Verfahren (a) und (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Reaktionshilfsmittels durchgeführt, und das Reaktionsgemisch wird mehrere Stunden bei der jeweils erforderlichen Temperatur gerührt. Die Aufarbeitung erfolgt bei den erfindungsgemäßen Verfahren jeweils nach üblichen Methoden (vgl. die Herstellungsbeispiele).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bei kämpfung von Krankheiten im Obst- und Gemüseanbau, wie beispielsweise gegen Podosphaera-Arten (z.B. an Äpfeln), Sphaerotheca-Arten (z.B. an Gurken) und Venturia-Arten (z.B. an Äpfeln), ferner zur Bekämpfung von Getreidekrankheiten, wie beispielsweise gegen Erysiphe-Arten (z.B. an Gerste), Pyrenophora-Arten (z.B. an Gerste), Cochliobolus-Arten (z.B. an Gerste und Weizen) und Septoria-Arten (z.B. an Weizen) sowie zur Bekämpfung von Reiskrankheiten, wie beispielsweise gegen Pyricularia-Arten eingesetzt werden.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden:

Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele:

### Beispiel 1

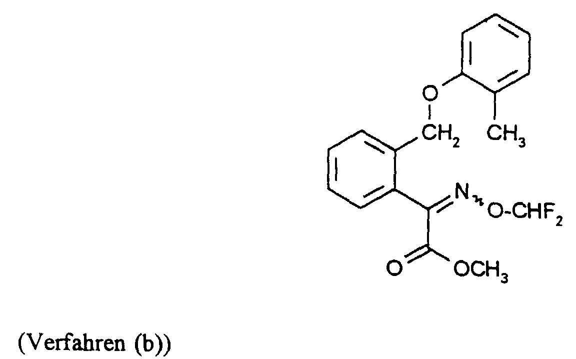

3,0 g (10 mmol) E/Z-α-Hydroximino-(2-(2-methylphenoxy)-methyl)-phenylessigsäure-methylester werden in 30 ml Dimethylsulfoxid gelöst und 2,76 g (20 mmol) Kaliumcarbonat sowie 0,5 g Kaliumiodid dazu gegeben. Nach 20-minütigem Rühren wird die Suspension mit Chlordifluormethan (Frigen-22) gesättigt und in einer Frigen-22-Atmosphäre 4 Tage bei 20°C gerührt.

Zur Aufarbeitung wird auf etwa das doppelte Volumen Eiswasser gegossen und mit t-Butyl-methylether extrahiert. Die organische Phase wird mit Natriumsulfat getrocknet und filtriert. Das Filtrat wird eingeengt und der Rückstand durch Säulenchromatographie (Kieselgel, Essigsäureethylester/Cyclohexan, Vol.: 1:5) gereinigt.

Man erhält 1,05 g (30% der Theorie) E/Z-α-Difluormethoximino-(2-(2-methylphenoxy)-methyl)-phenylessigsäure-methylester als Öl.

¹H-NMR (D₆-Dimethylsulfoxid, δ): 4,98 ppm (-O-CH₂-).

### Beispiel 2

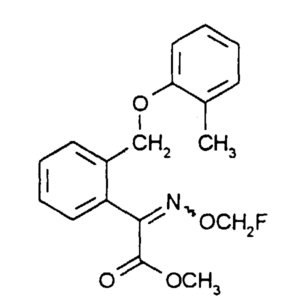

5,98 g (20 mmol) E/Z-α-Hydroximino-(2-(2-methylphenoxy)-methyl)-phenylessigsäure-methylester werden in 30 ml Dimethylsulfoxid gelöst und mit 5,52 g (40 mmol) Kaliumcarbonat sowie 0,5 g Kaliumiodid versetzt. Nach 20-minütigem Rühren bei 20°C wird das Gemisch auf 10°C abgekühlt, eine Lösung von 2,71 g (24 mmol) Bromfluormethan in 5 ml vorgekühltem Dimethylformamid tropfenweise dazu gegeben und die Reaktionsmischung über Nacht bei 20°C gerührt.

Zur Aufarbeitung wird das Gemisch auf etwa das doppelte Volumen Eiswasser gegossen und dreimal mit Diethylether extrahiert. Die vereinigten organischen Phasen werden mit Natriumsulfat getrocknet und filtriert. Das Filtrat wird eingeengt und der Rückstand durch Säulenchromatographie (Kieselgel, Essigsäureethylester/Cyclohexan, Vol.: 1:5) gereinigt.

Man erhält als erste Fraktion 1,8 g (27% der Theorie) Z-α-Fluormethoximino-(2-(2-methylphenoxy)-methyl)-phenylessigsäure-methylester als Öl [¹H-NMR (D₆-Dimethylsulfoxid, δ): 5,23 ppm (-O-CH₂-), 5,76 ppm (-CH₂-F); als zweite Fraktion 2,6 g (39% der Theorie) E-α-Fluormethoximino-(2-(2-methylphenoxy)-methyl)-phenylessigsäure-methylester als kristallines Produkt vom Schmelzpunkt 108°C.

Analog Beispiel 1 oder 2 sowie entsprechend der allgemeinen Beschreibung der erfindungsgemäßen Herstellungsverfahren können beispielsweise auch die in den nachstehenden Tabellen aufgeführten Verbindungen der Formel (I) hergestellt werden.

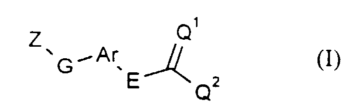

Die ¹H-NMR-Spektren wurden aufgenommen in CDCl₃ mit Tetramethylsilan als innerem Standard. Angegeben sind in der Regel die chemischen Verschiebungen als δ-Werte.

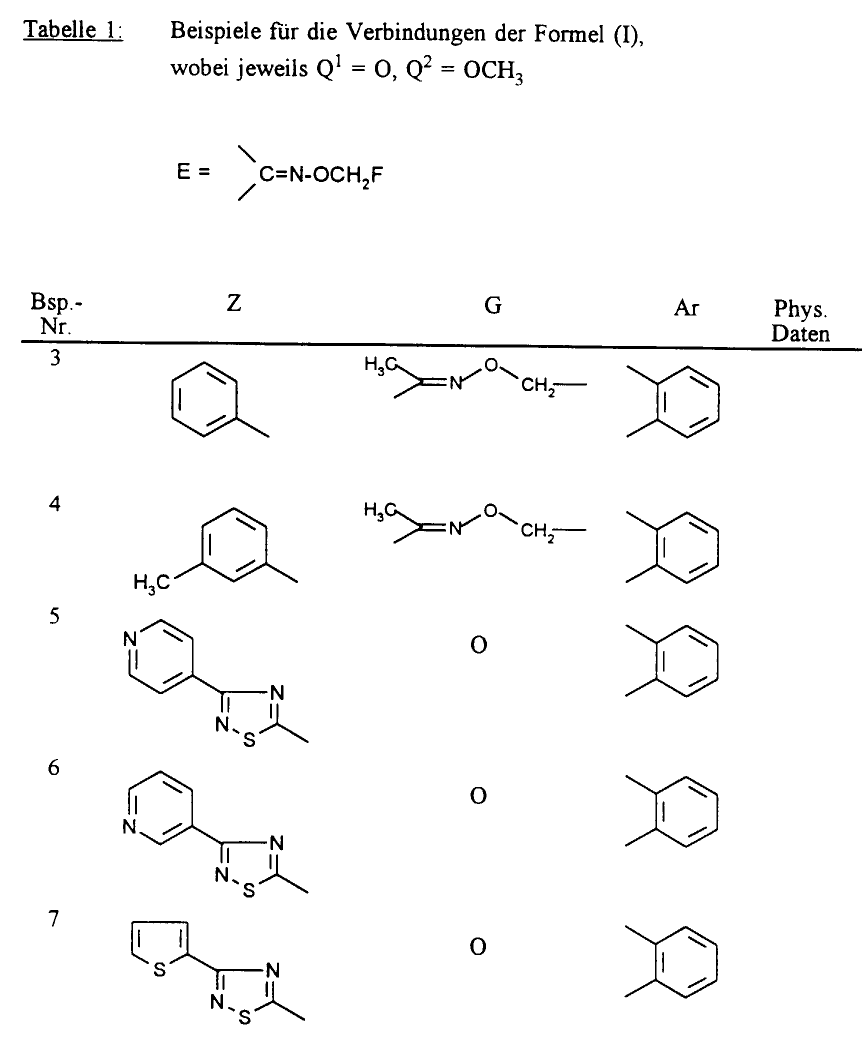

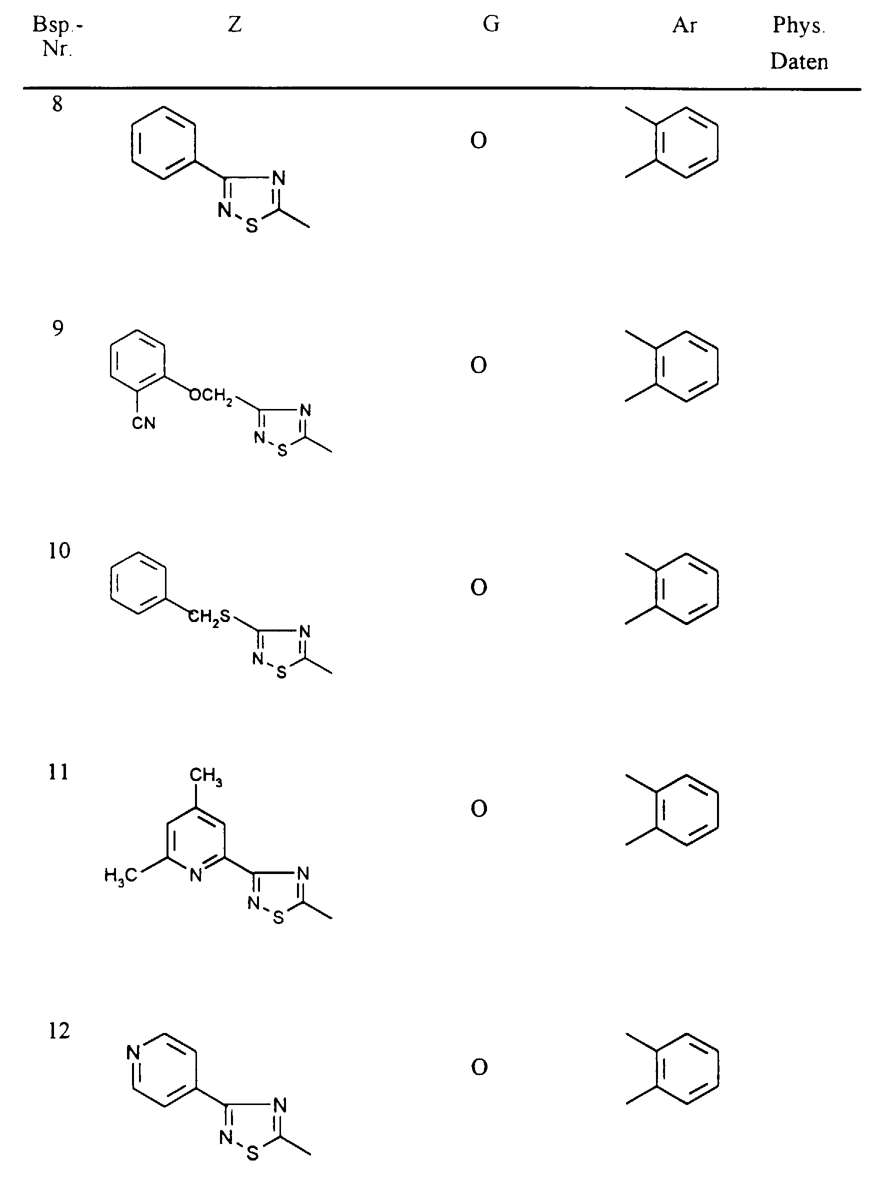

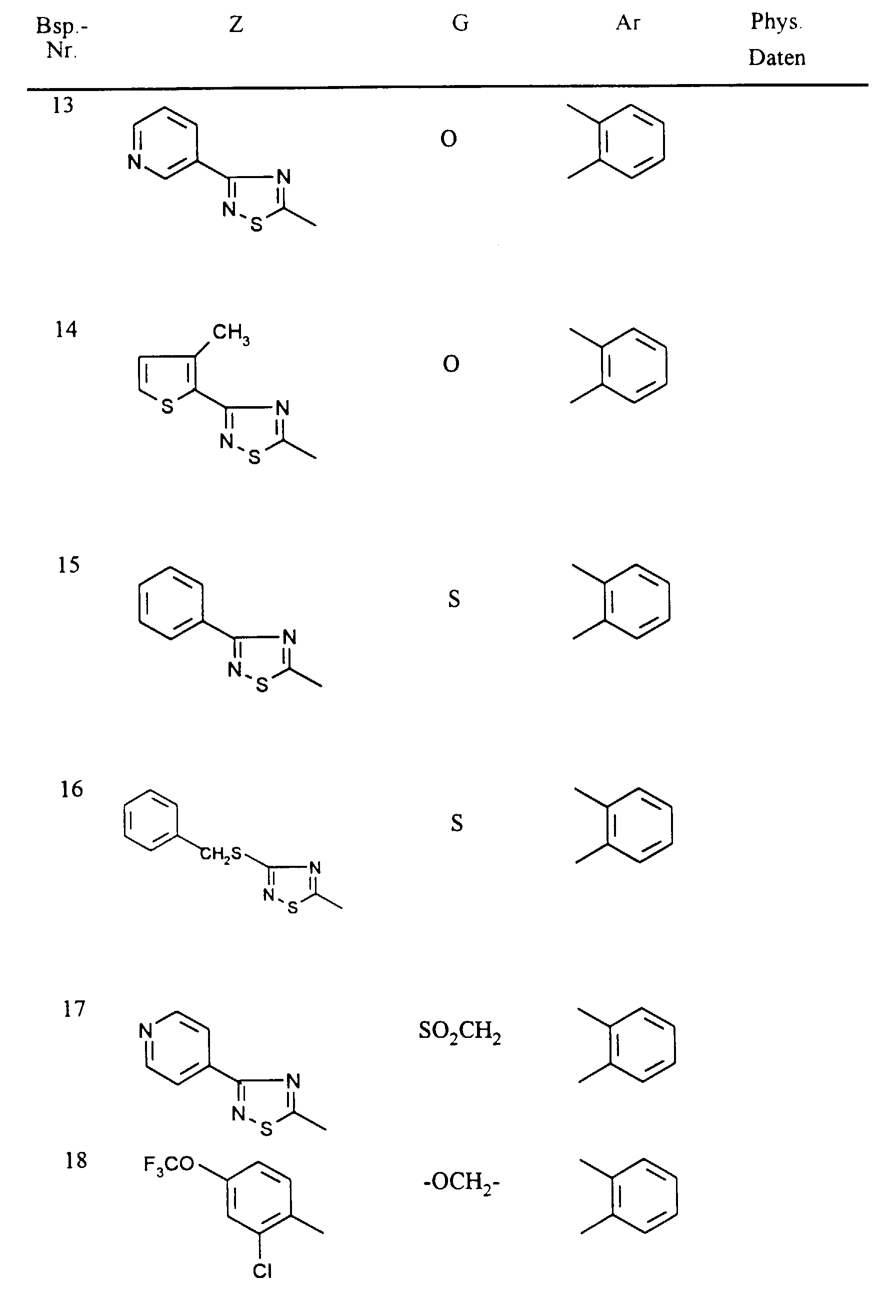

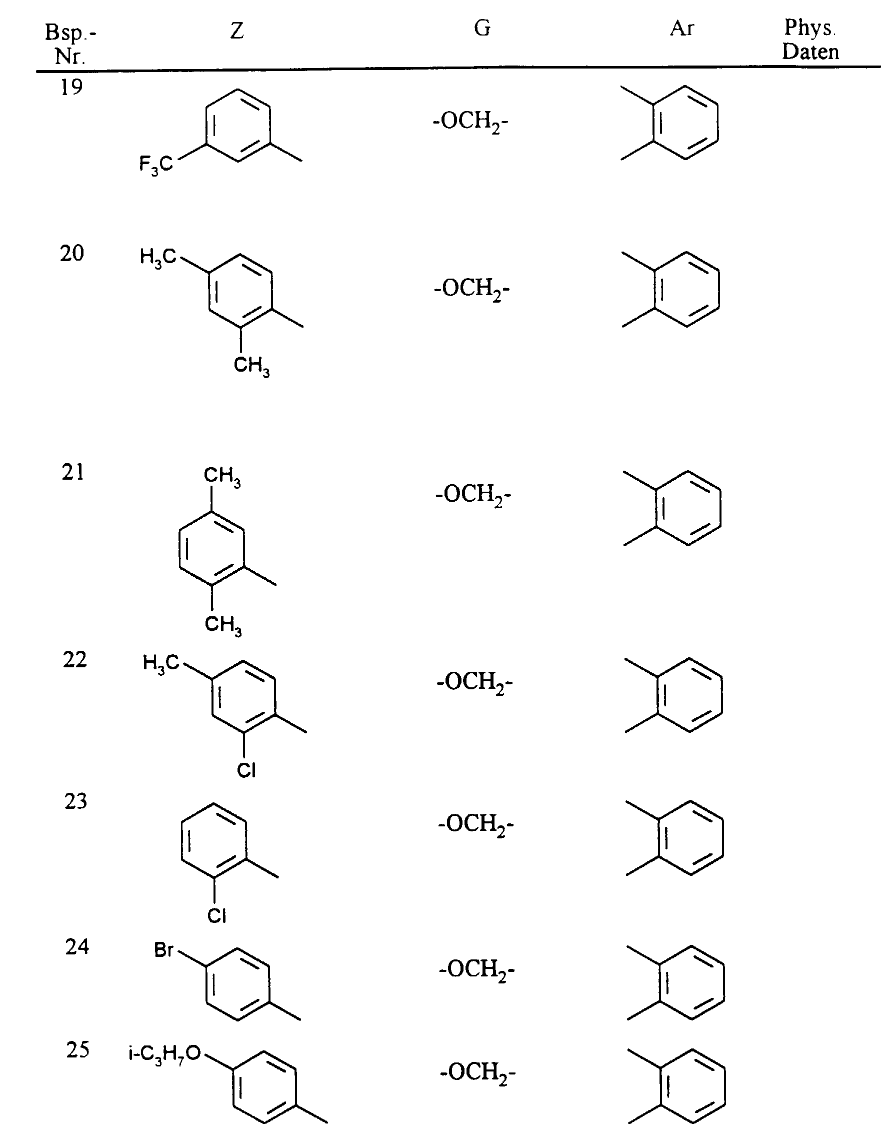

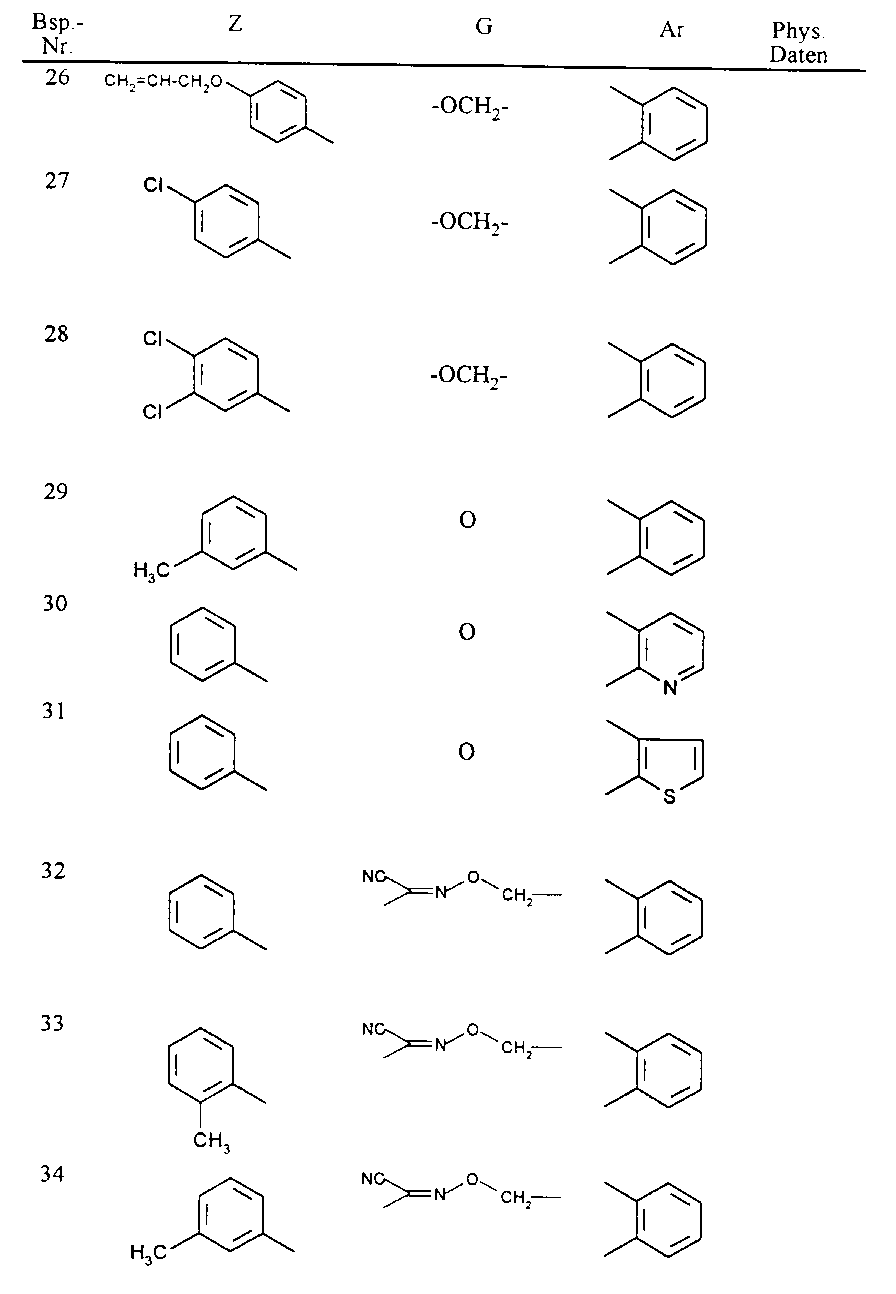

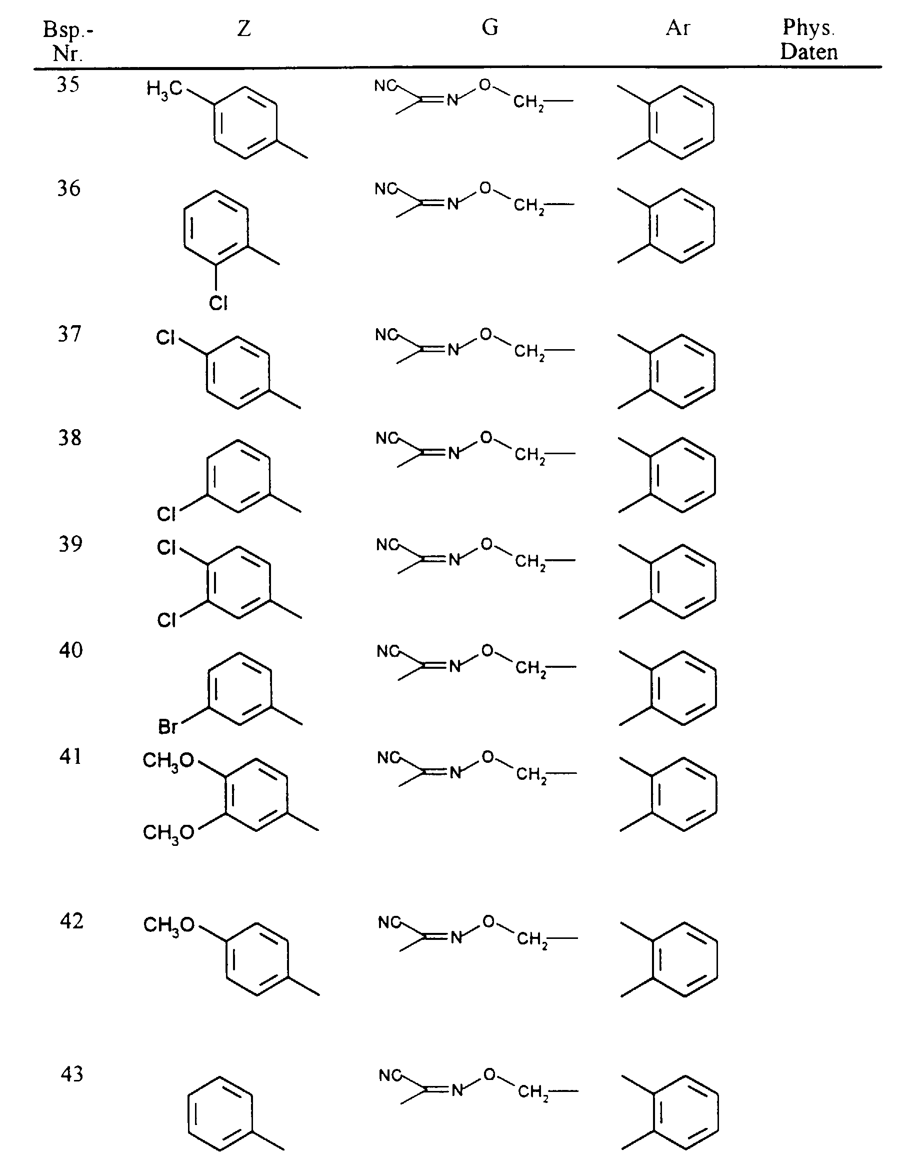

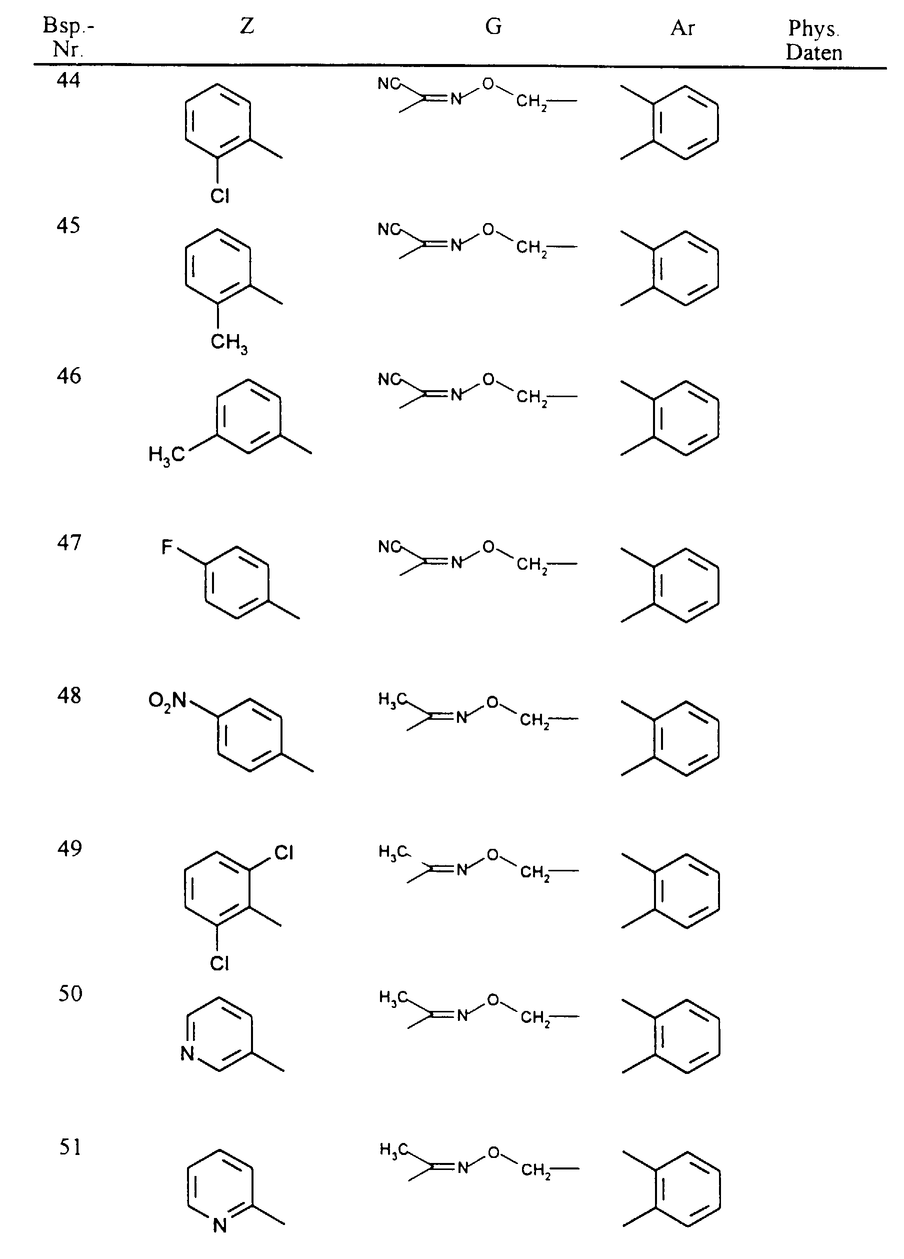

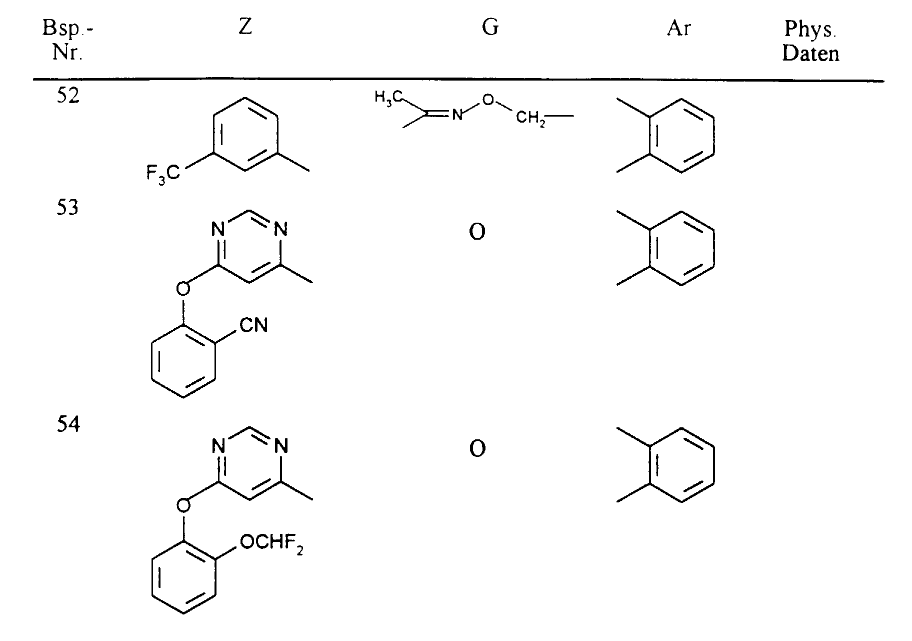

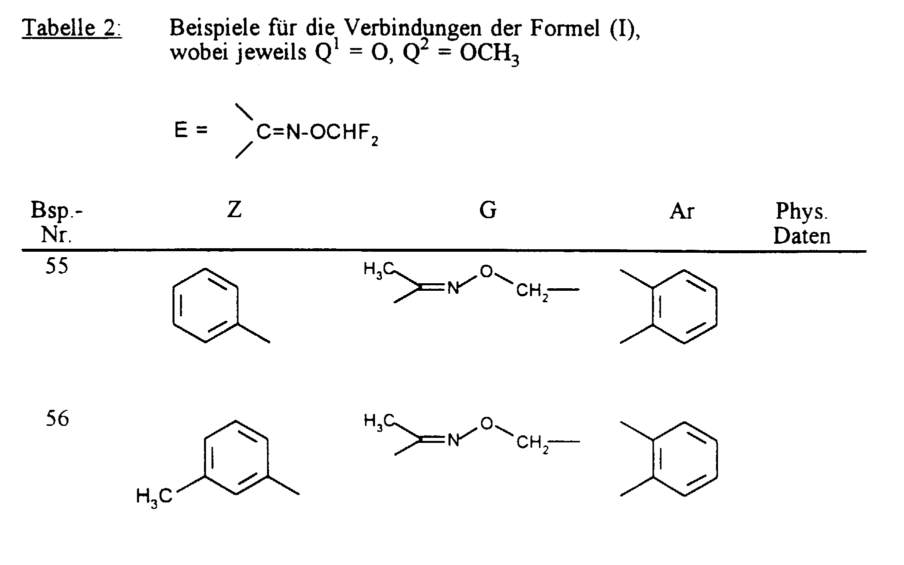

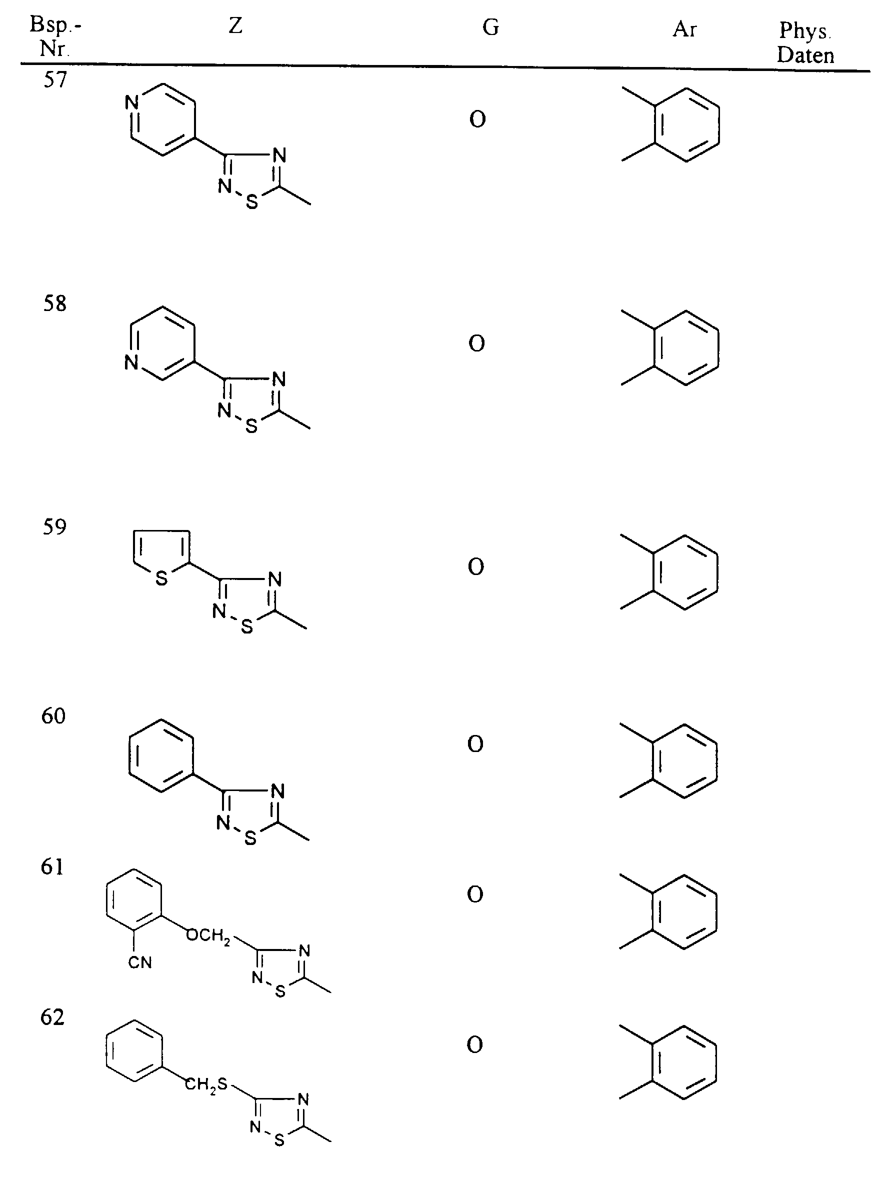

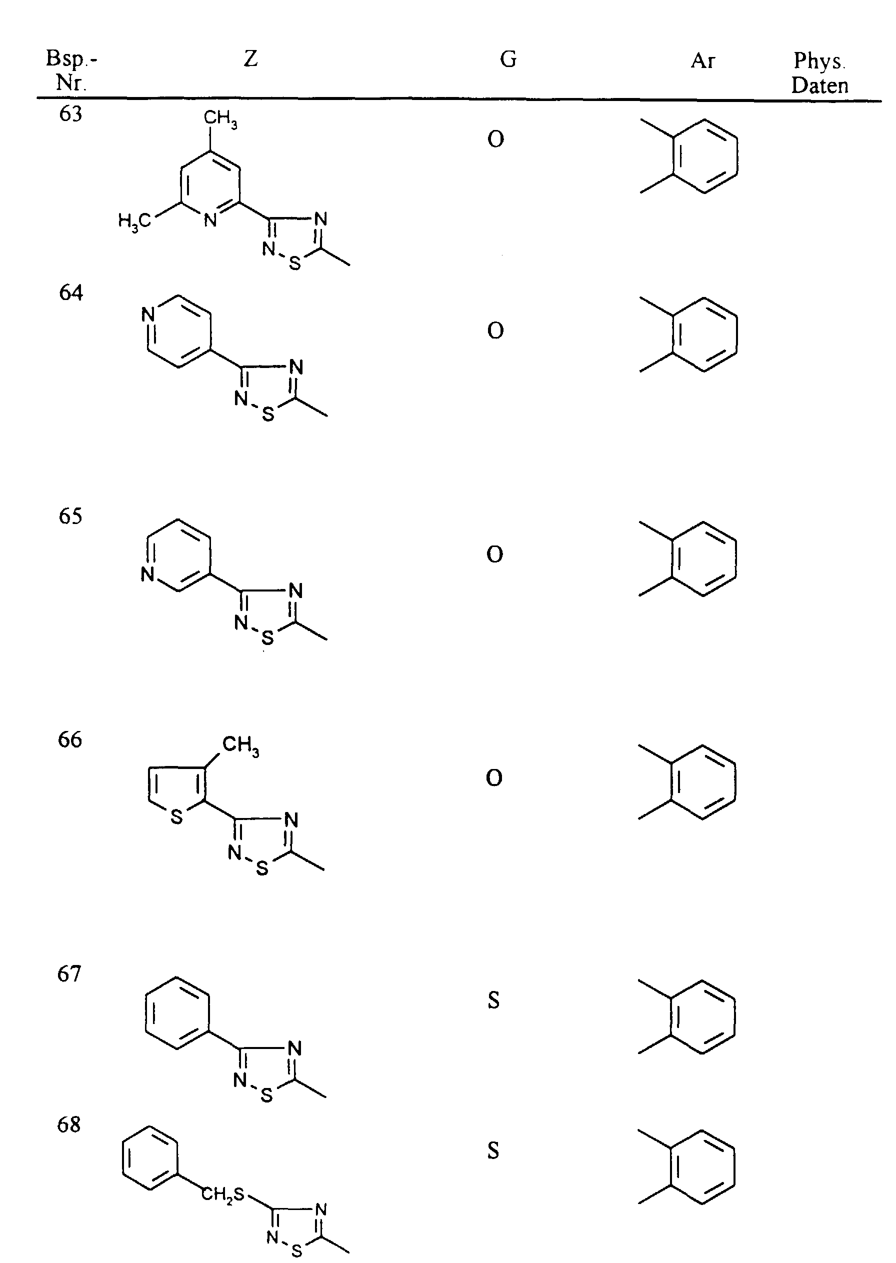

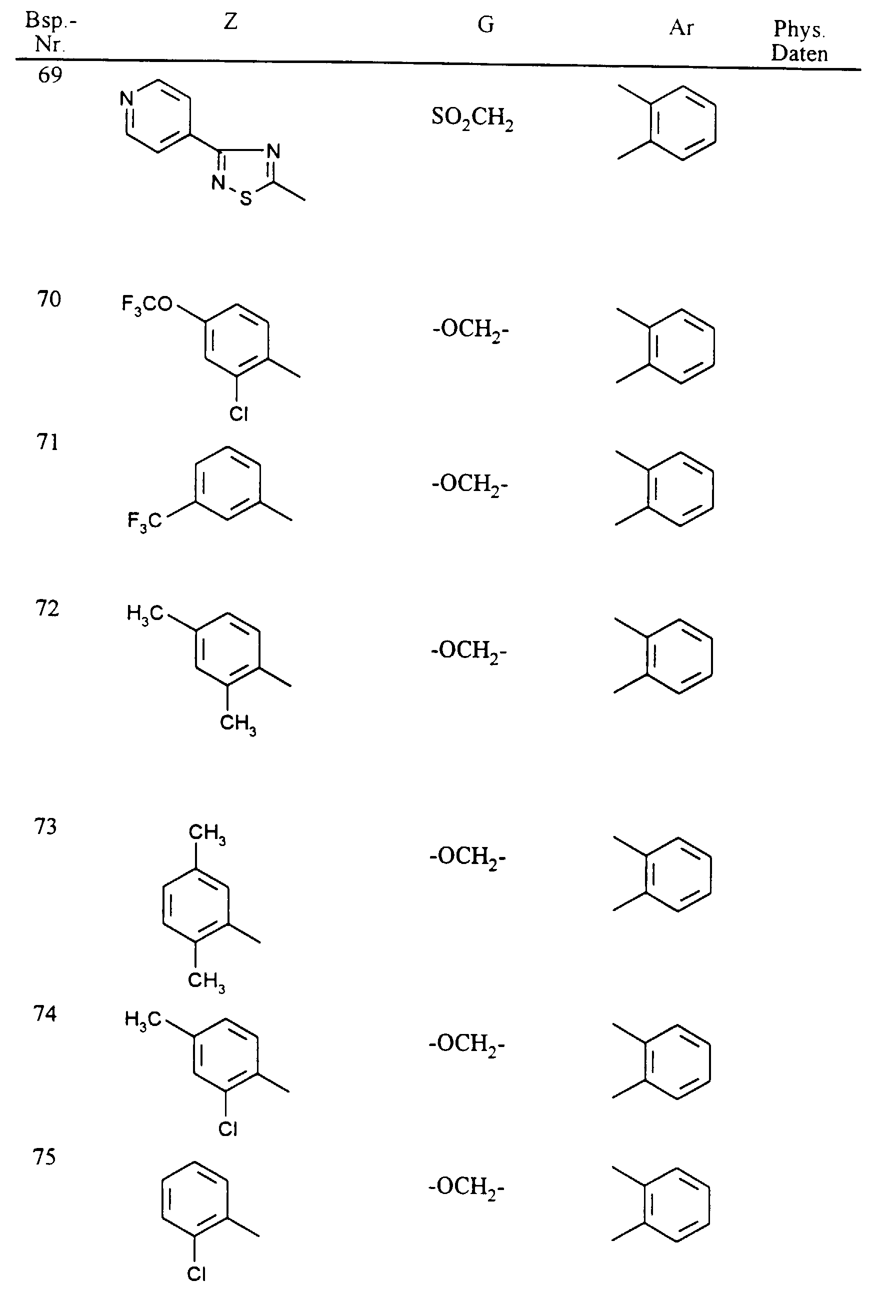

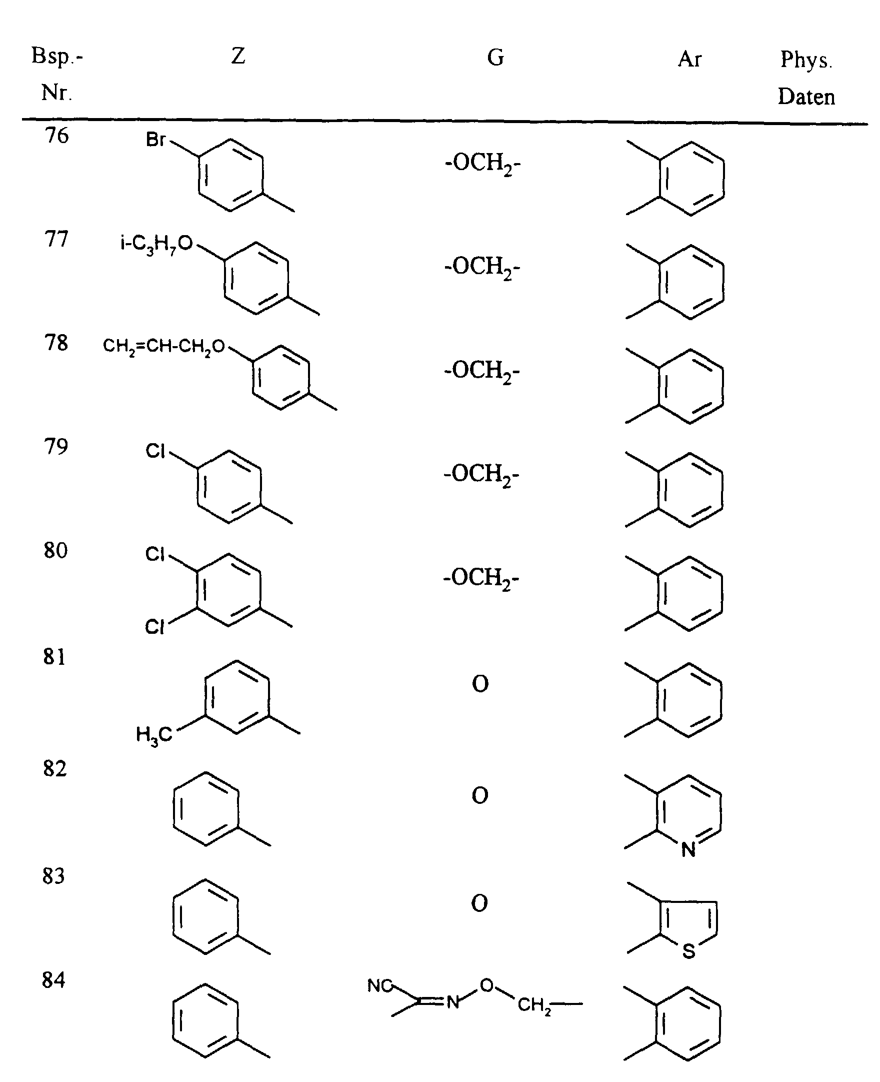

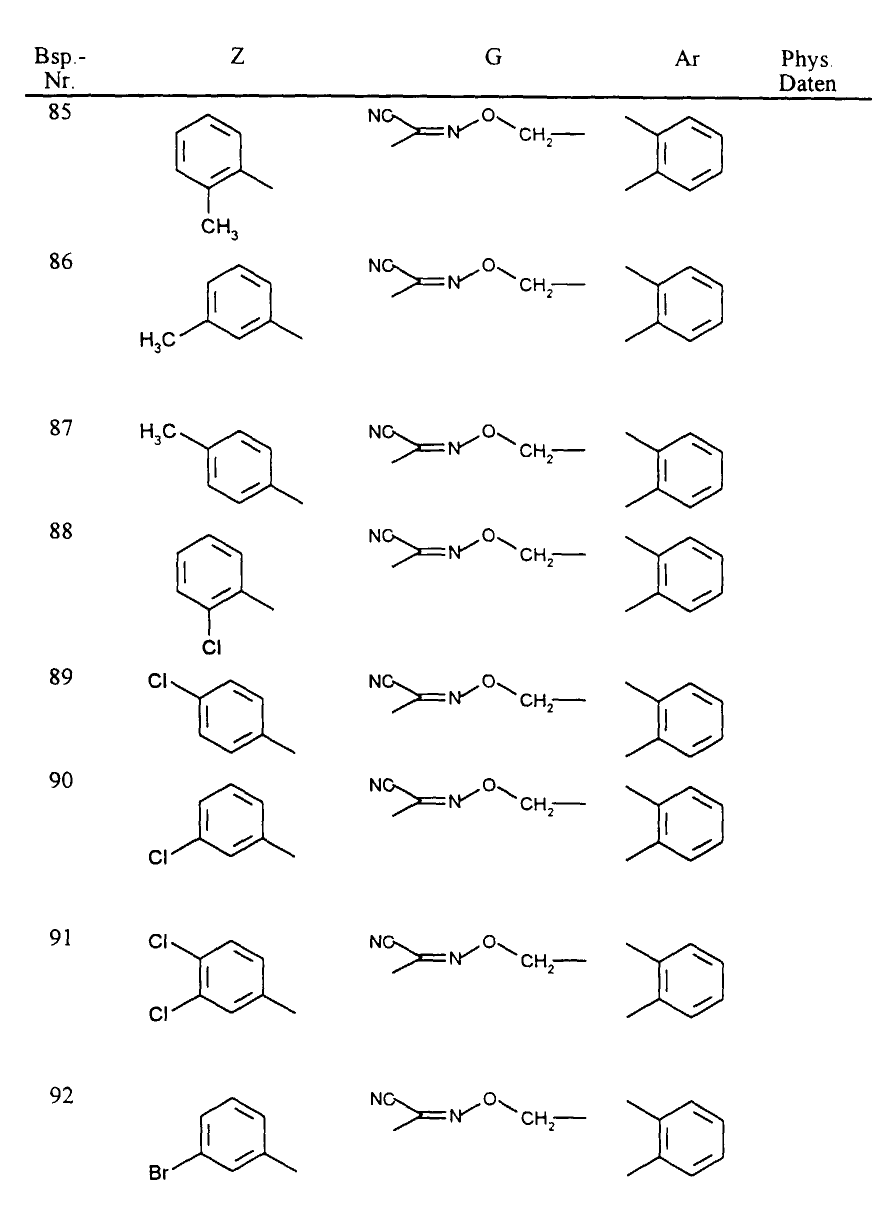

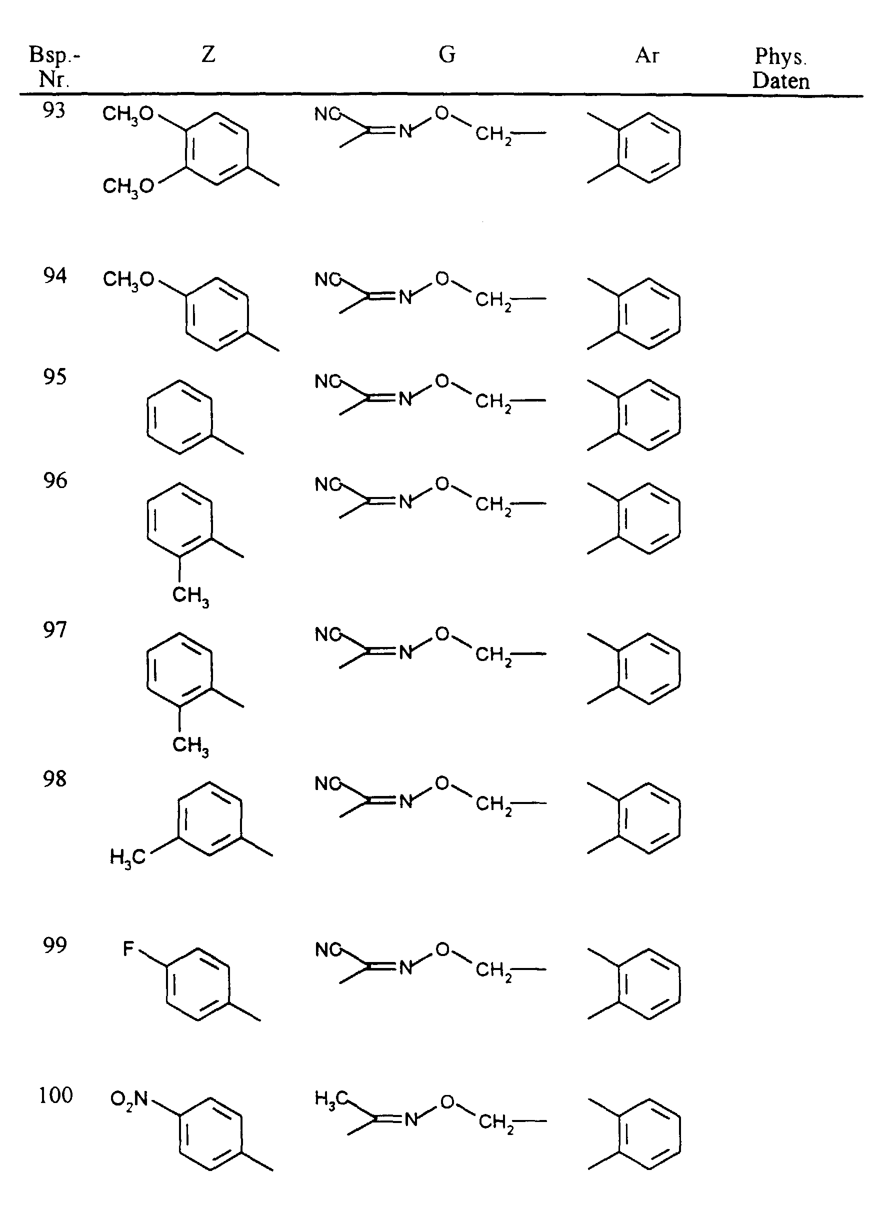

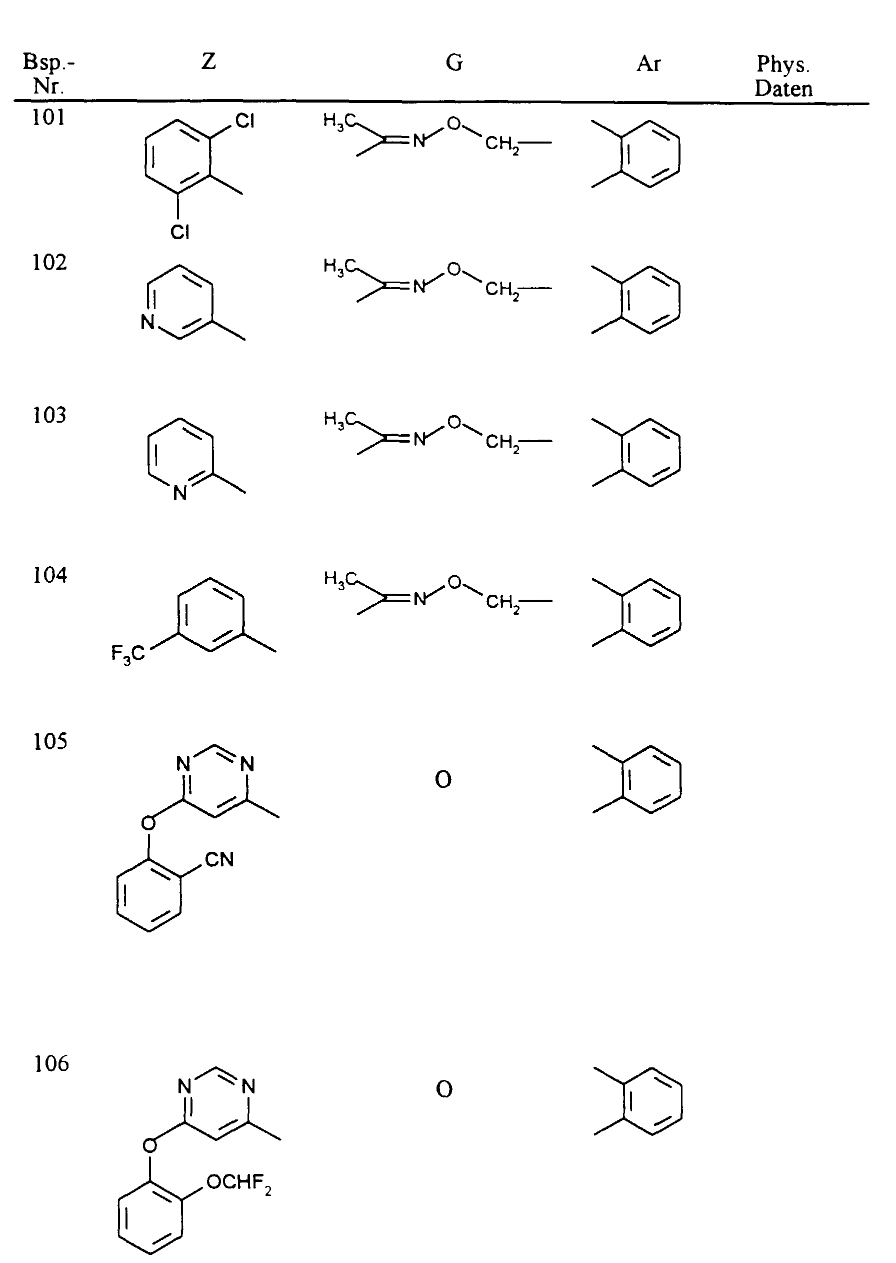

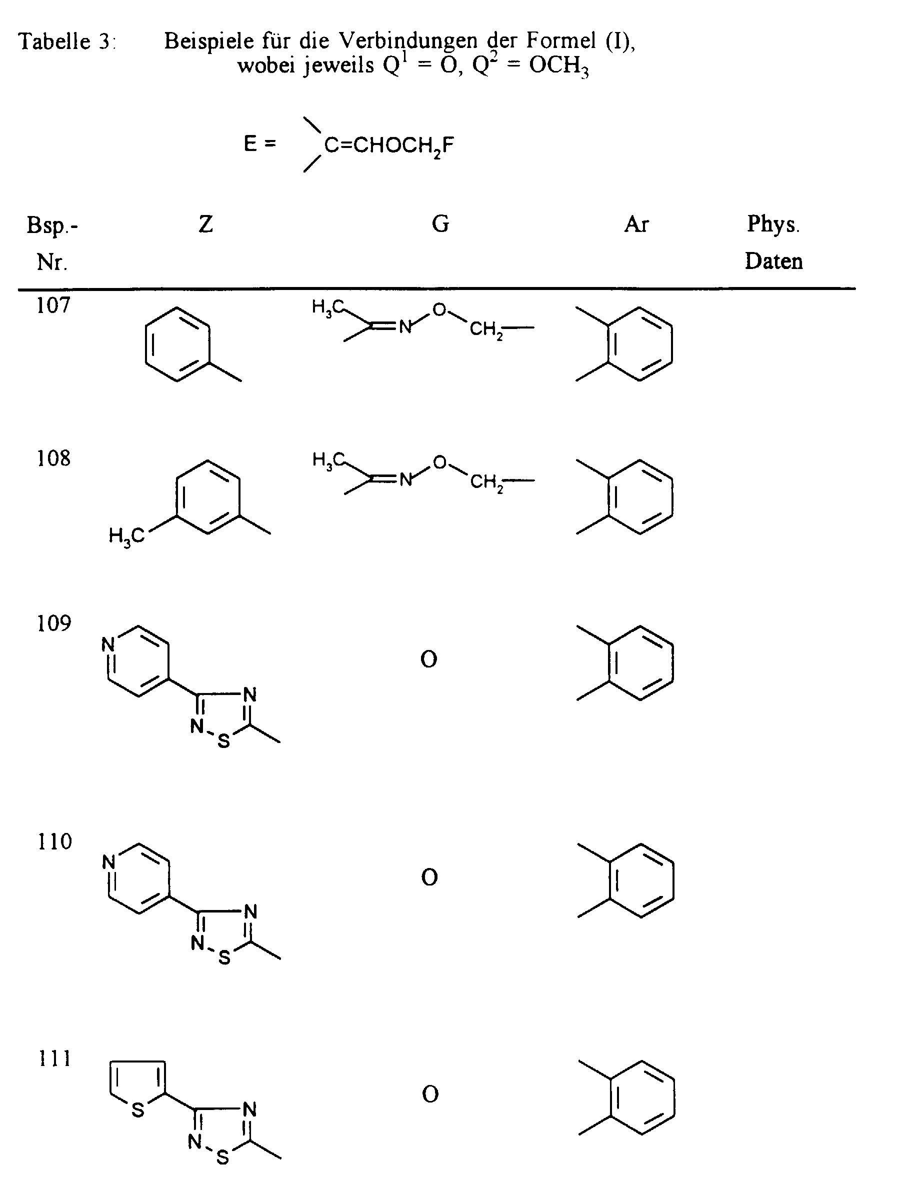

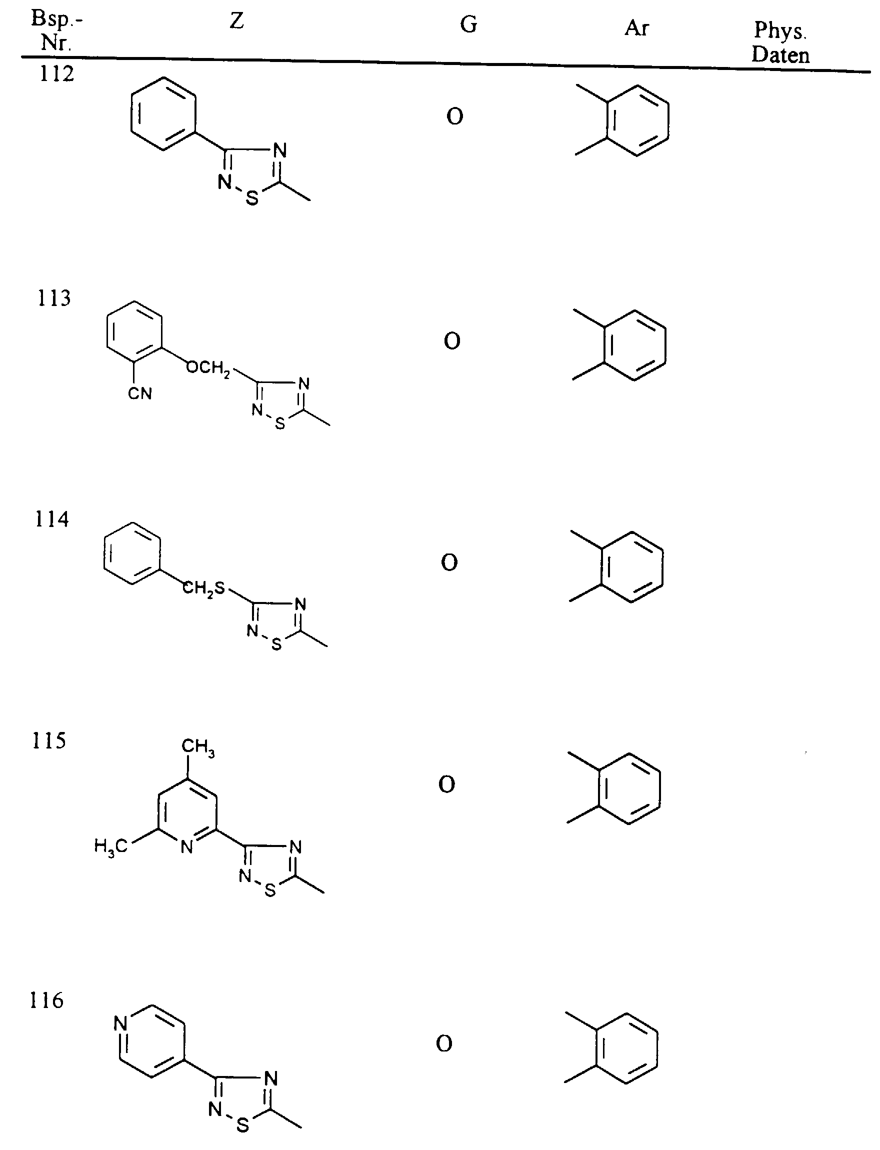

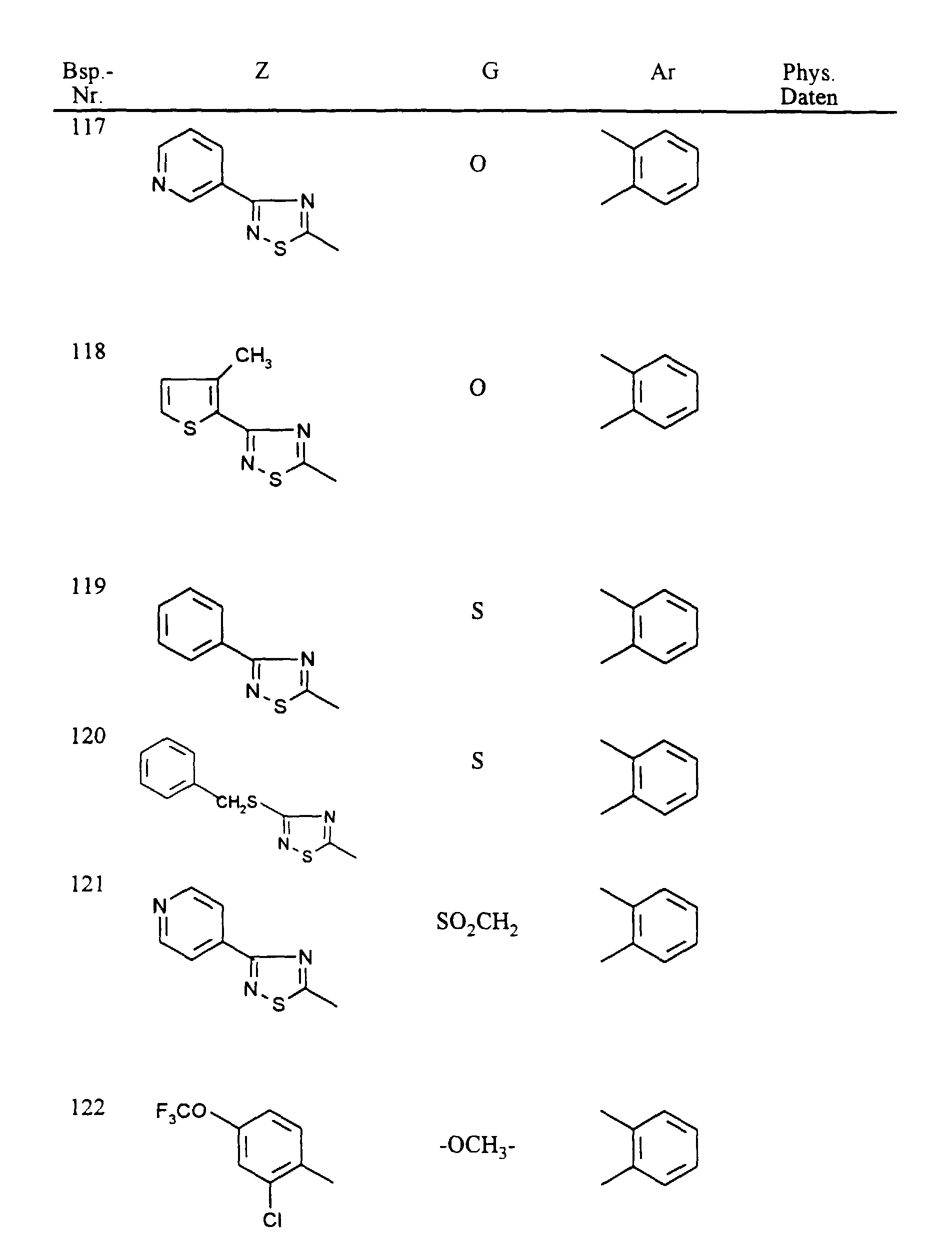

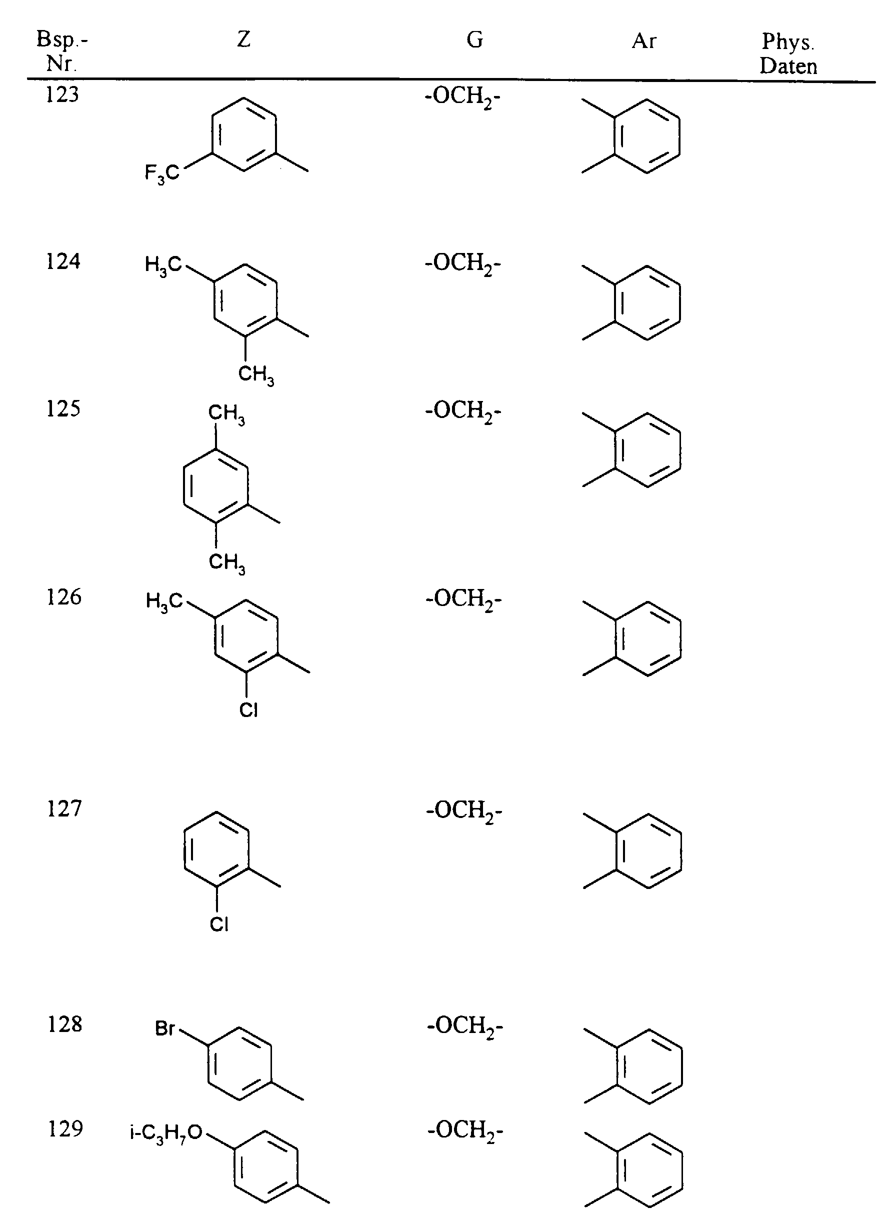

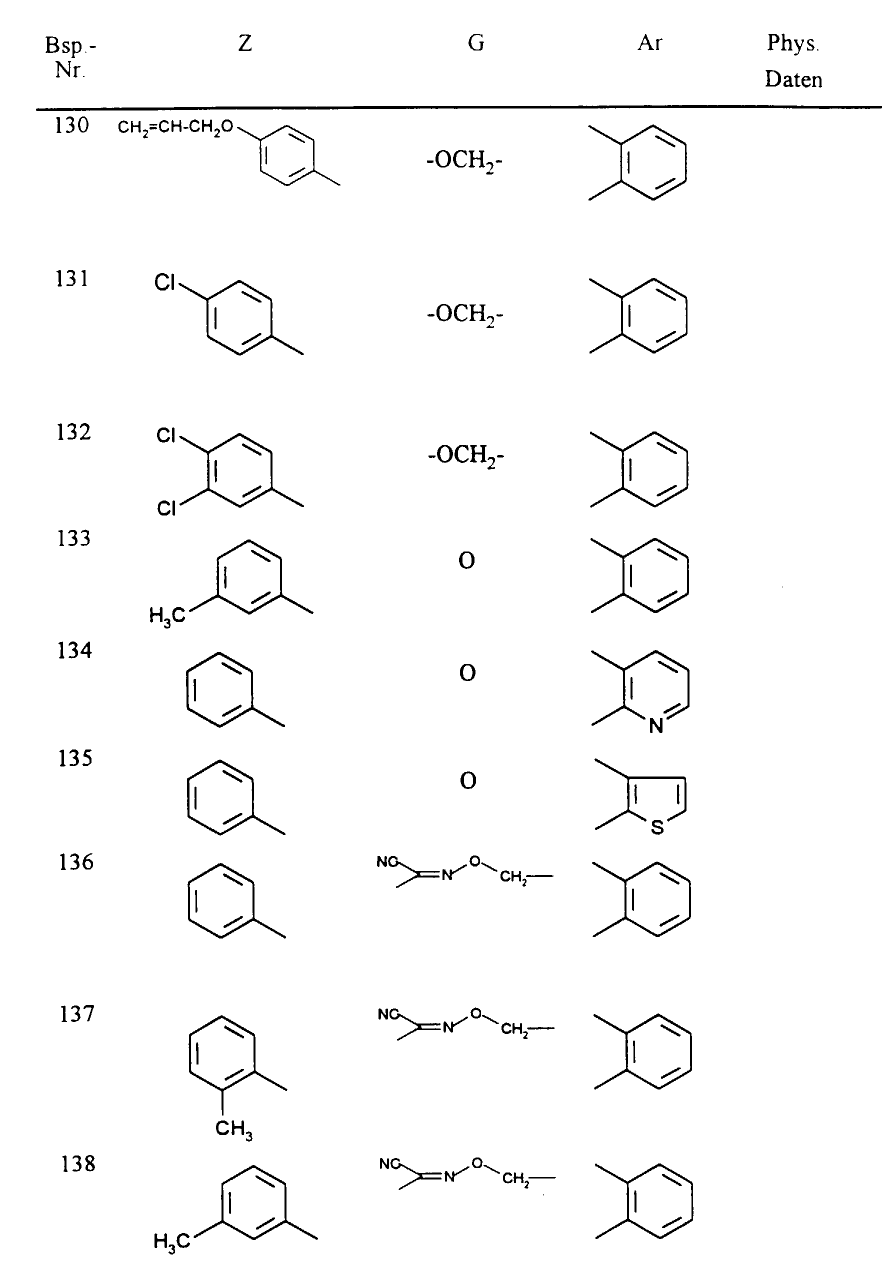

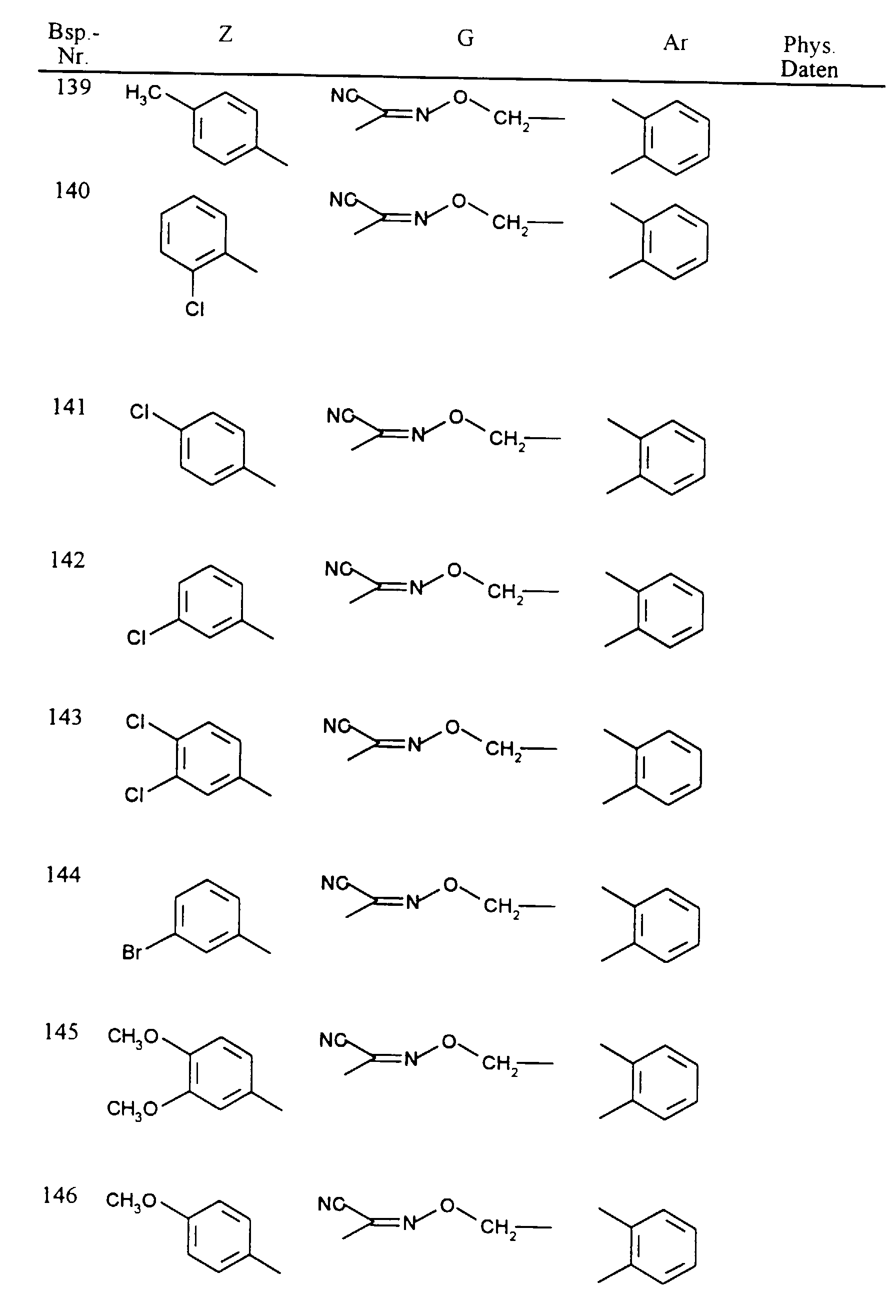

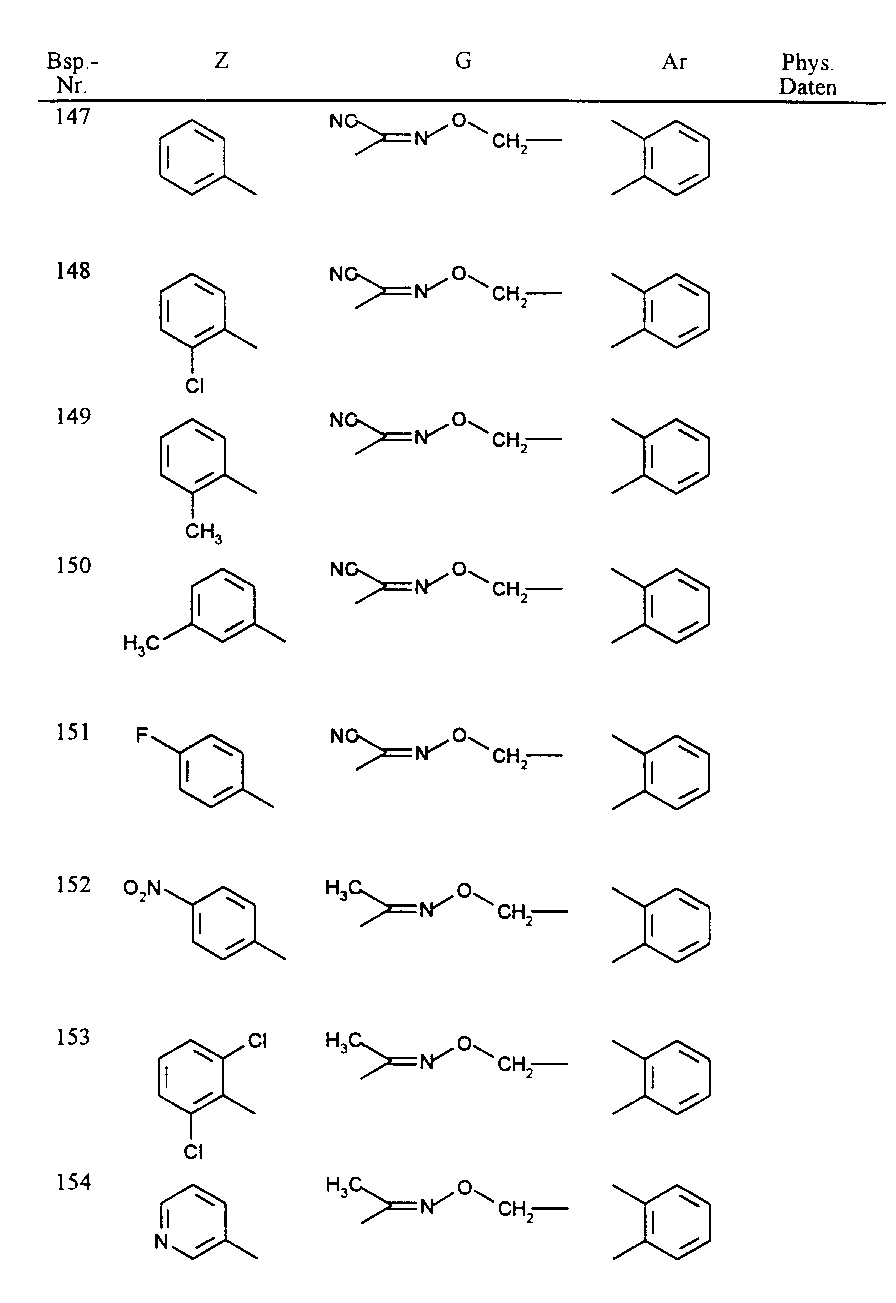

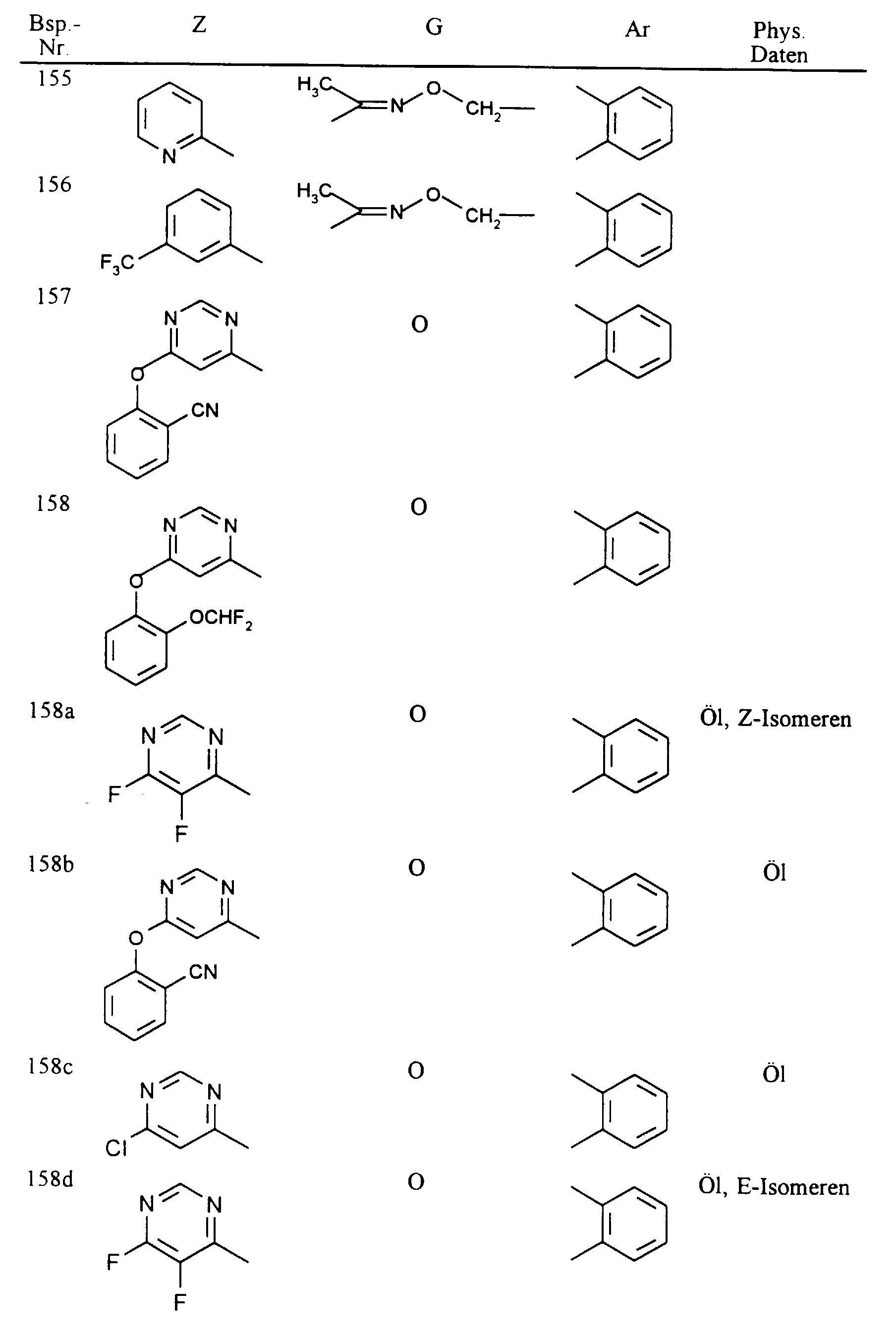

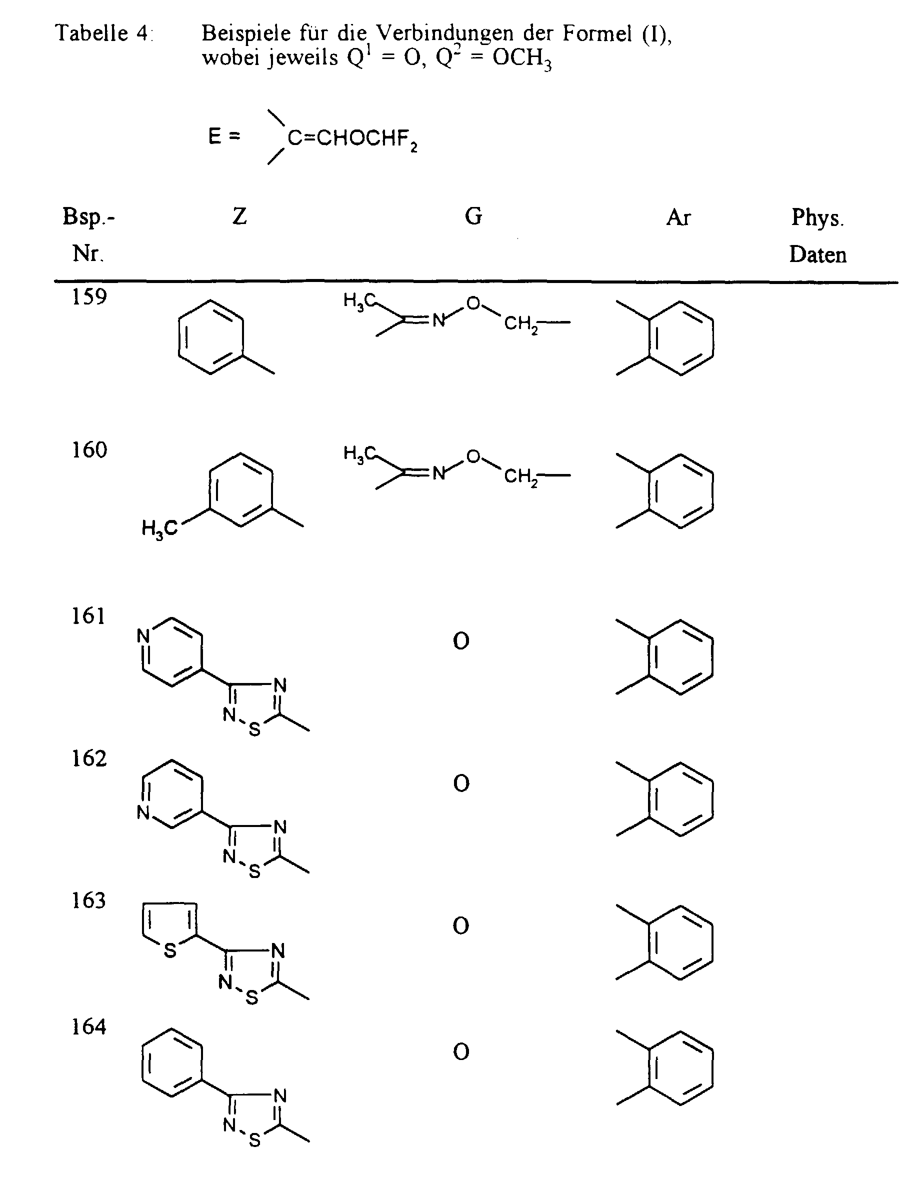

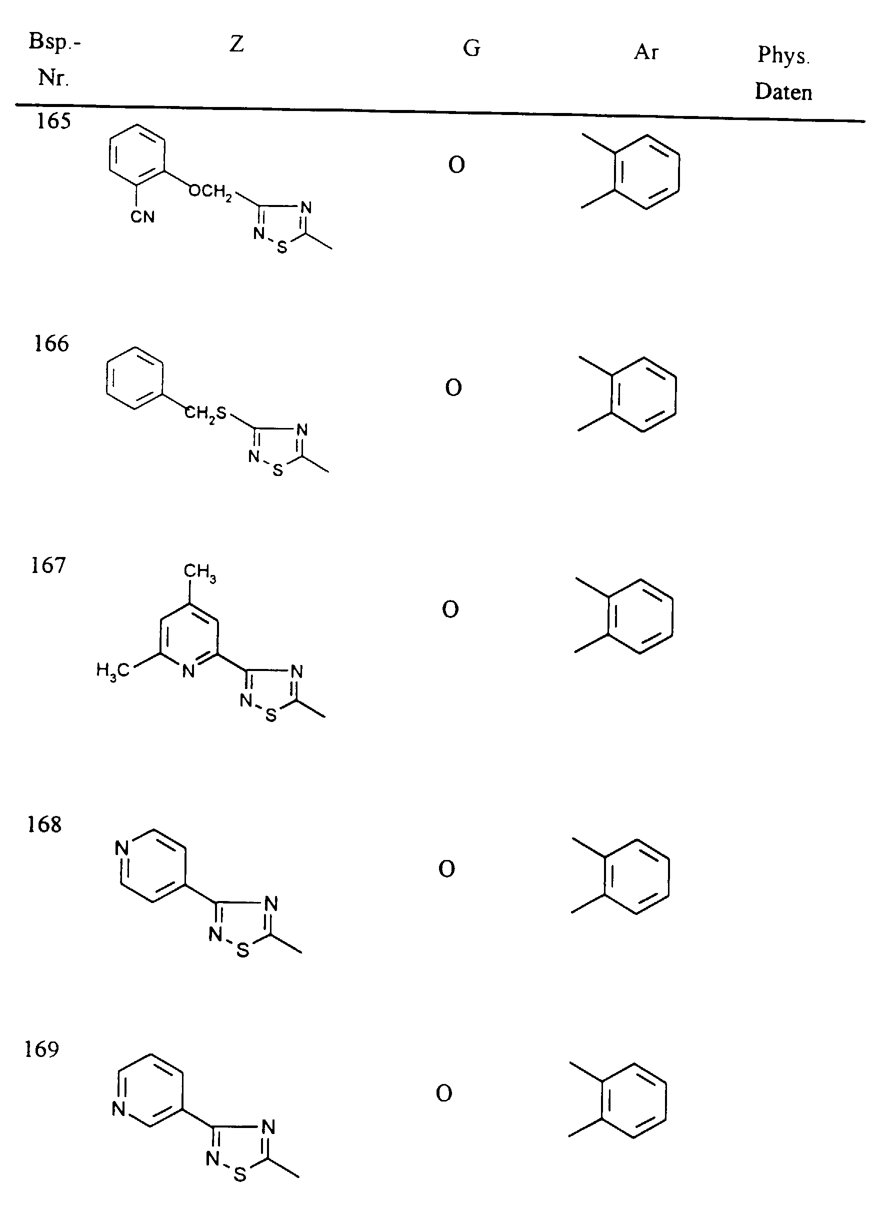

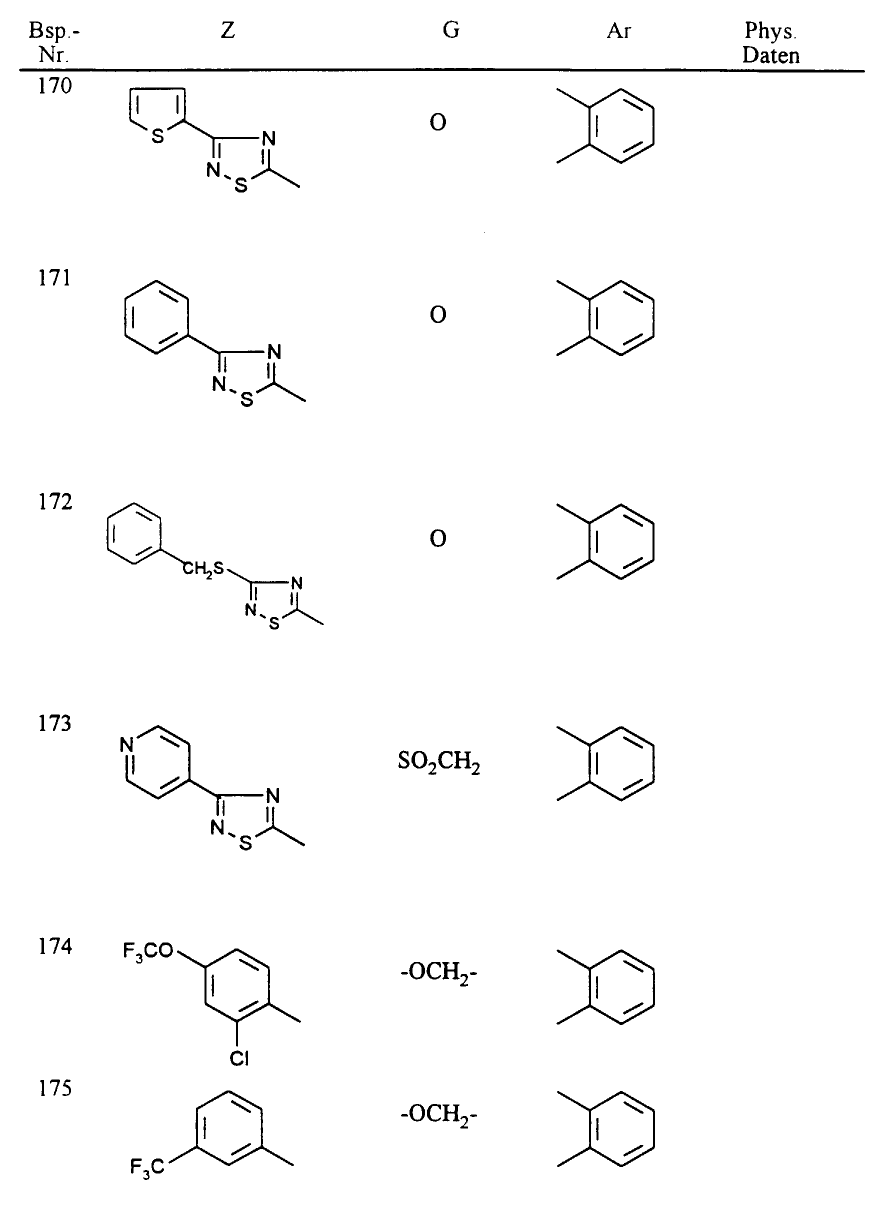

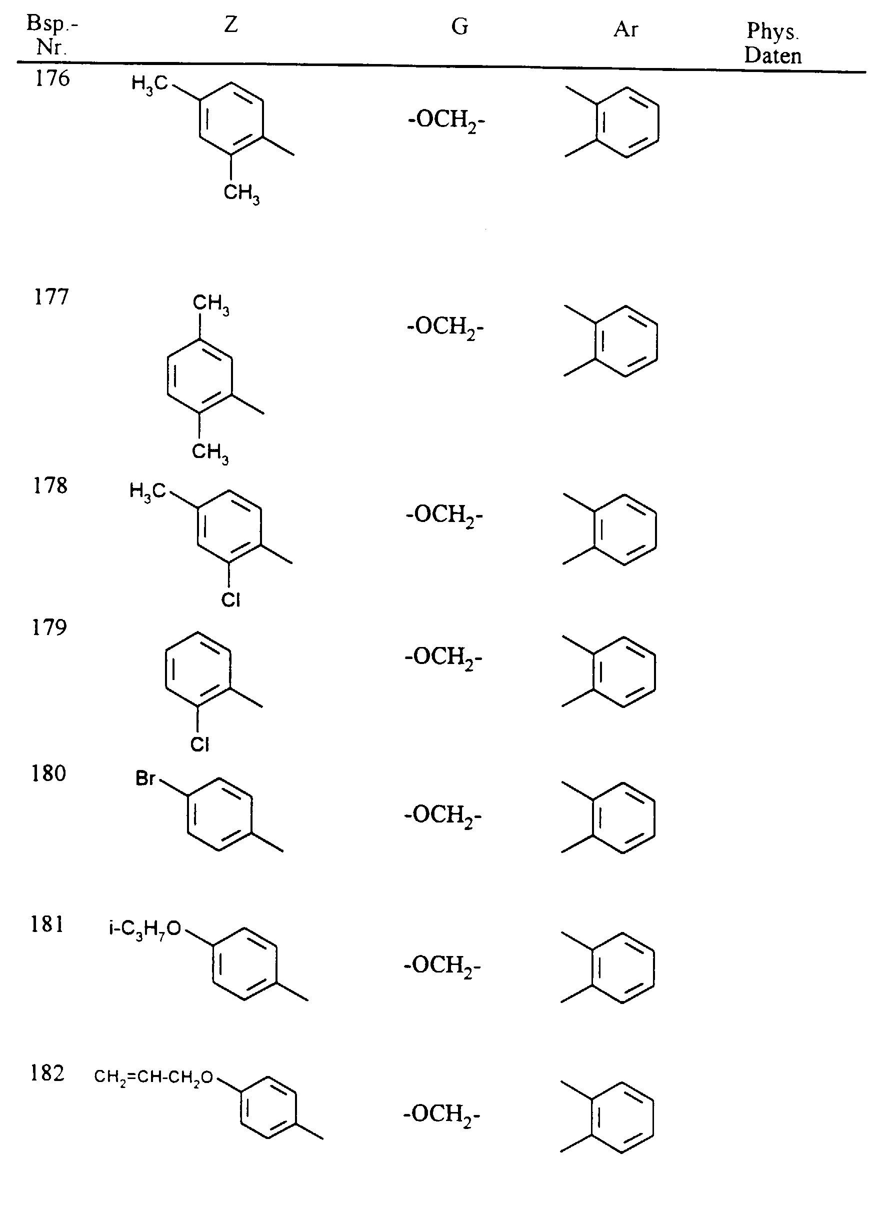

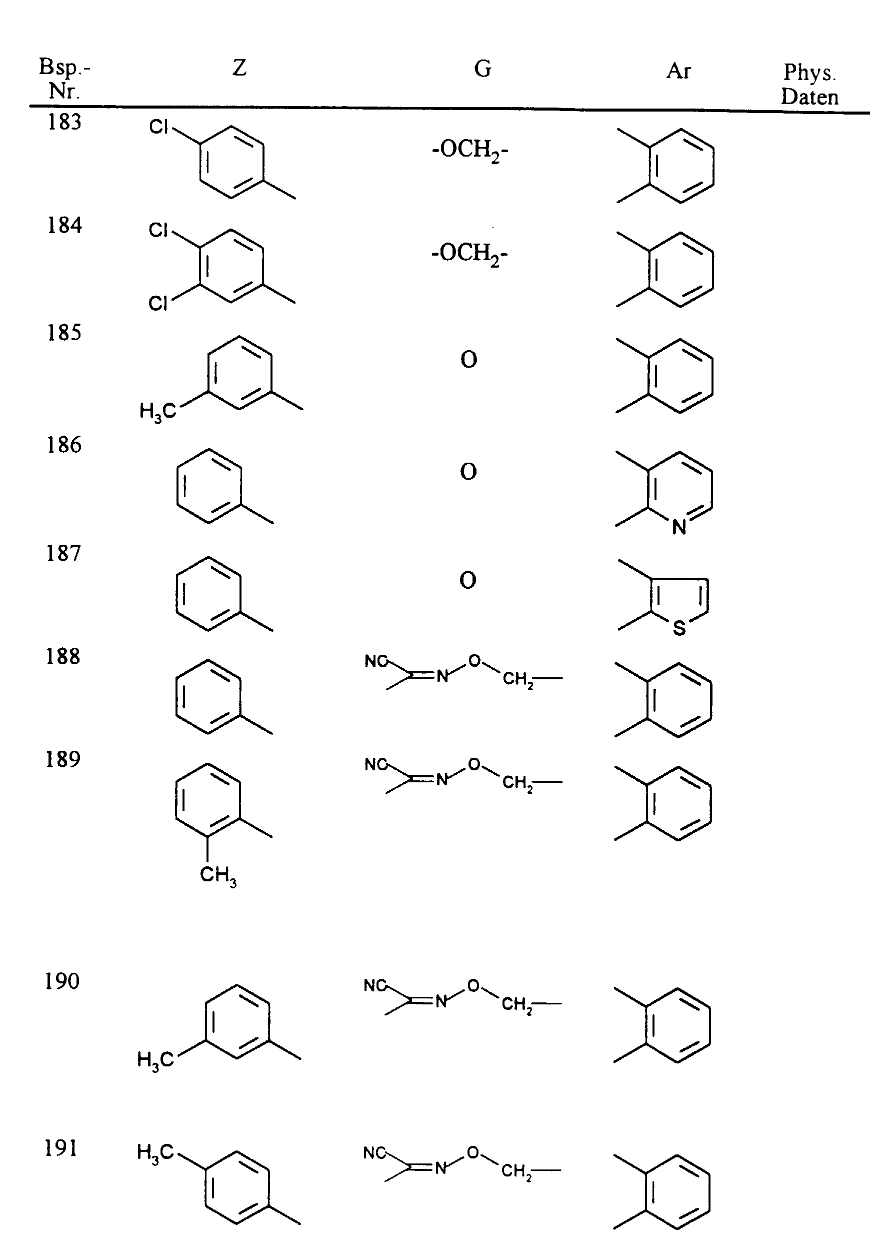

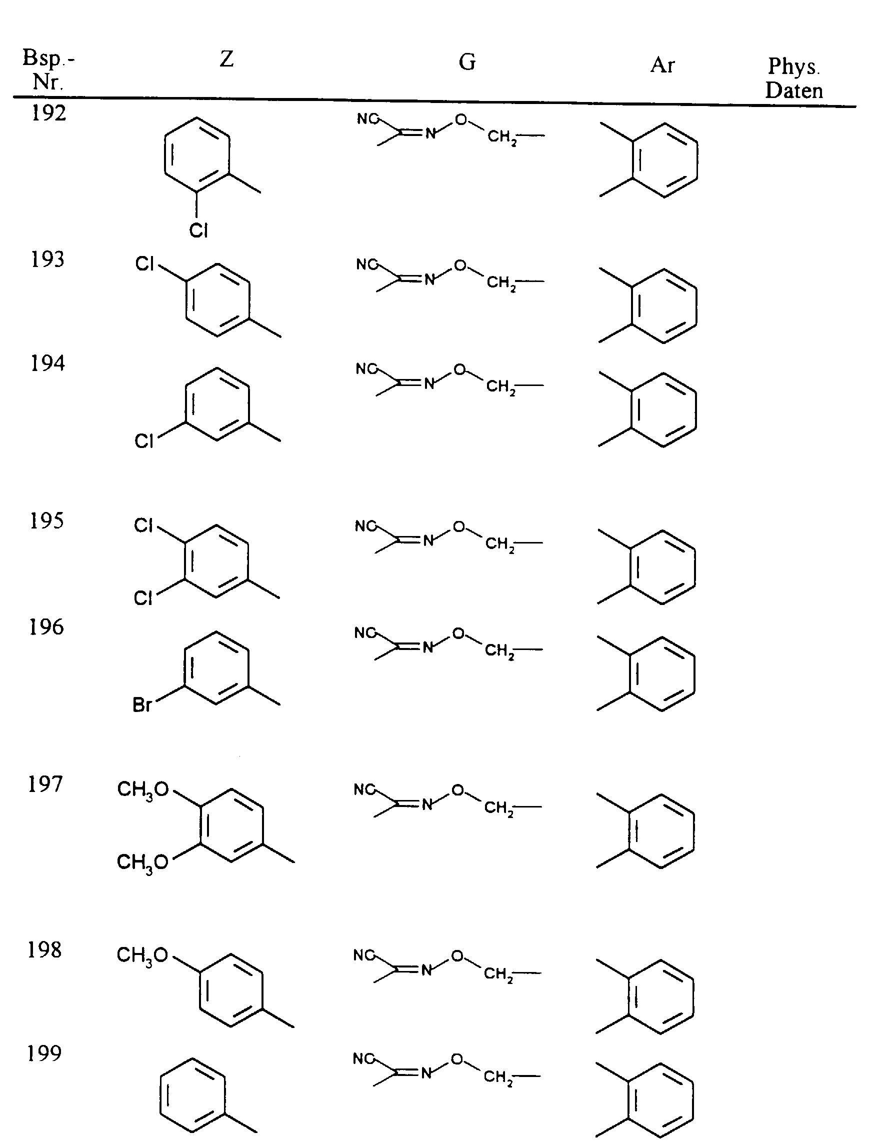

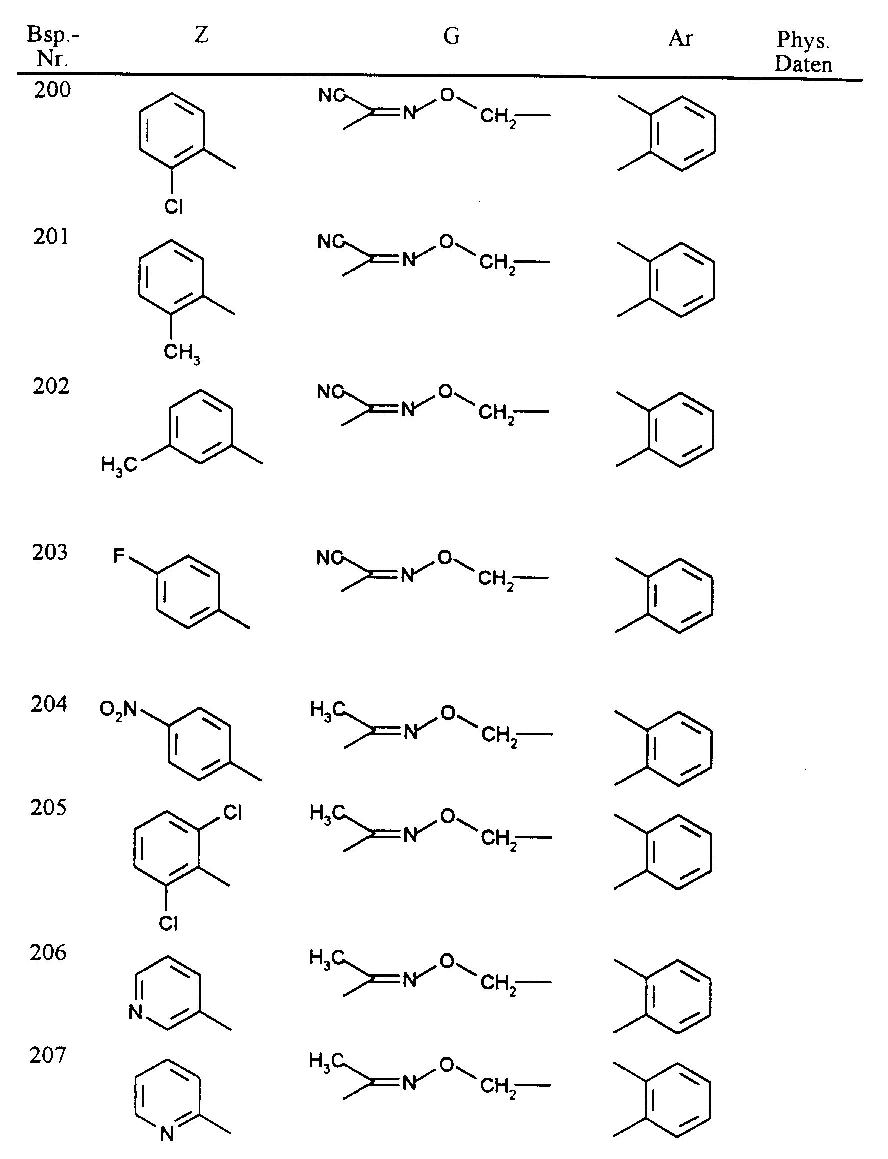

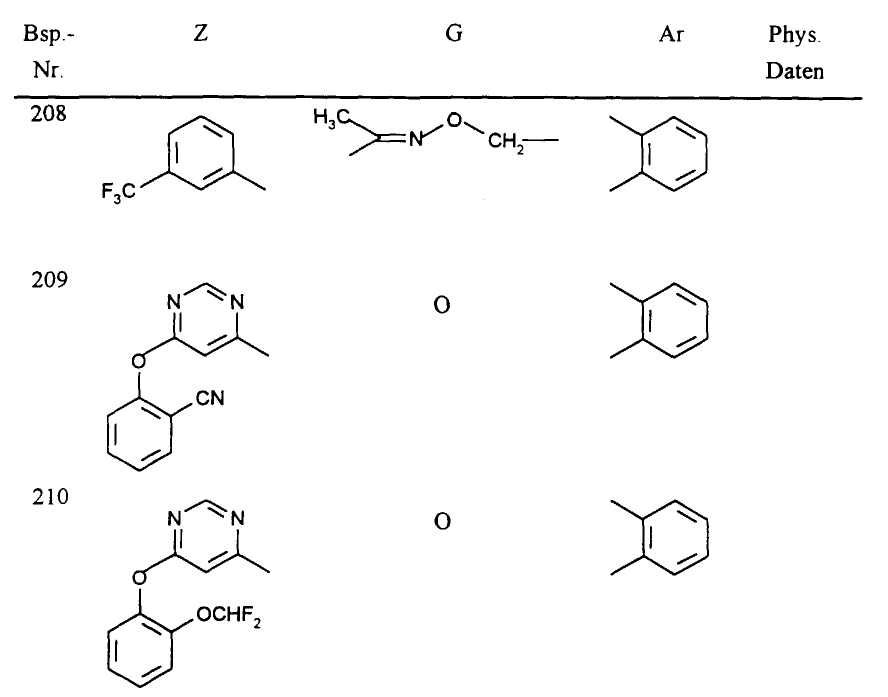

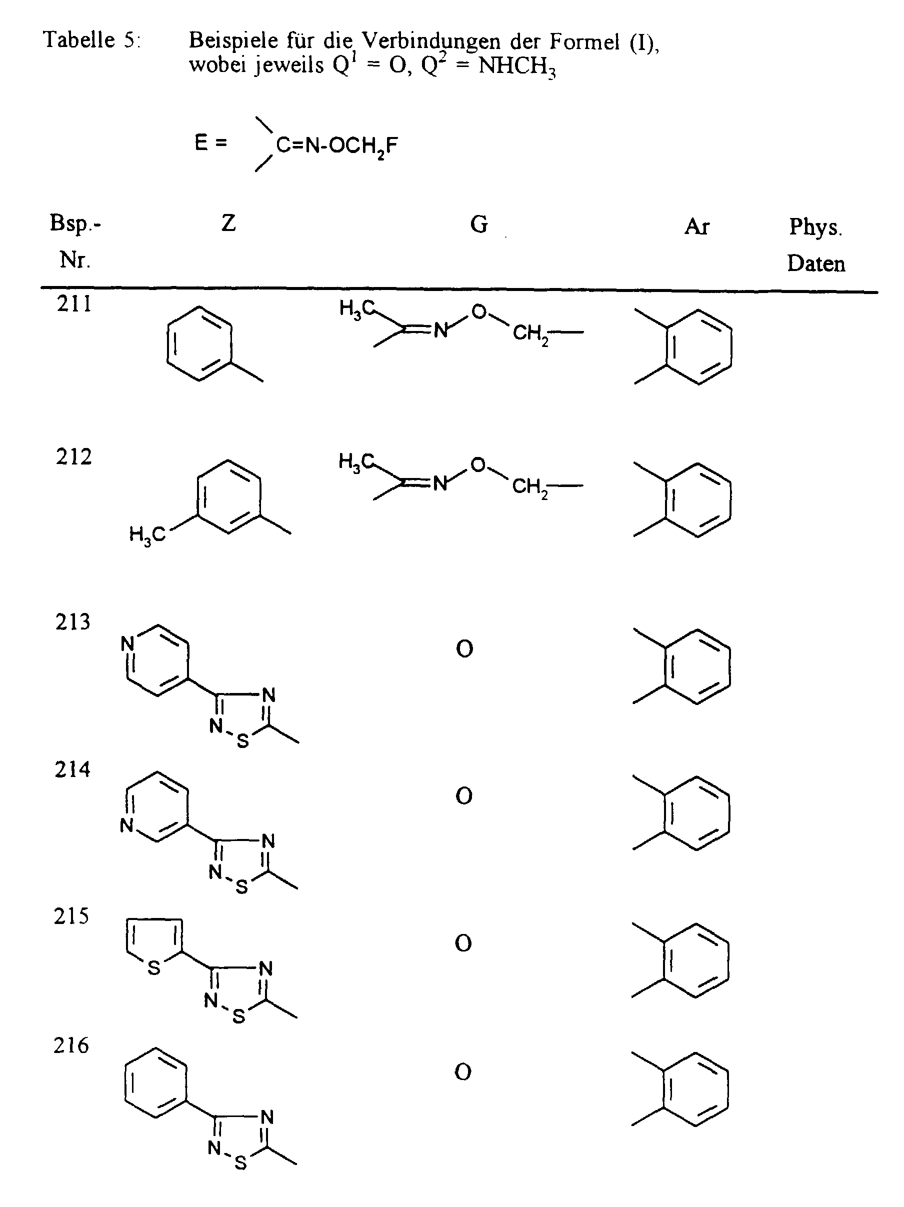

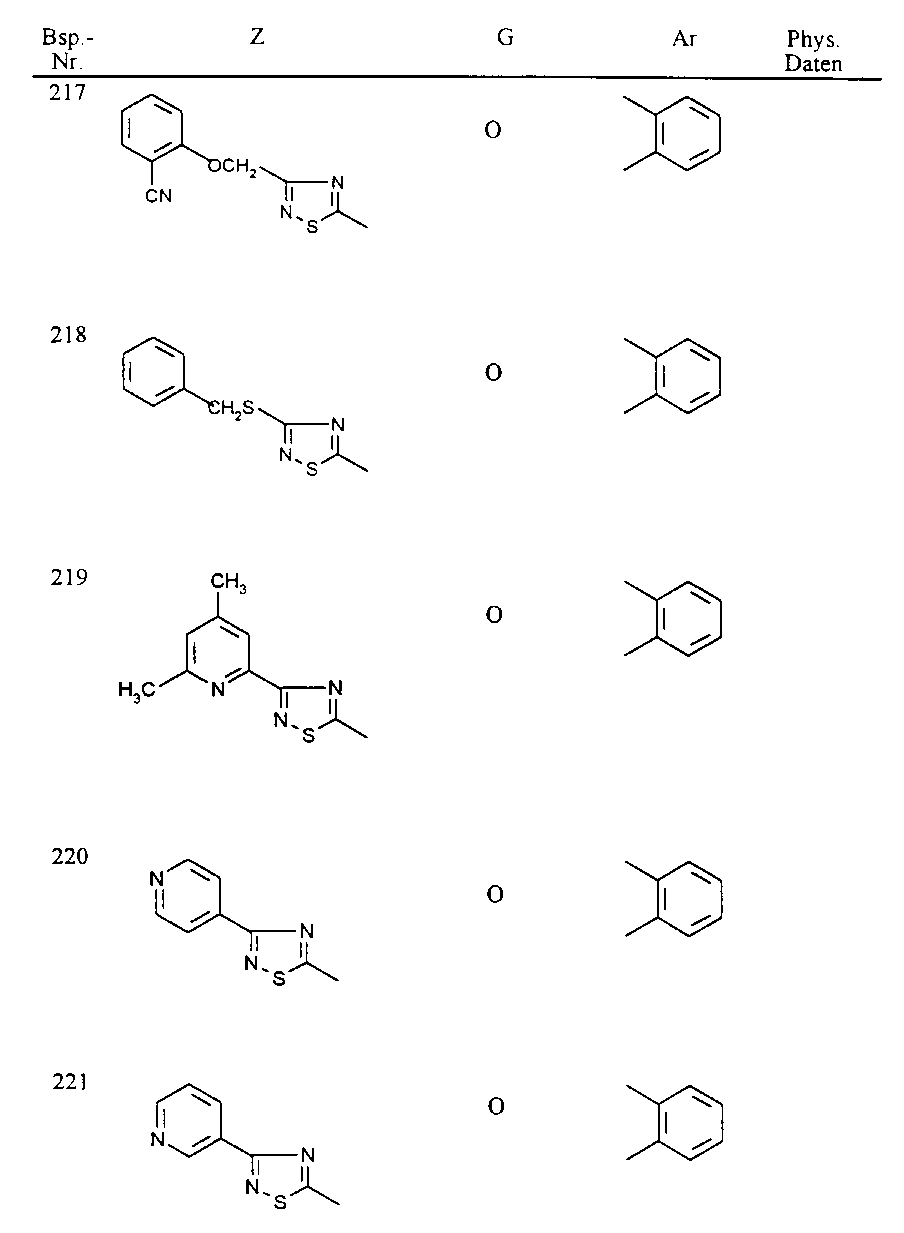

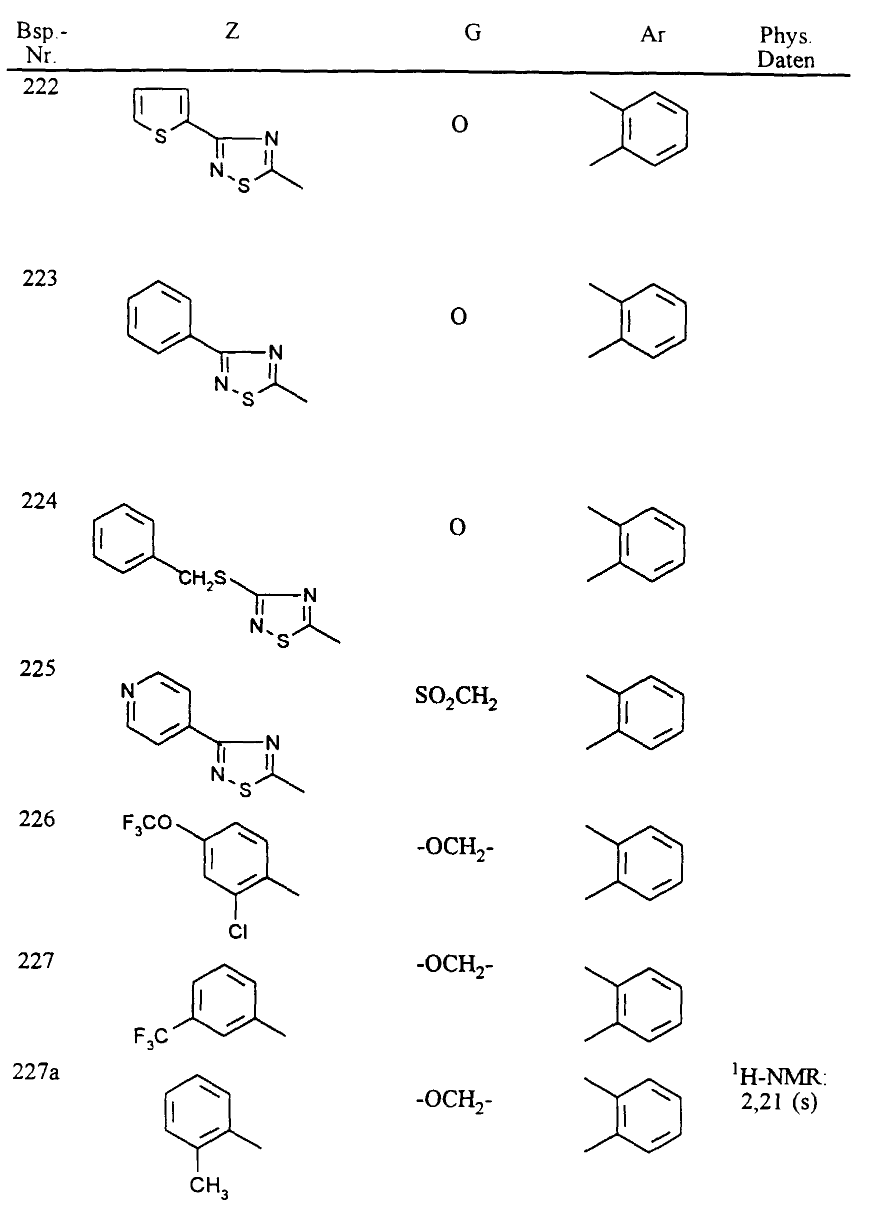

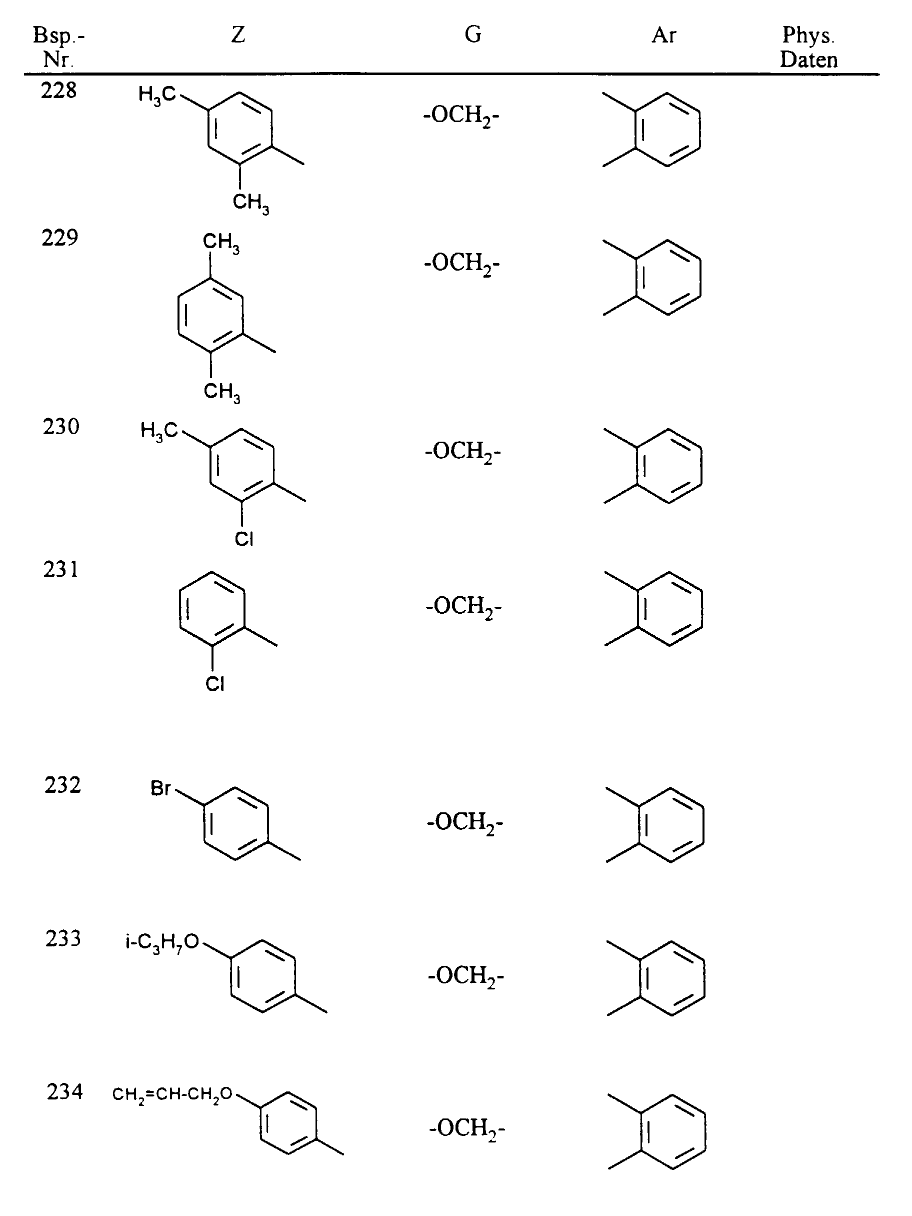

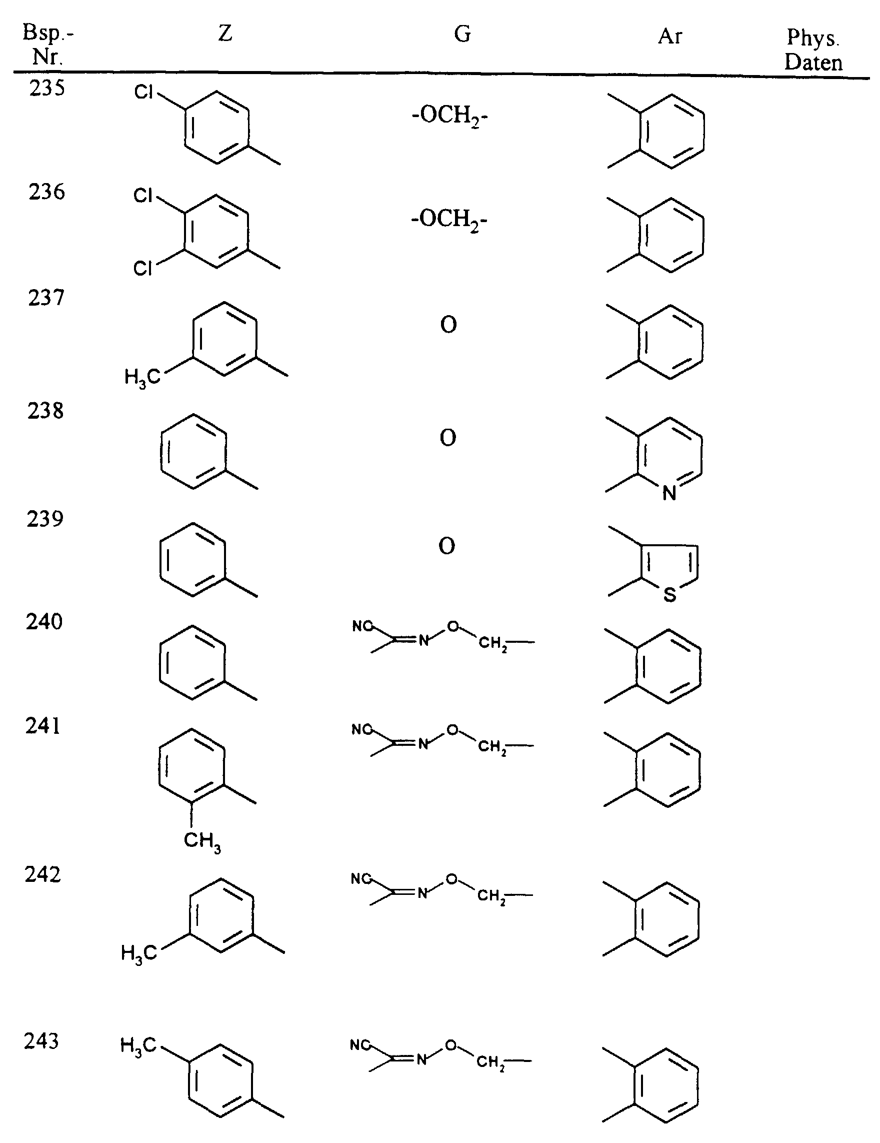

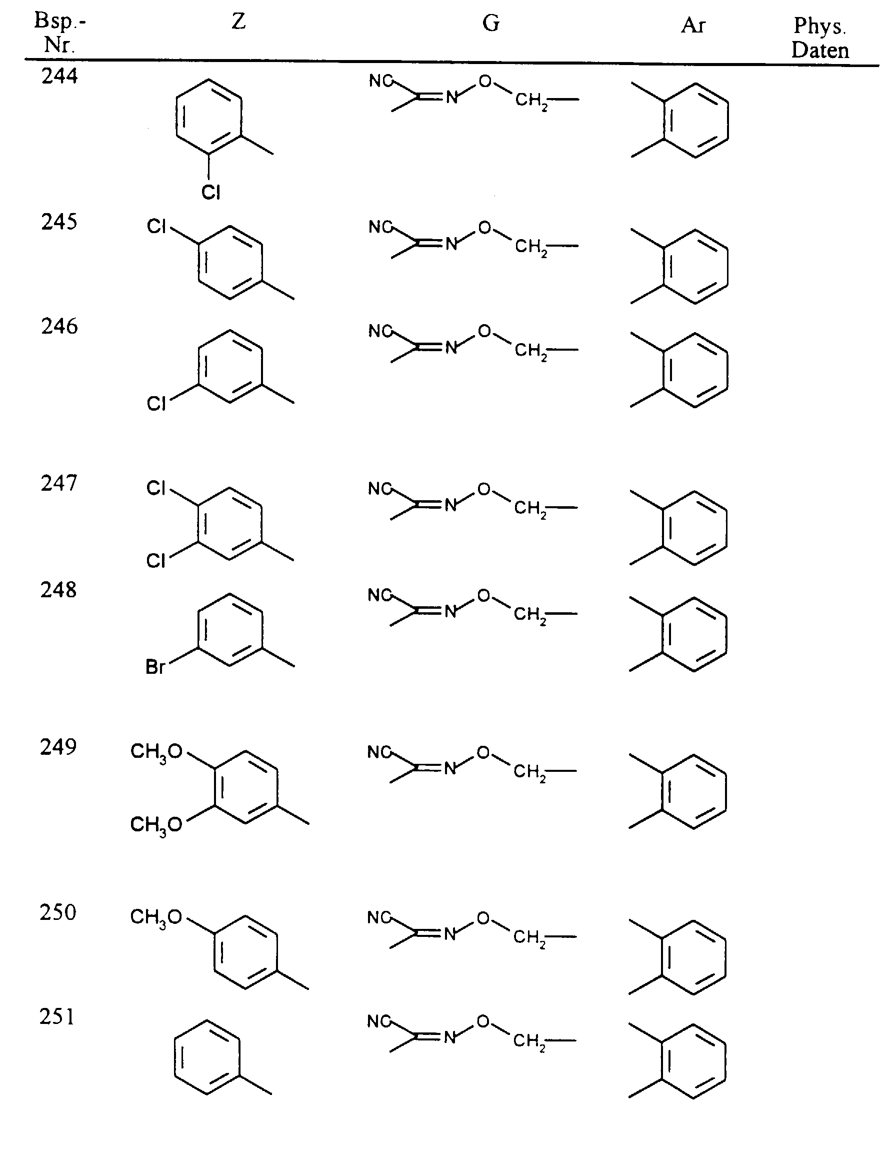

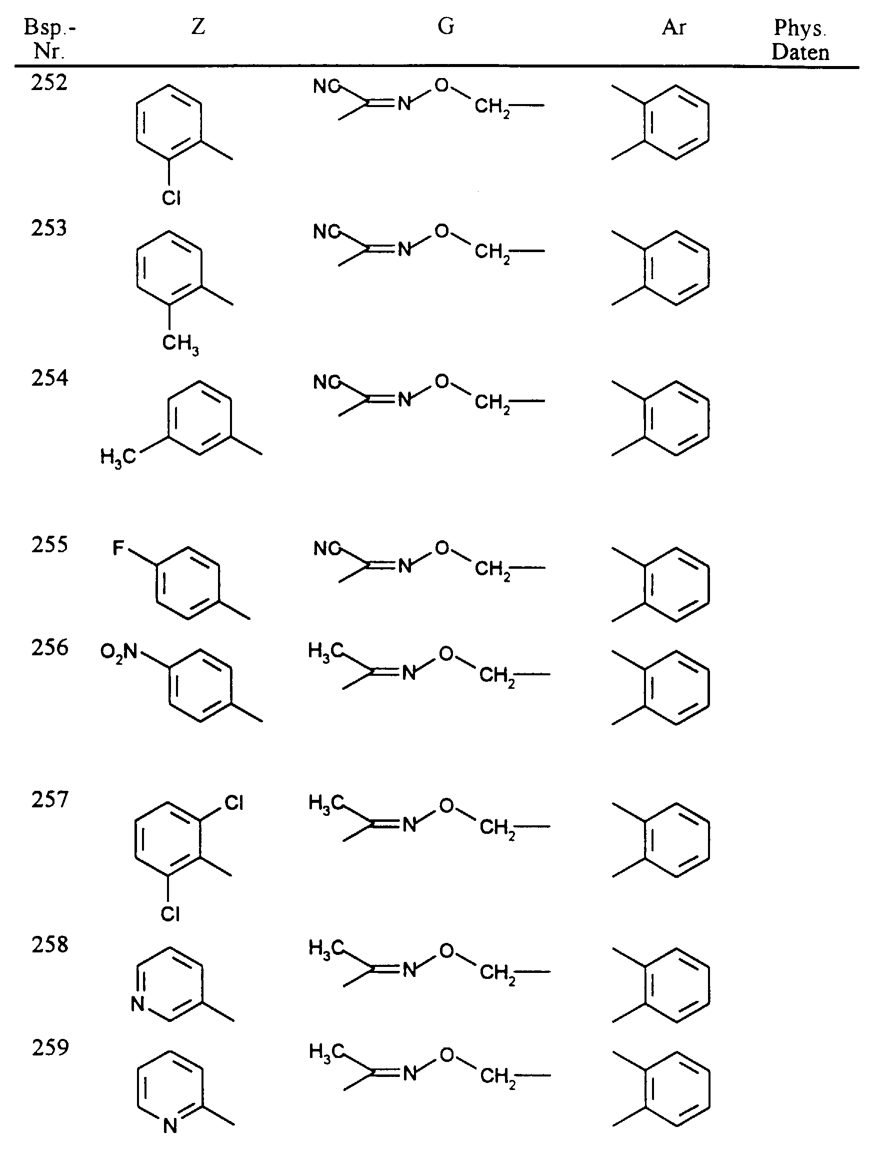

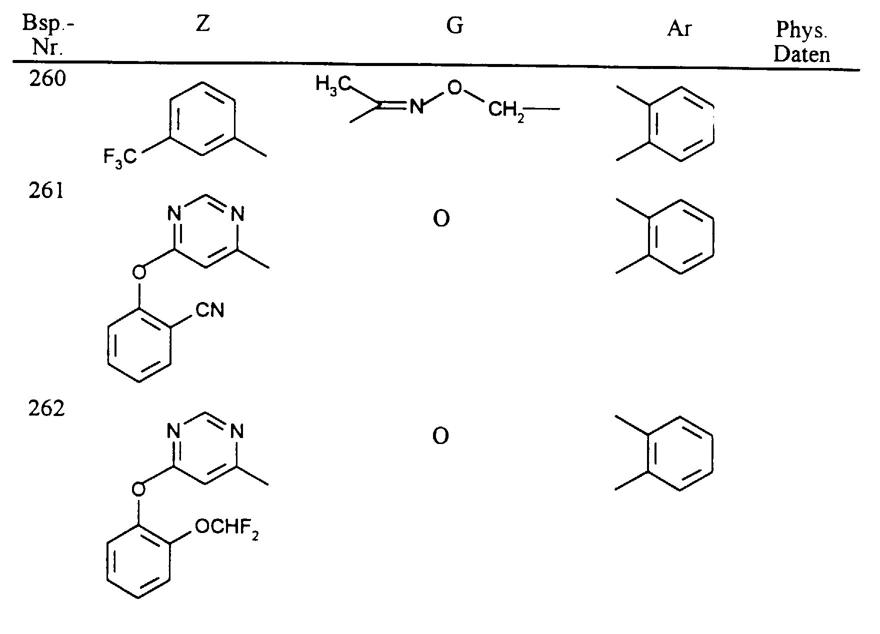

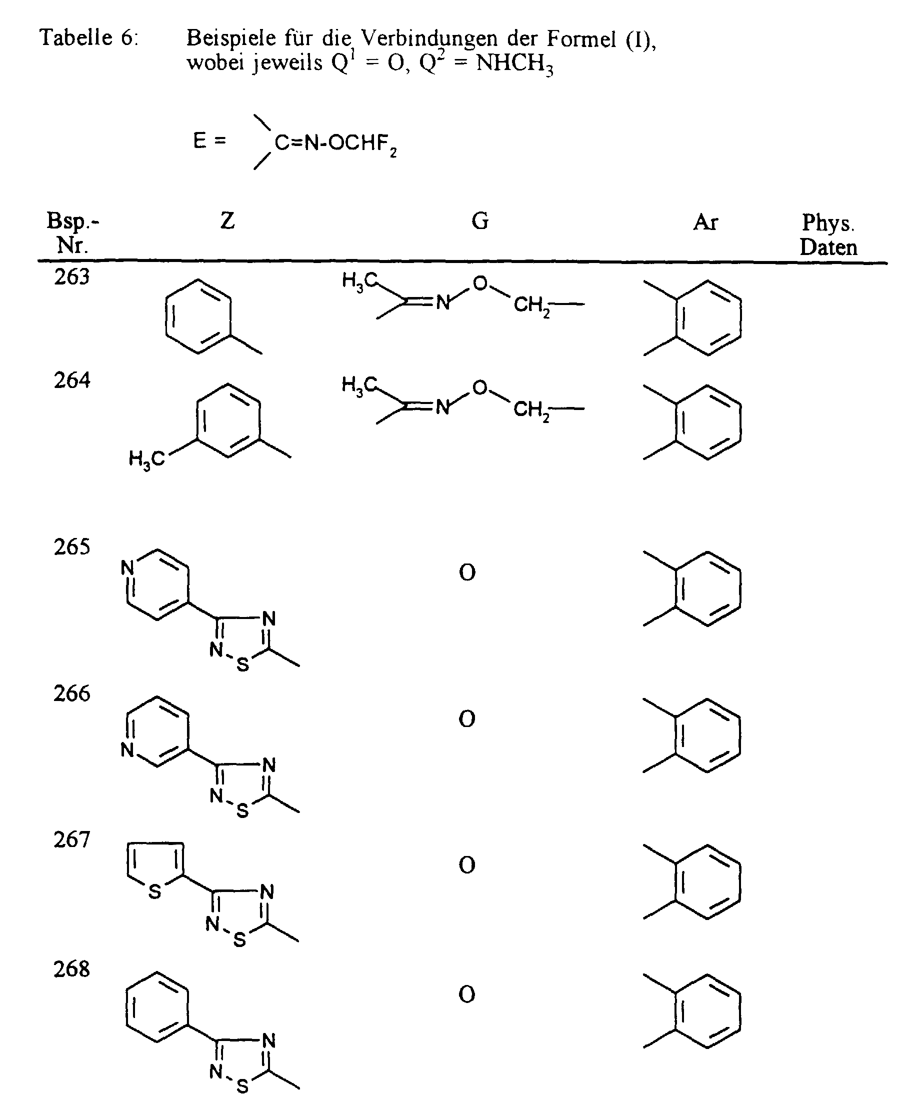

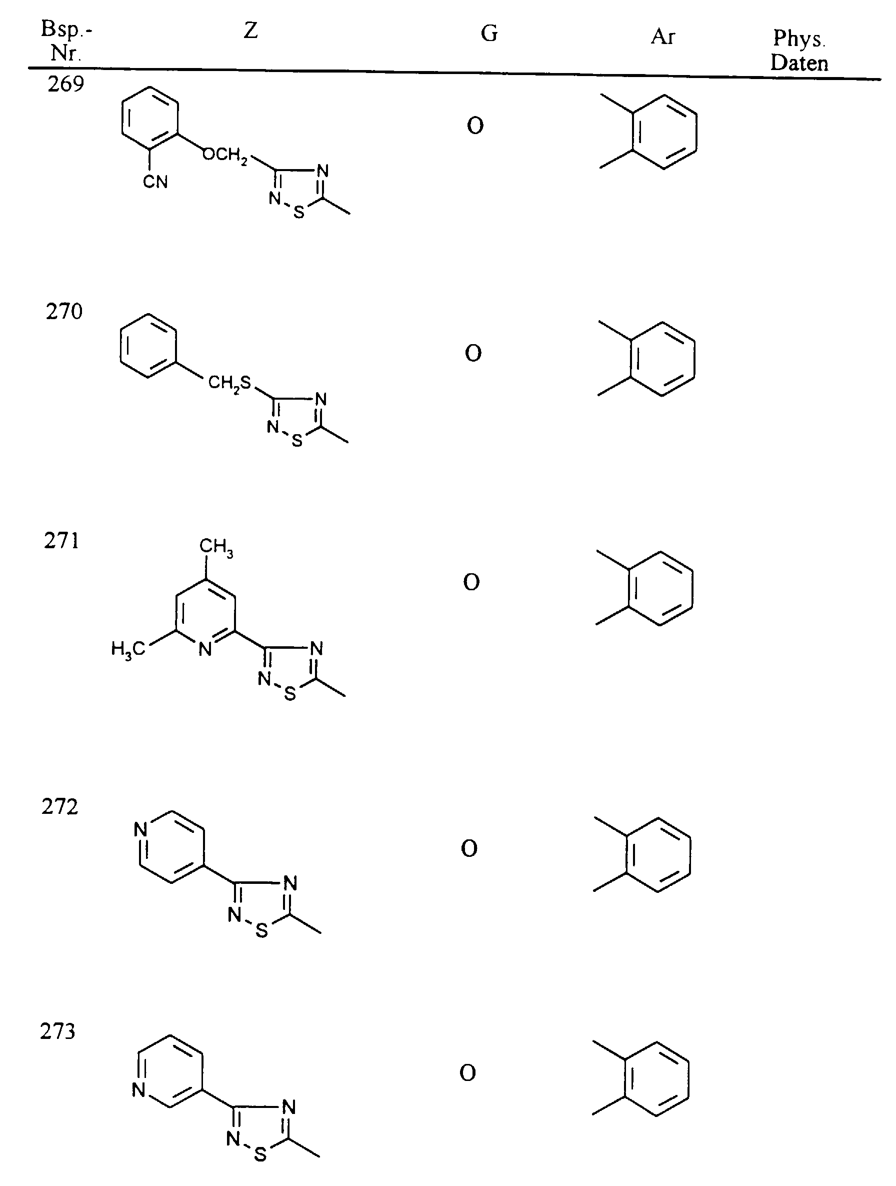

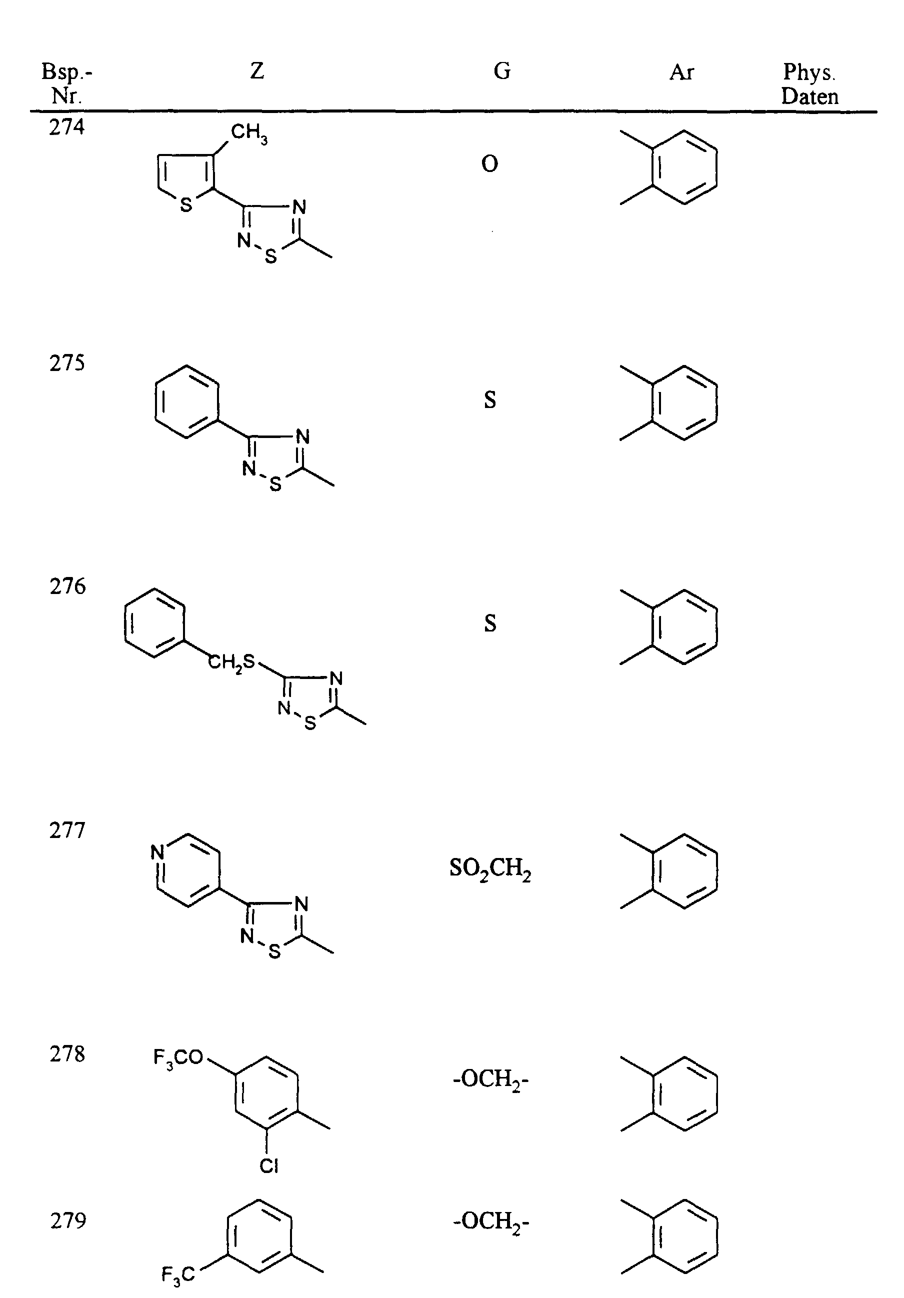

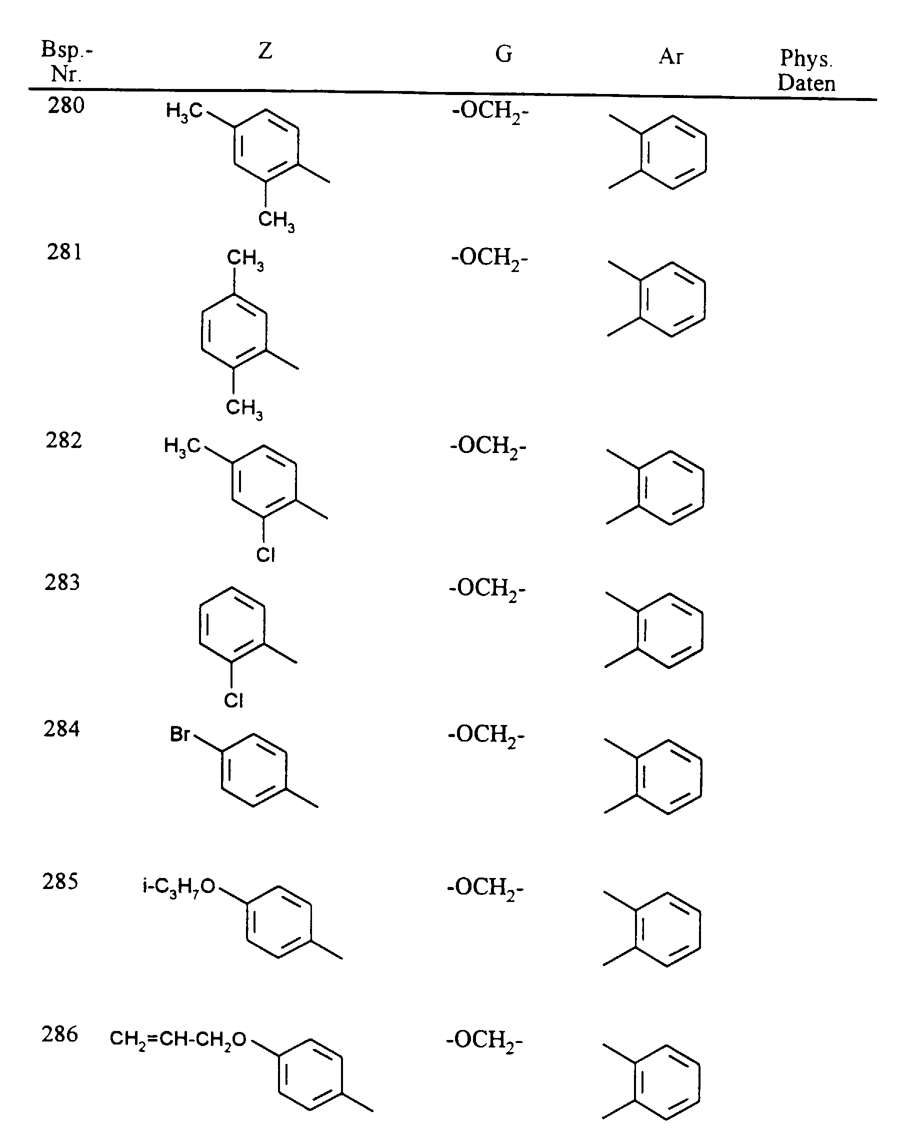

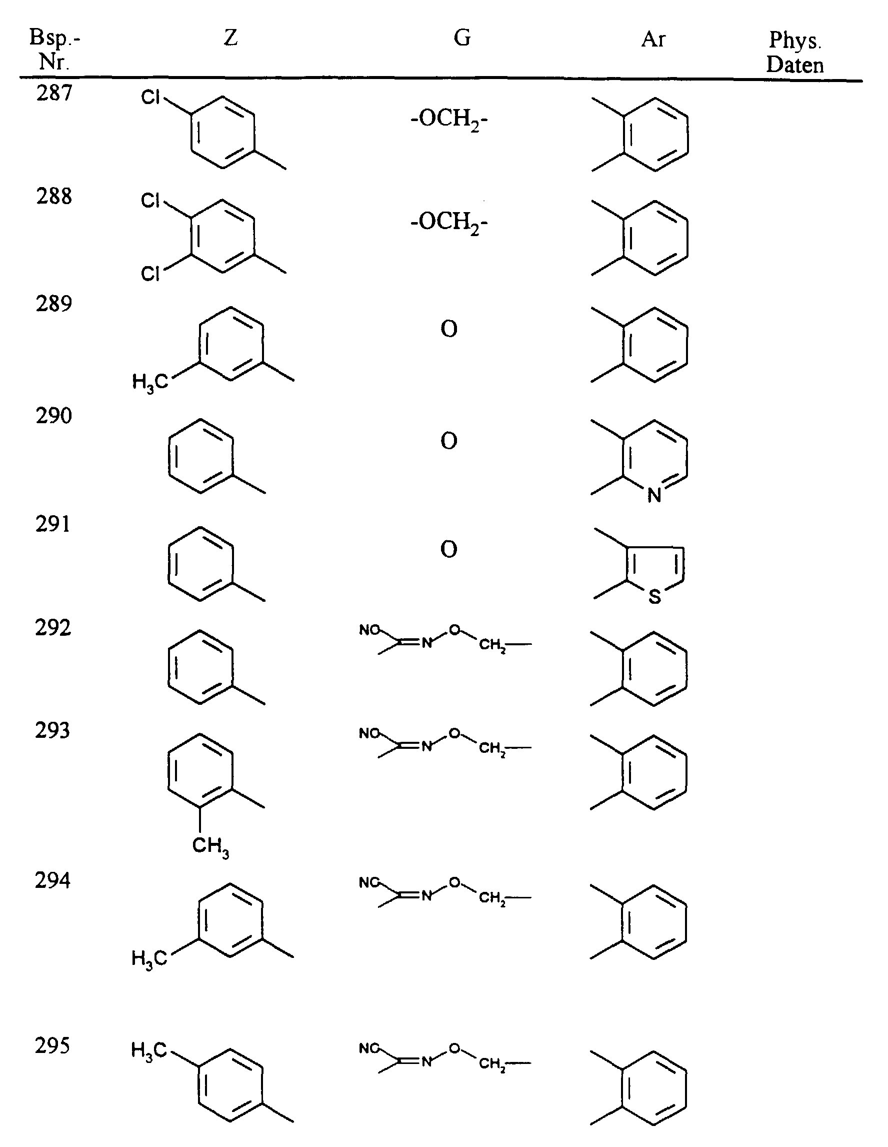

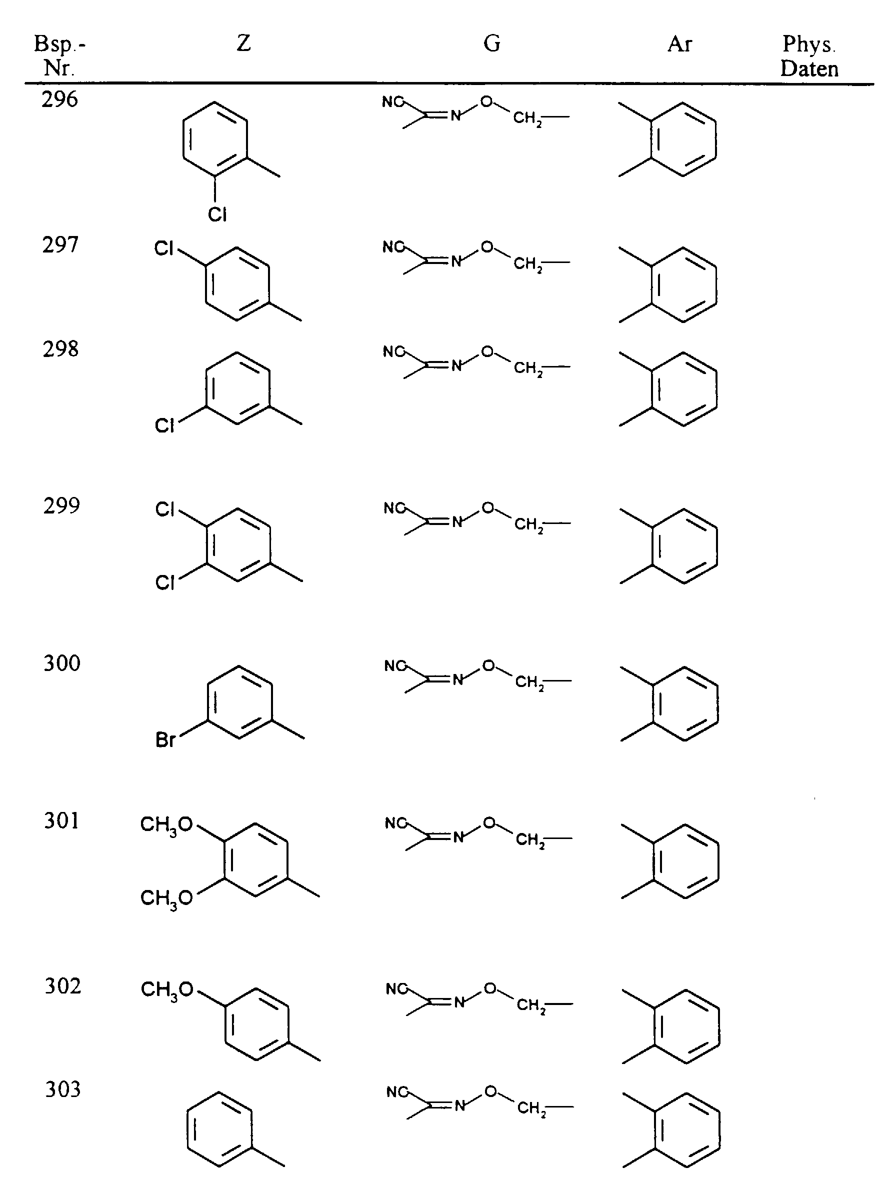

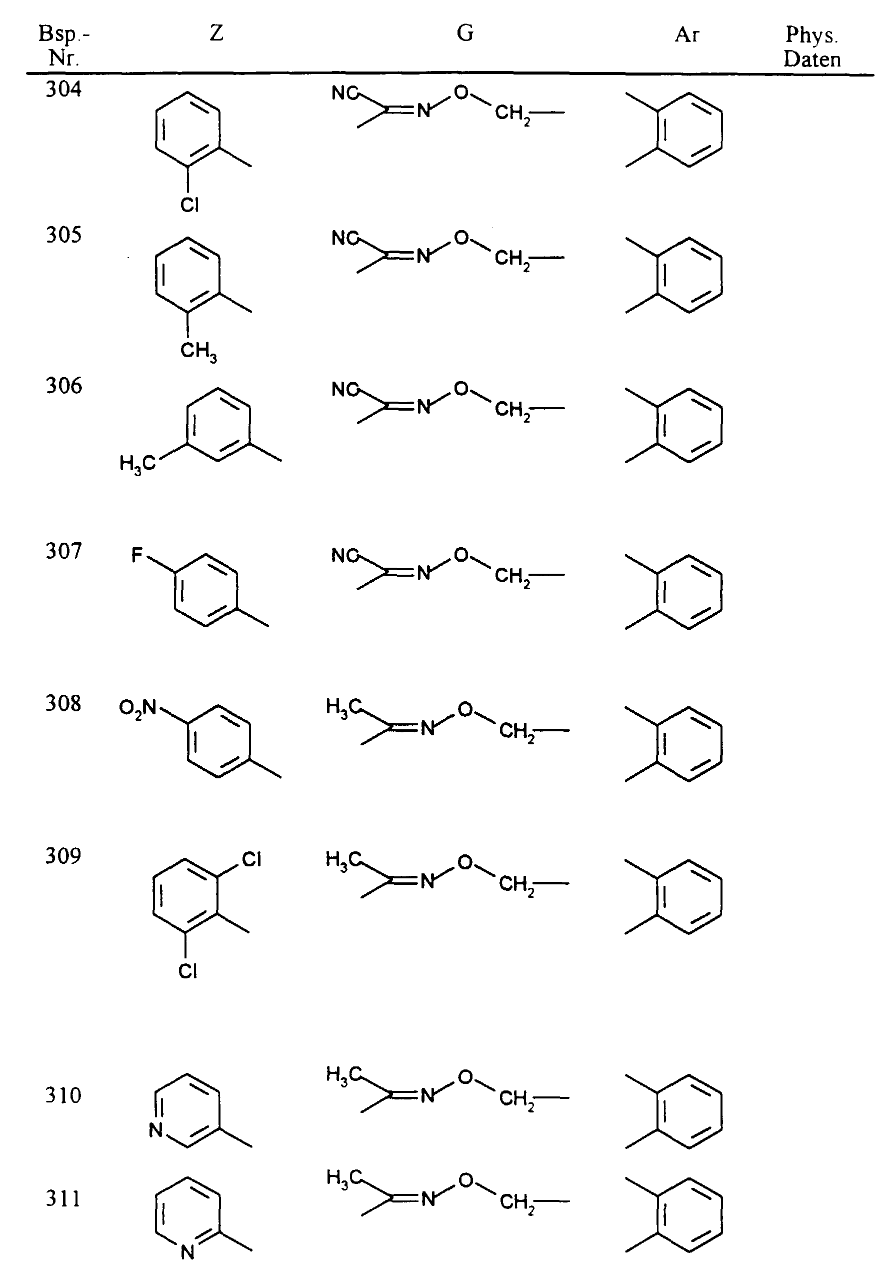

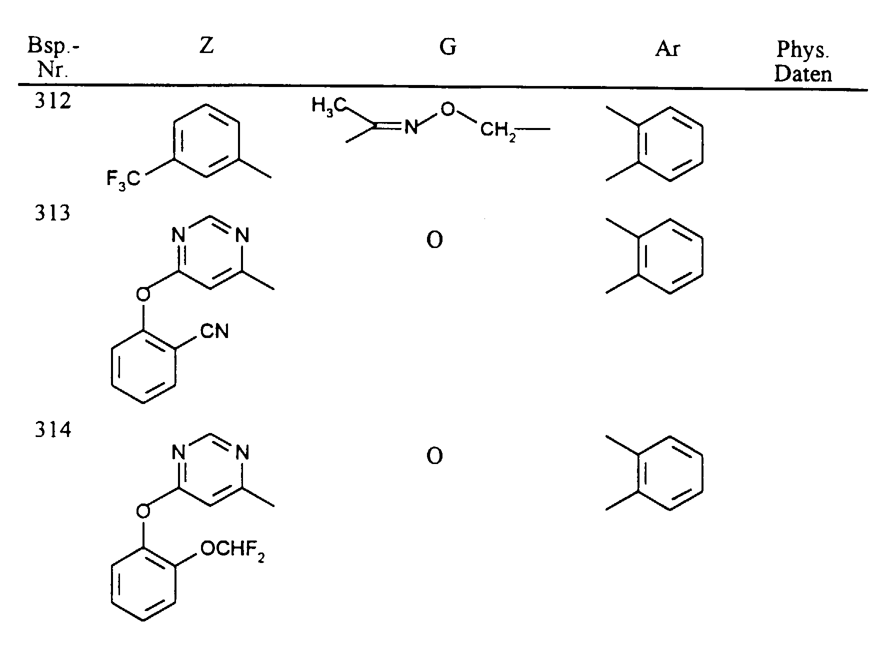

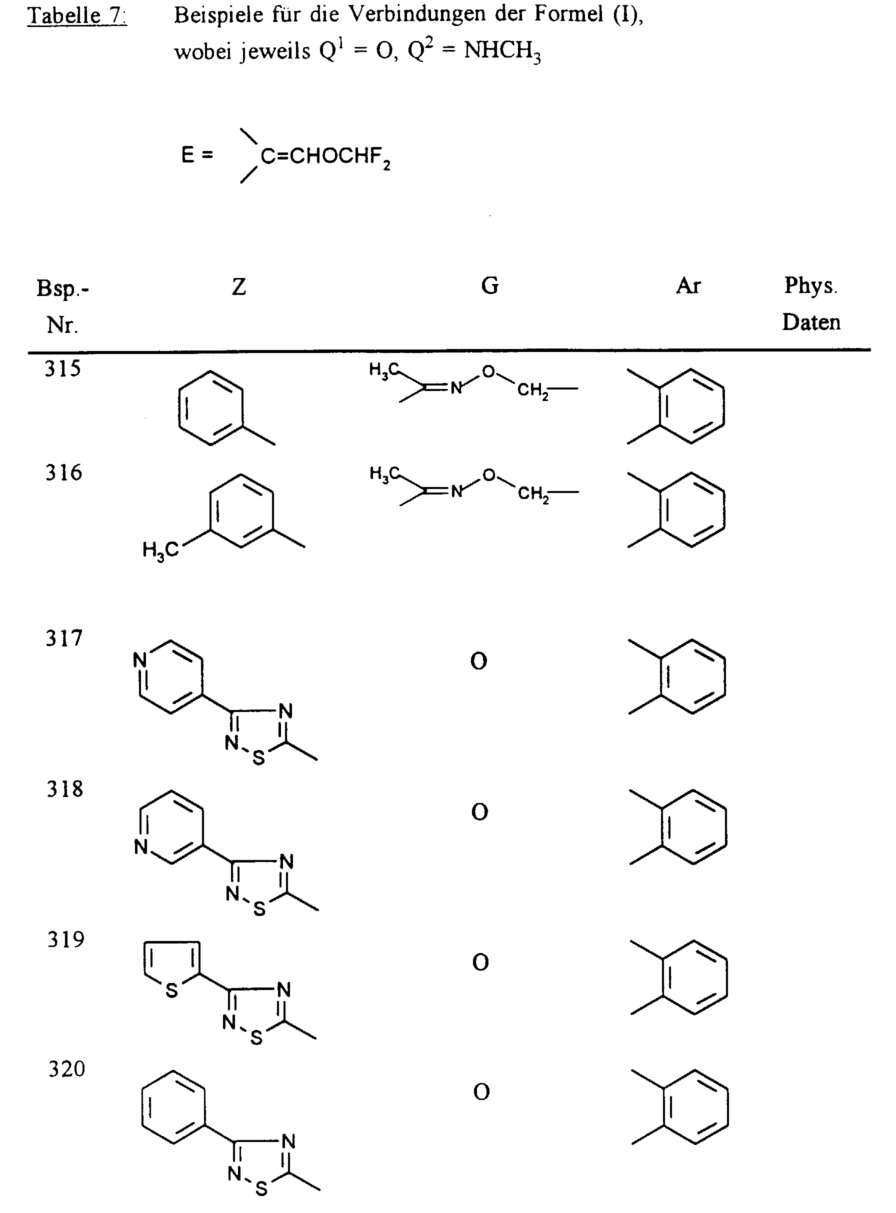

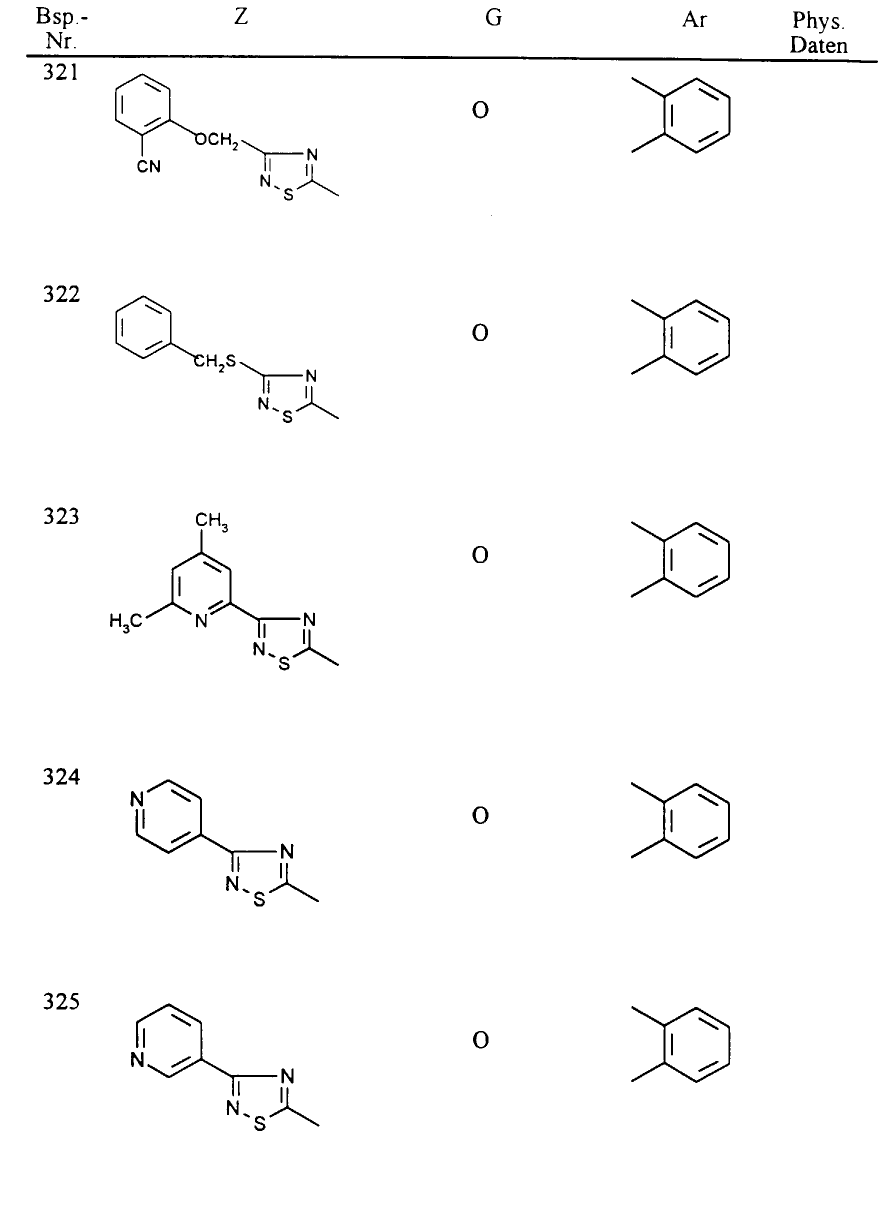

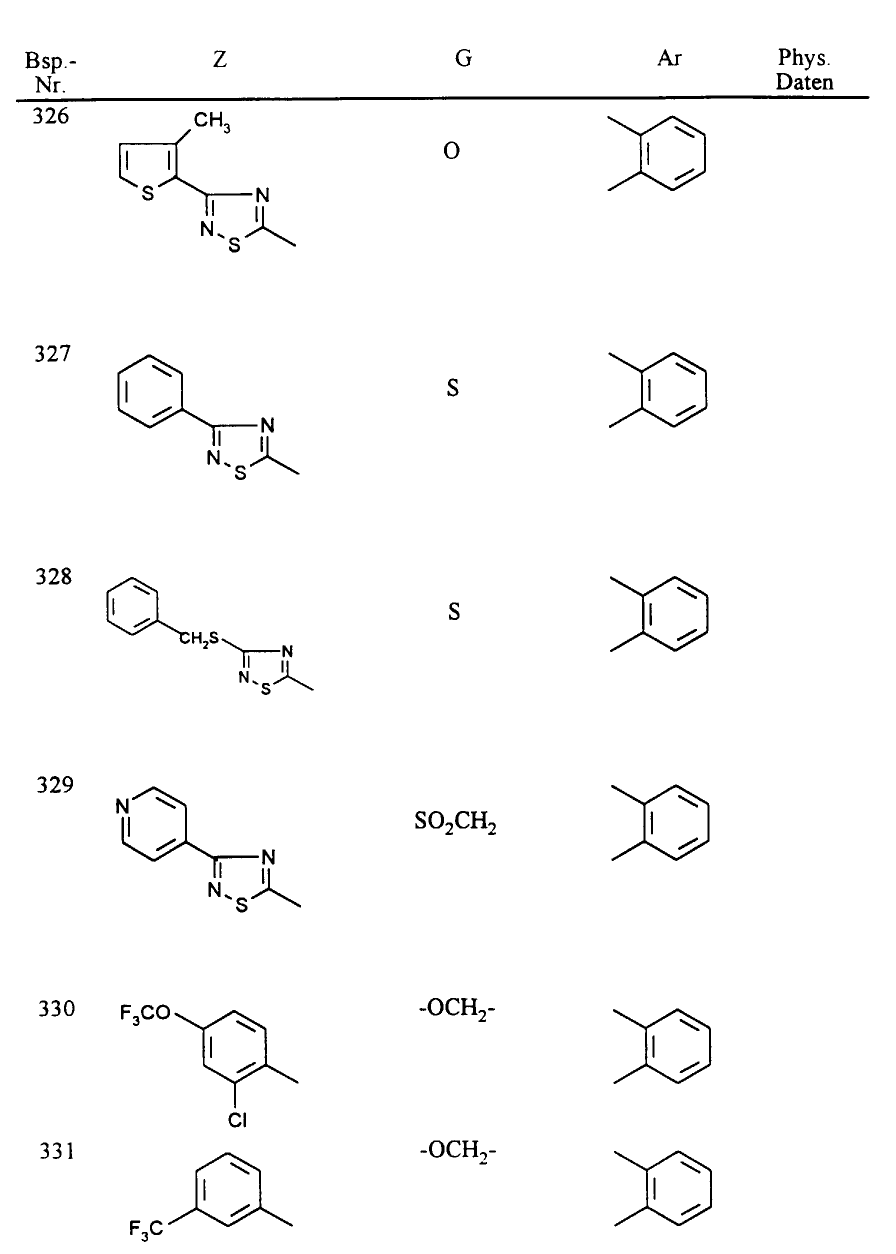

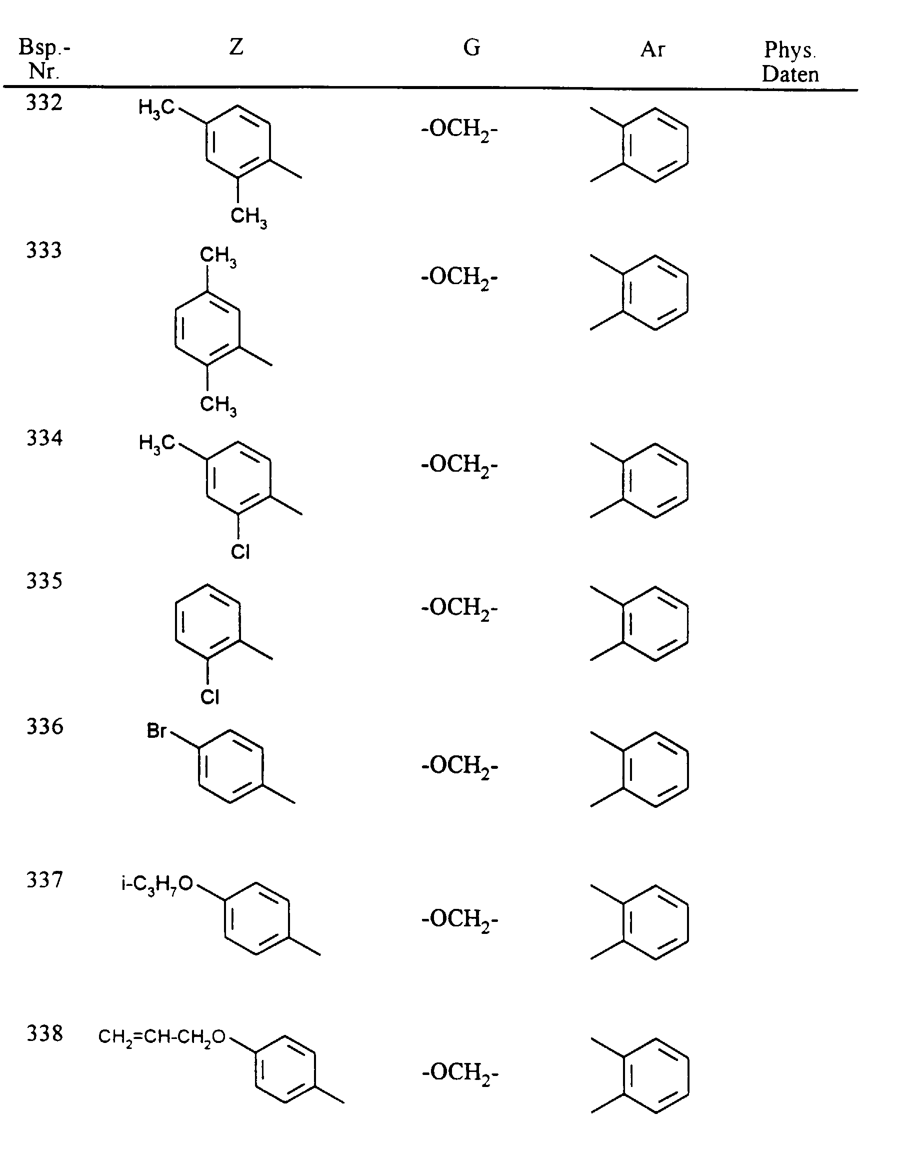

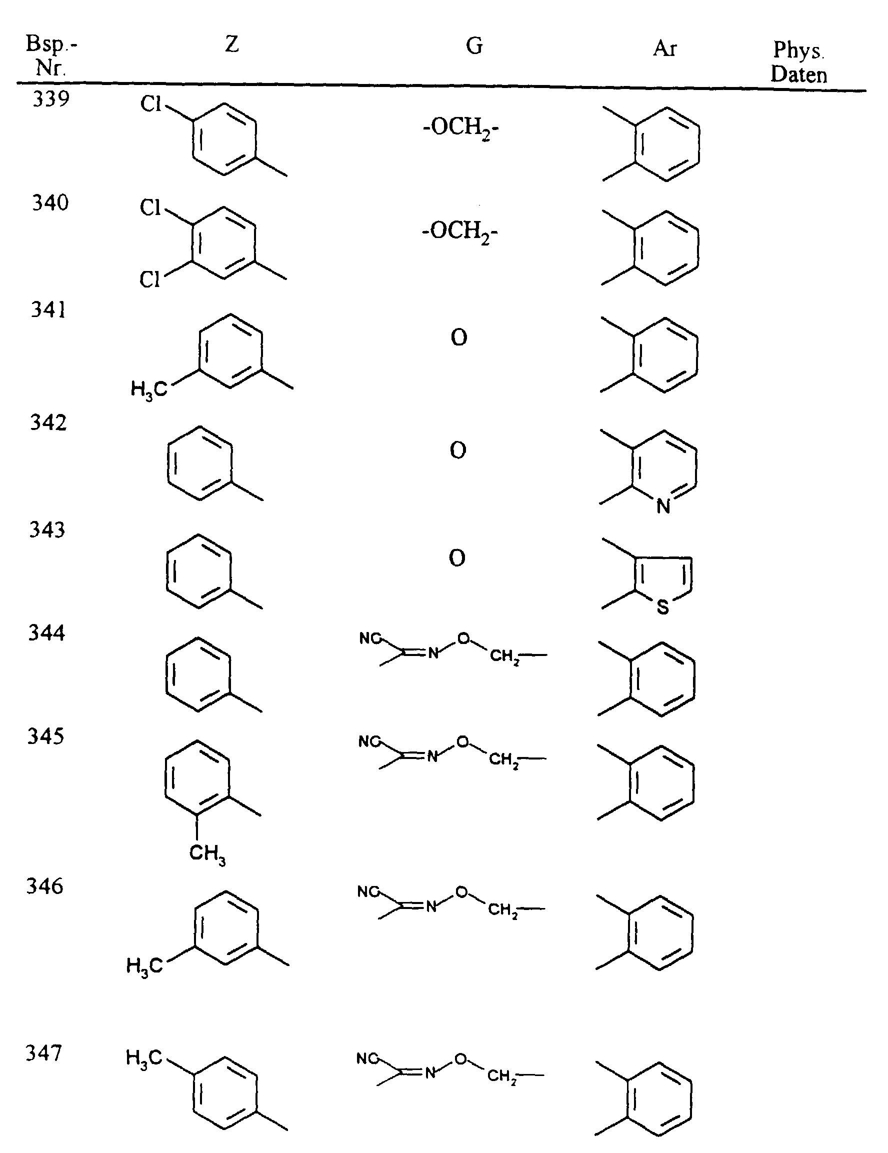

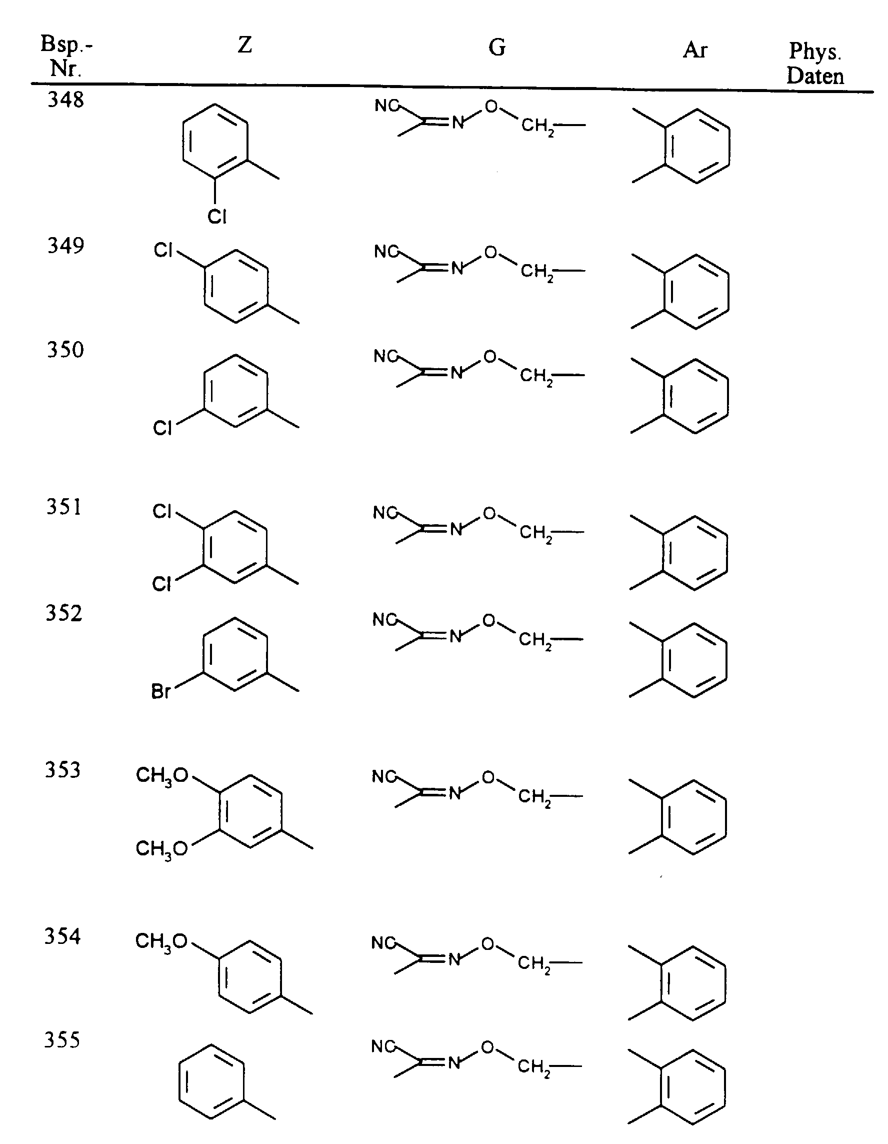

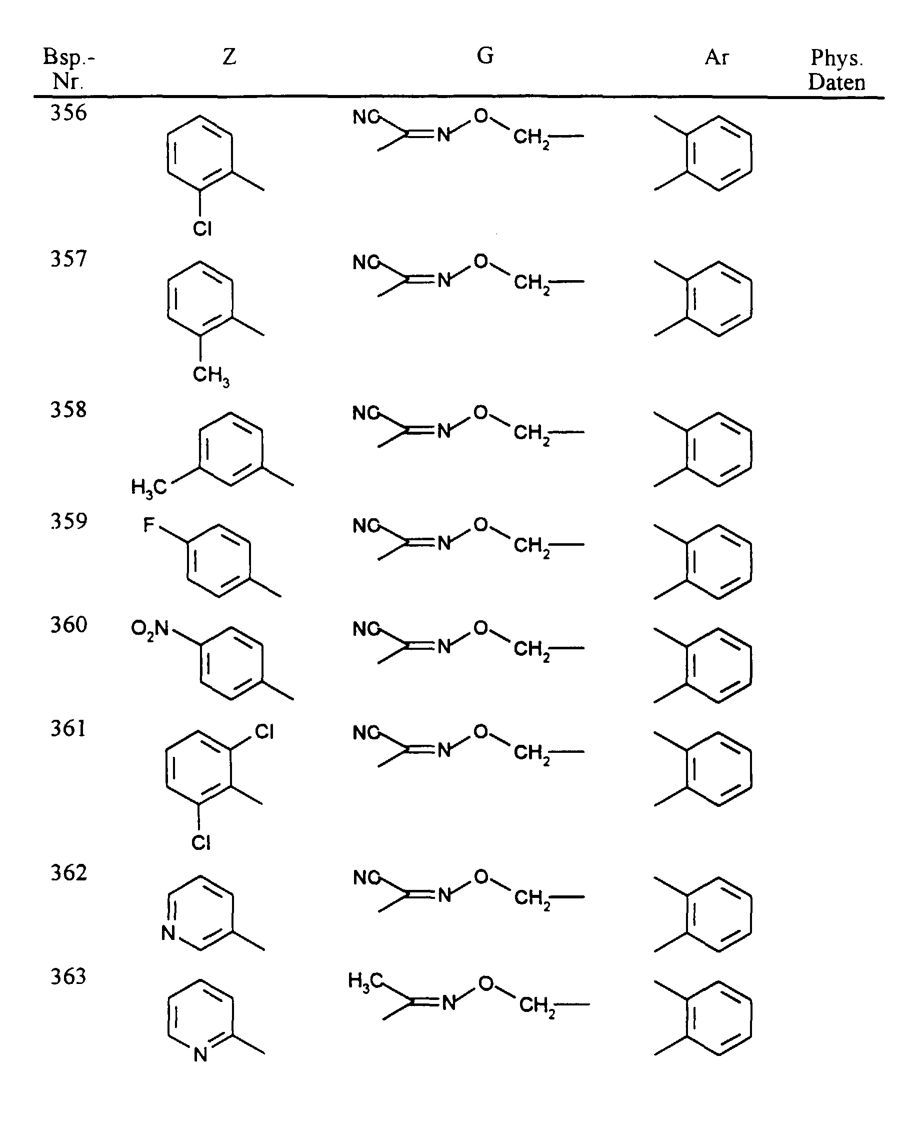

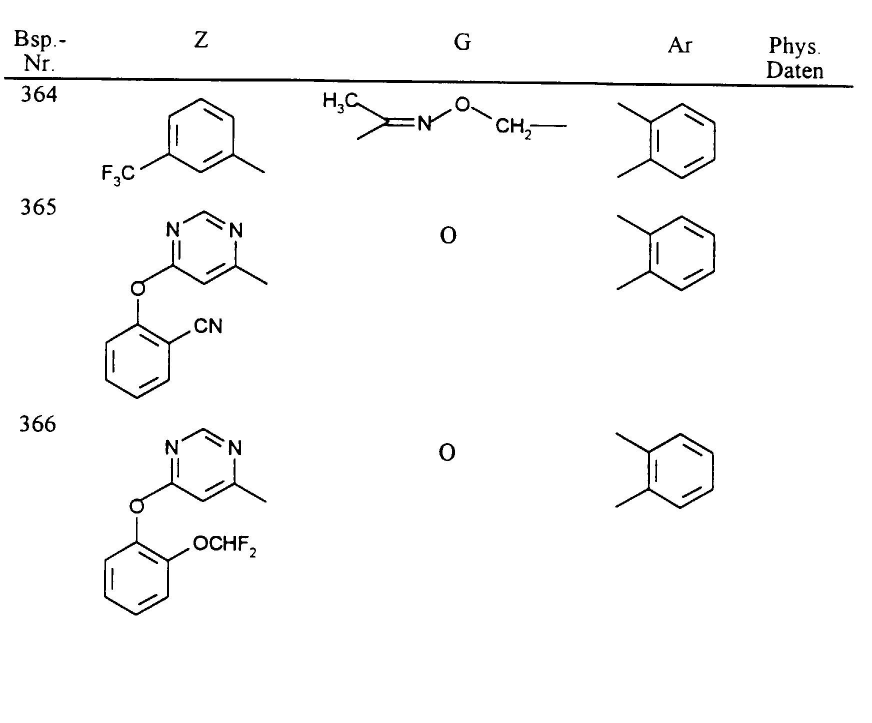

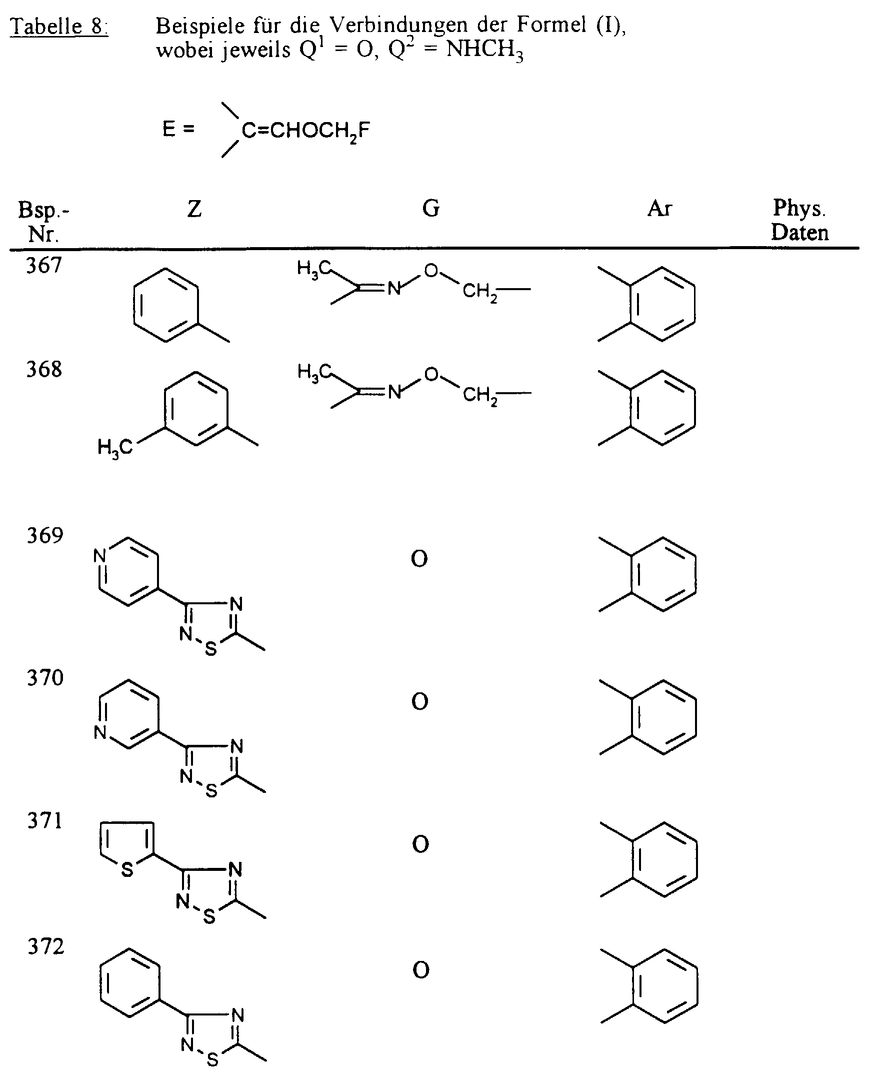

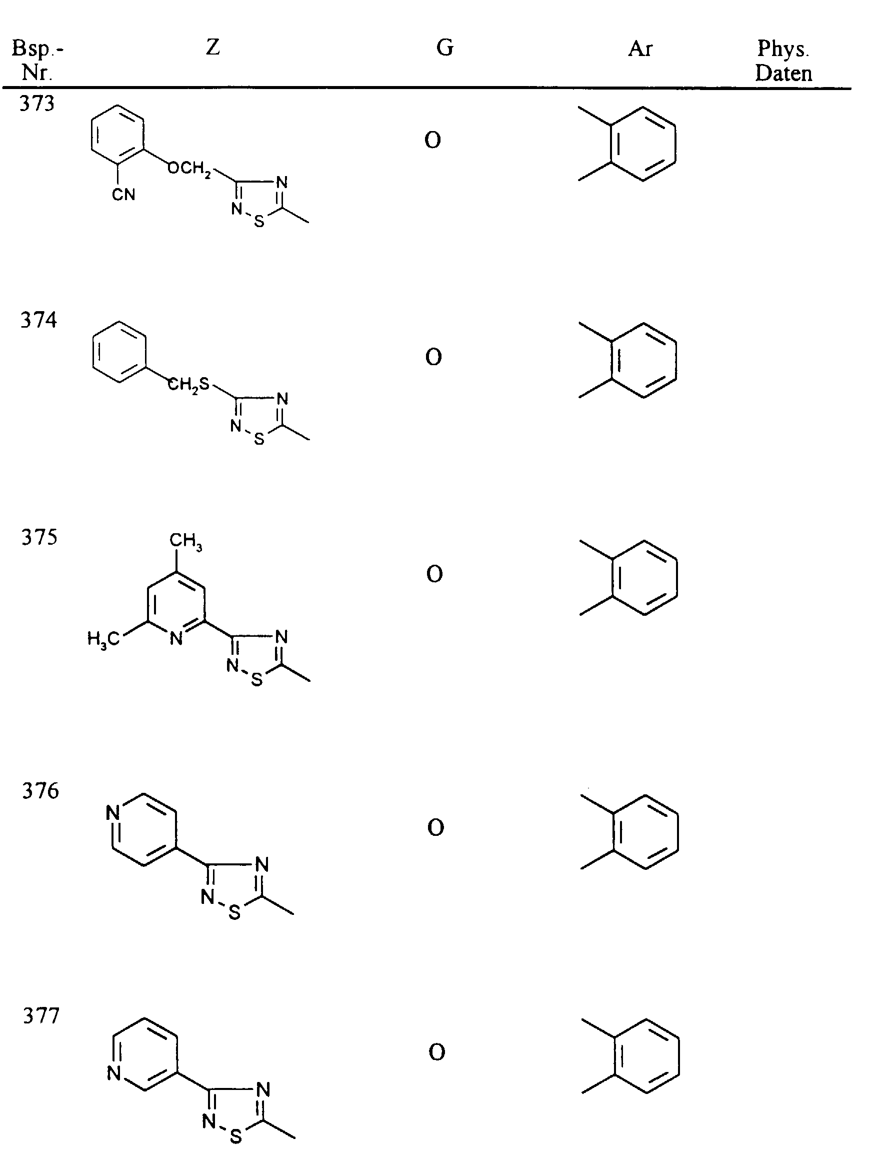

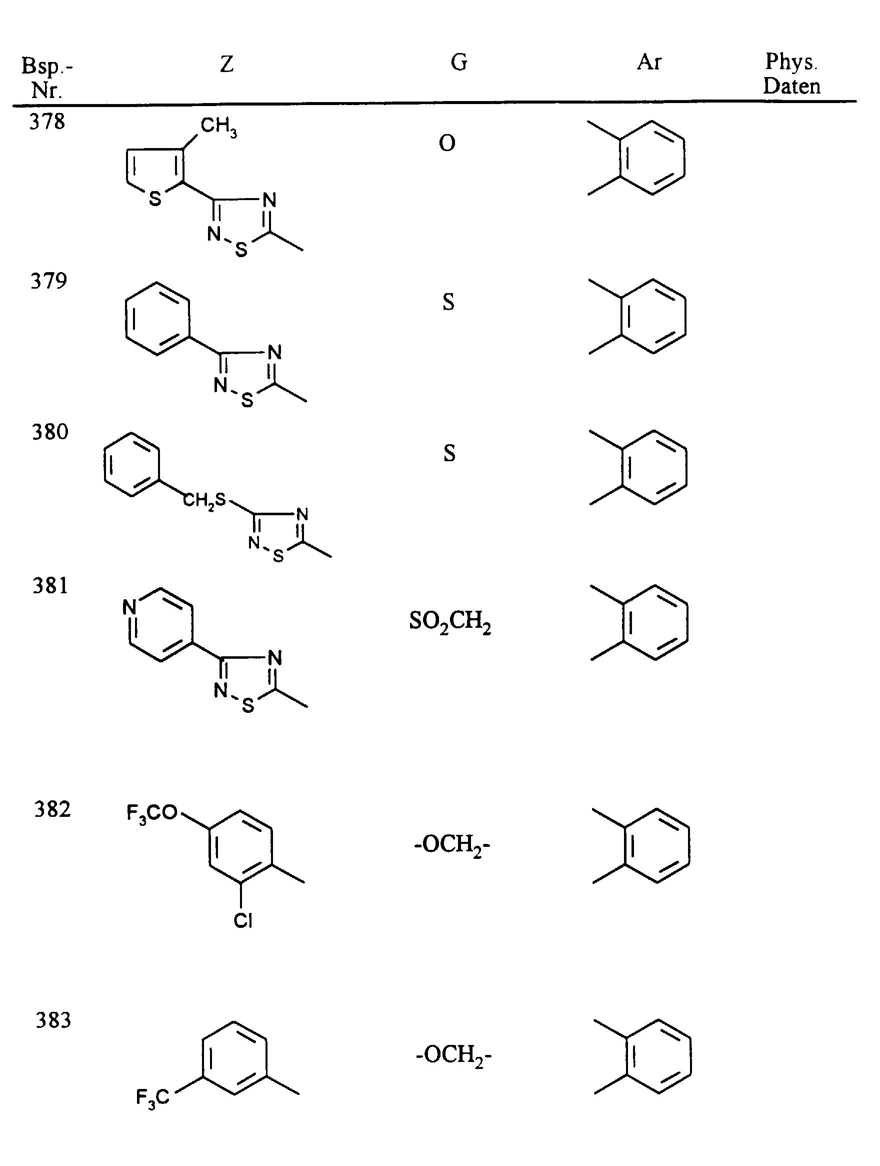

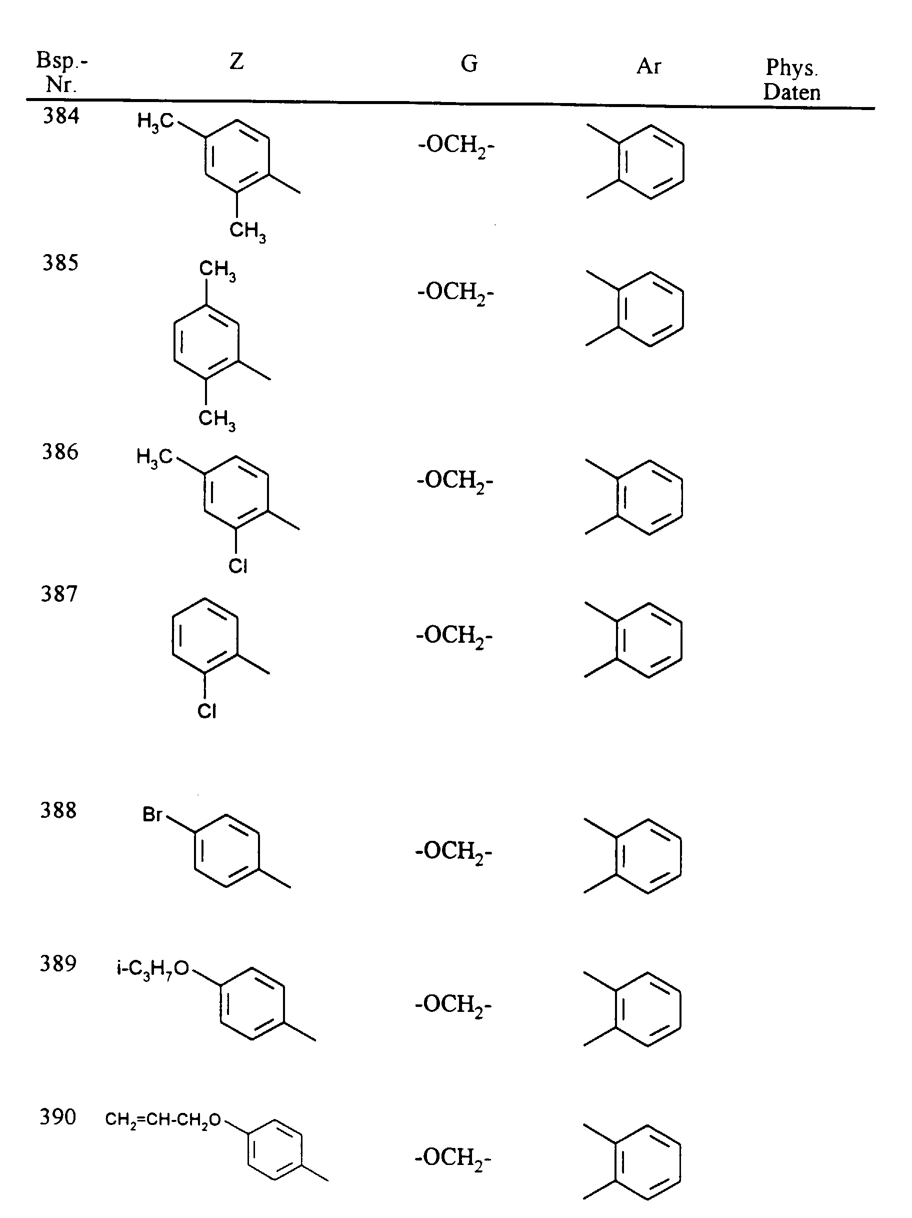

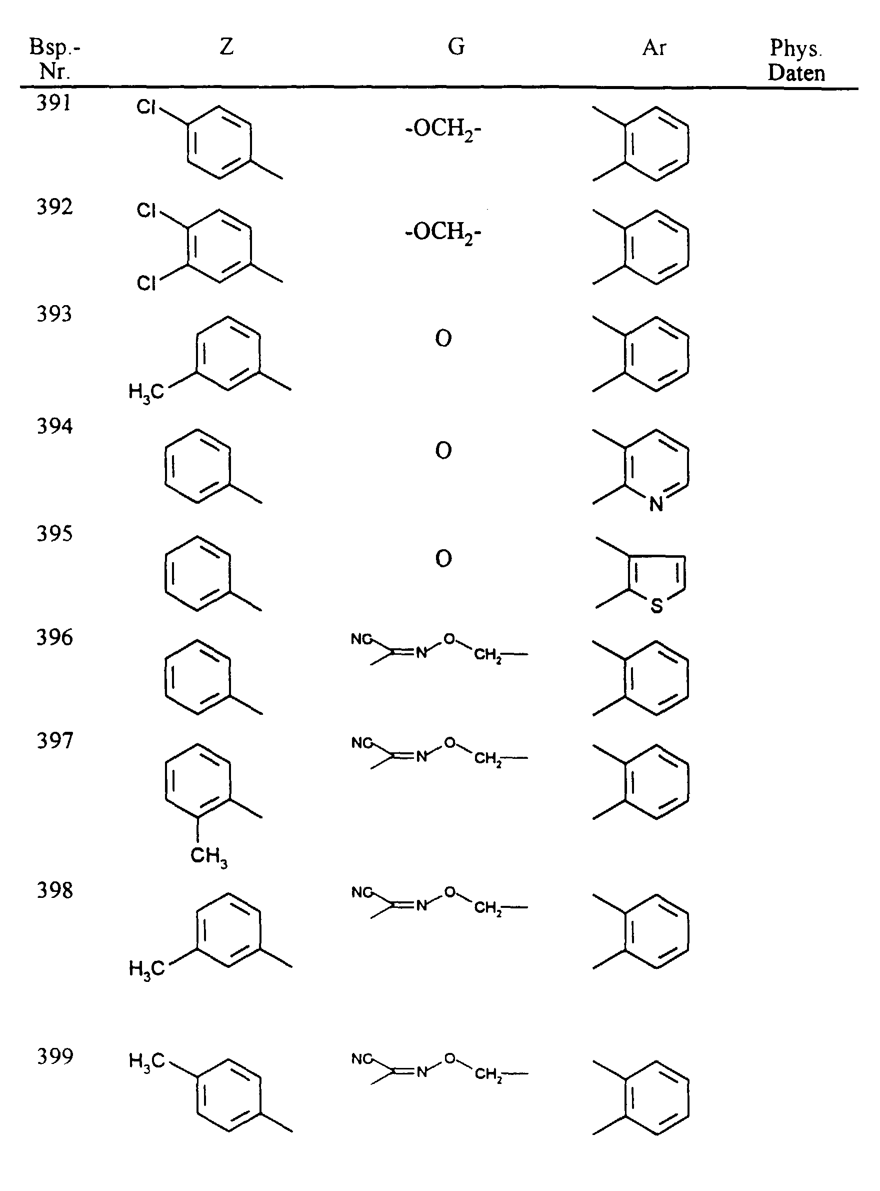

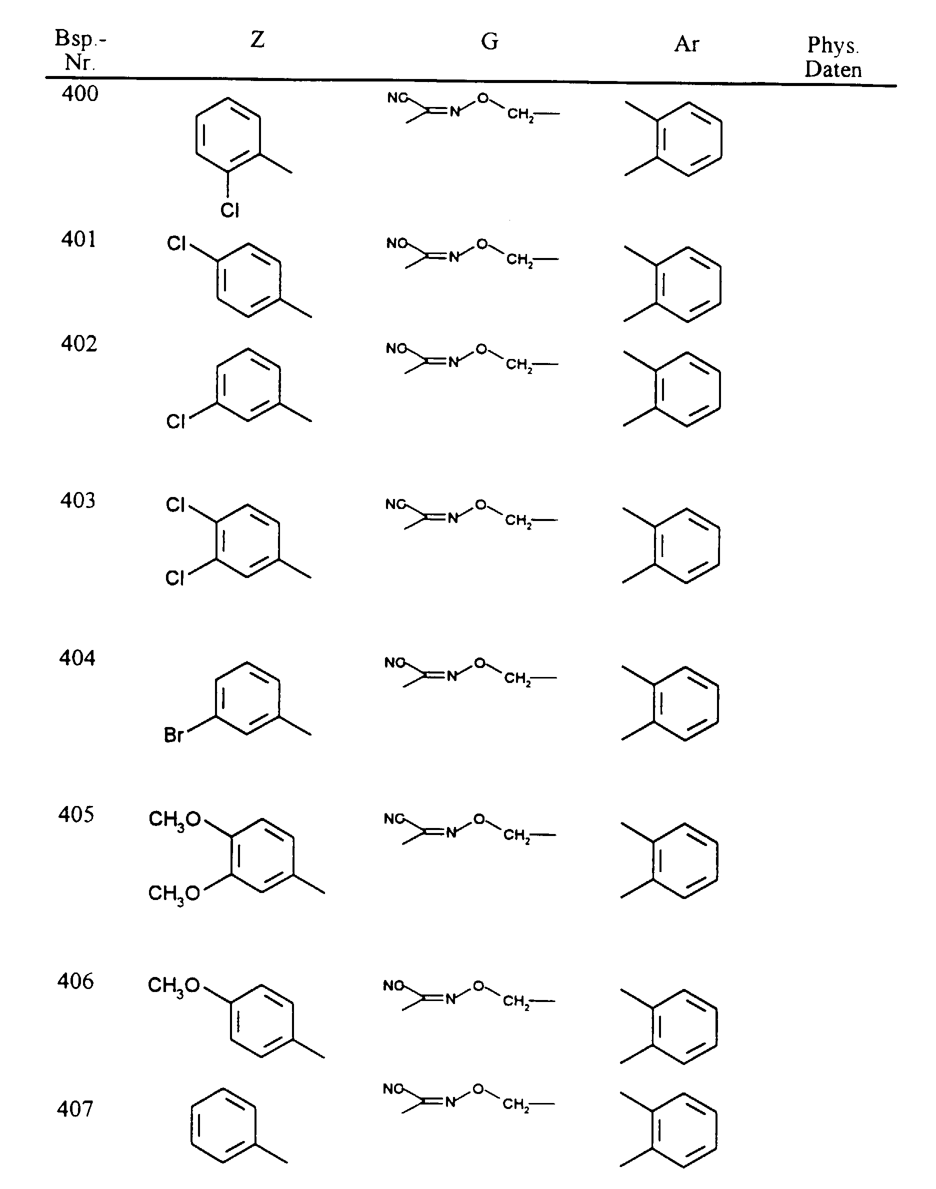

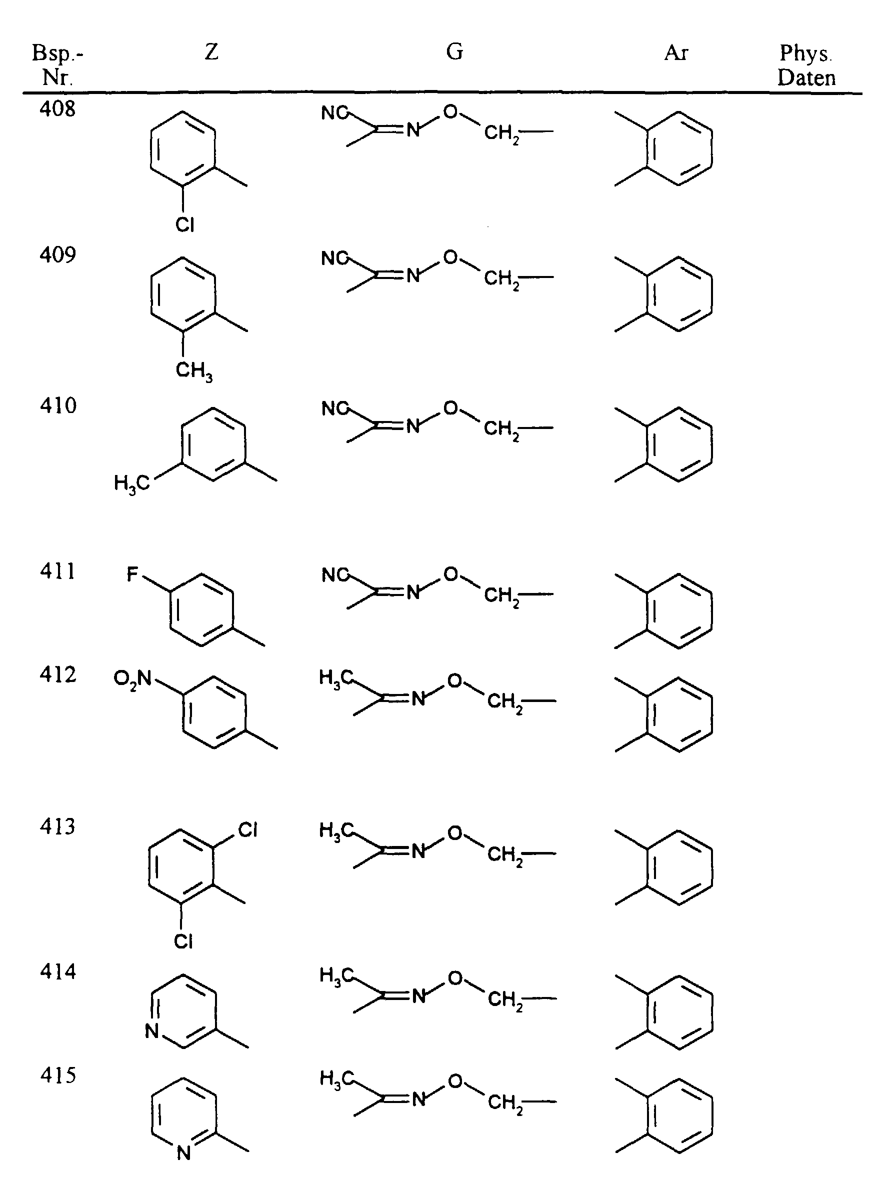

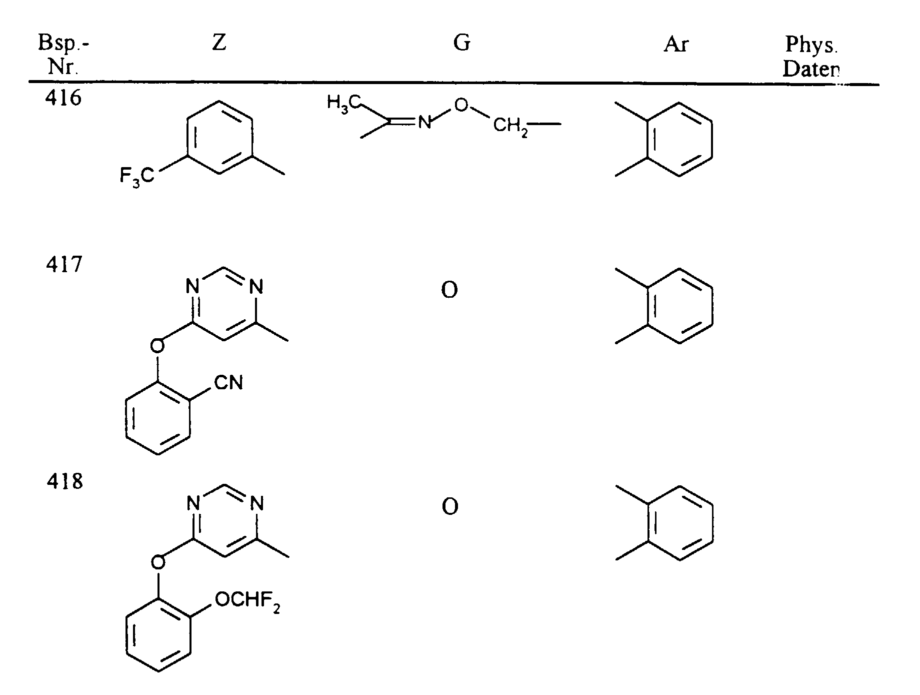

### Anwendungsbeispiele:

### Beispiel A

### Sphaerotheca-Test (Gurke) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

In diesem Test zeigt beispielsweise die Verbindung gemäß Herstellungsbeispiel 2 (E-Isomeres) sehr starke Wirkung.

### Beispiel B

### Podosphaera-Test (Apfel) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers Podosphaera leucotricha inokuliert.

Die Pflanzen werden dann im Gewächshaus bei 23°C und einer relativen Luftfeuchtigkeit von ca. 70 % aufgestellt.

10 Tage nach der Inokulation erfolgt die Auswertung.

In diesem Test zeigt beispielsweise die Verbindung gemäß Herstellungsbeispiel 2 (E-Isomeres) sehr starke Wirkung.

### Beispiel C

### Venturia-Test (Apfel) / protektiv

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers Venturia inaequalis inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

In diesem Test zeigt beispielsweise die Verbindung gemäß Herstellungsbeispiel 2 (E-Isomeres) sehr starke Wirkung.

### Beispiel D

### Erysiphe-Test (Gerste) / protektiv

- Lösungsmittel:: 10 Gew.-Teile N-Methyl-pyrrolidon
- Emulgator:: 0,6 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt beispielsweise die Verbindung gemäß Herstellungsbeispiel 2 (E-Isomeres) bei einer Wirkstoffaufwandmenge von 250 g/ha einen 100%igen Wirkungsgrad.

### Beispiel E

### Erysiphe-Test (Gerste) / kurativ

- Lösungsmittel:: 10 Gew.-Teile N-Methyl-pyrrolidon
- Emulgator:: 0,6 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt beispielsweise die Verbindung gemäß Herstellungsbeispiel 2 (E-Isomeres) bei einer Aufwandmenge von 250 g/ha einen 100%igen Wirkungsgrad.

## Patentansprüche

1. Arylessigsäurederivate der allgemeinen Formel (I), 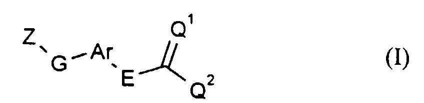 in welcher
Ar für jeweils gegebenenfalls substituiertes Arylen oder Heteroarylen steht,
E für eine 1-Alken-1,1-diyl-Gruppierung oder für eine 2-Aza-1-alken-1,1-diyl-Gruppierung steht, die jeweils in 2-Position eine Halogenalkoxy-Gruppe enthalten,
G für Sauerstoff für eine direkte Bindung, für jeweils gegebenenfalls durch Halogen, Hydroxy, Alkyl, Halogenalkyl oder Cycloalkyl substituiertes Alkandiyl, Alkendiyl, Alkindiyl oder eine der nachstehenden Gruppierungen
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R¹)=N-O-, -C(R¹)=N-O-CH₂-, -N(R²)-, -CQ-N(R²)-, -N(R²)-CQ-, -Q-CQ-N(R²)-, -N=C(R¹)-Q-CH₂-, -CH₂-O-N=C(R¹)-, -N(R²)-CQ-Q-, -CQ-N(R²)-CQ-Q- oder -N(R²)-CQ-Q-CH₂- steht,
wobei
n für die Zahlen 0, 1 oder 2 steht,
Q für Sauerstoff oder Schwefel steht,
R¹ für Wasserstoff, Cyano oder jeweils gegebenenfalls substituiertes Alkyl, Alkoxy, Alkylthio, Alkylamino, Dialkylamino oder Cycloalkyl steht, und
R² für Wasserstoff, Hydroxy, Cyano oder jeweils gegebenenfalls substituiertes Alkyl, Alkoxy oder Cycloalkyl steht,
Q¹ für Sauerstoff oder Schwefel steht,
Q² für Hydroxy, Mercapto, Amino oder für jeweils gegebenenfalls substituiertes Alkoxy, Alkylthio, Alkylamino, Alkoxyamino, Dialkylamino oder N-Alkoxy-N-alkyl-amino steht, sowie
Z für jeweils gegebenenfalls substituiertes Alkyl, Alkenyl, Alkinyl, Cycloalkyl, Aryl oder Heteroaryl steht.

2. Arylessigsäurederivate der Formel (I) gemäß Anspruch 1, in welcher
Ar für jeweils gegebenenfalls substituiertes Phenylen oder Naphthylen oder für Heteroarylen mit 5 oder 6 Ringgliedern, von denen mindestens eines für Sauerstoff, Schwefel oder Stickstoff steht und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
E für eine der nachstehenden Gruppierungen steht 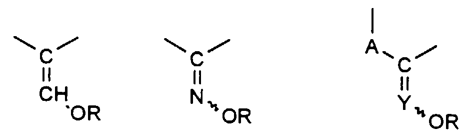 worin
R für Halogenalkyl mit 1 bis 6 Kohlenstoffatomen steht,
A für Sauerstoff, Schwefel, Imino (NH) oder Alkylimino (N-Alkyl - letzteres mit 1 bis 4 Kohlenstoffatomen) steht, und
Y für Stickstoff oder eine CH-Gruppierung steht,
G für Sauerstoff für eine direkte Bindung, für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Alkindiyl mit jeweils bis zu 4 Kohlenstoffatomen oder eine der nachstehenden Gruppierungen
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R¹)=N-O-, -C(R¹)=N-O-CH₂-, -N(R²)-, -CQ-N(R²)-, -N(R²)-CQ-, -Q-CQ-N(R²)-, -N=C(R¹)-Q-CH₂-, -CH₂-O-N=C(R¹)-, -N(R²)-CQ-Q-, -CQ-N(R²)-CQ-Q- oder -N(R²)-CQ-Q-CH₂- steht,
wobei
n für die Zahlen 0, 1 oder 2 steht,
Q für Sauerstoff oder Schwefel steht,
R¹ für Wasserstoff, Cyano, für jeweils gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht, und
R² für Wasserstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht,
Q¹ für Sauerstoff oder Schwefel steht,
Q² für Hydroxy, Mercapto, Amino oder für jeweils gegebenenfalls durch Halogen oder C₁-C₄-Alkoxy substituiertes C₁-C₆-Alkoxy, C₁-C₆-Alkylthio, C₁-C₆-Alkylamino, C₁-C₆-Alkoxyamino, Di-(C₁-C₄-alkyl)-amino oder N-(C₁-C₄-Alkoxy)-N-(C₁-C₄-alkyl)-amino steht, sowie
Z für gegebenenfalls durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sind) substituiertes Alkyl mit 1 bis 8 Kohlenstoffatomen, für jeweils gegebenenfalls durch Halogen substituiertes Alkenyl oder Alkinyl mit jeweils bis zu 8 Kohlenstoffatomen, für gegebenenfalls durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substiuiertes Cycloalkyl mit 3 bis 6 Kohlenstoffatomen, für jeweils gegebenenfalls substituiertes Phenyl, Naphthyl oder Heteroaryl mit 5 oder 6 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, Cycloalkyl mit 3 bis 6 Kohlenstoffatomen, gegebenenfalls substituiertes Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -, sowie jeweils gegebenenfalls im Phenylteil einfach oder mehrfach, gleich oder verschieden durch Halogen, Cyano und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen substituiertes Phenyl, Phenoxy, Phenoxymethyl, Benzyl, Benzyloxy, Benzylthio, Phenylethyl oder Phenylethyloxy.

3. Arylessigsäurederivate der Formel (I) gemäß Anspruch 1, in welcher
Ar für jeweils gegebenenfalls substituiertes ortho-, meta- oder para-Phenylen, für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl (insbesondere Pyridin-2,3-diyl), Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind: Fluor, Chlor, Cyano, Methyl, Ethyl, Trifluormethyl, Methoxy, Ethoxy, Methylthio, Methylsulfinyl oder Methylsulfonyl,
E für eine der nachstehenden Gruppierungen steht 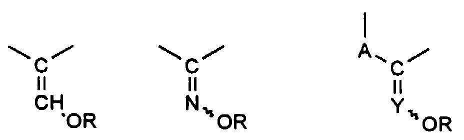 worin
R für jeweils durch Fluor und/oder Chlor substituiertes Methyl, Ethyl, n- oder i-Propyl, n-, i-, s- oder t-Butyl steht,
A für Sauerstoff, Schwefel, Imino (NH) oder Methylimino (N-Methyl) steht, und
Y für Stickstoff oder eine CH-Gruppierung steht,
G für Sauerstoff für eine direkte Bindung, für jeweils gegebenenfalls durch Fluor, Chlor, Hydroxy, Methyl, Ethyl, n- oder i-Propyl, Trifluormethyl, Cyclopropyl, Cyclobutyl, Cyclopentyl oder Cyclohexyl substituiertes Methylen, Dimethylen (Ethan-1,2-diyl), Ethen-1,2-diyl, Ethin-1,2-diyl oder eine der nachstehenden Gruppierungen
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R¹)=N-O-, -C(R¹)=N-O-CH₂-, -N(R²)-, -CQ-N(R²)-, -N(R²)-CQ-, -Q-CQ-N(R²)-, -N=C(R¹)-Q-CH₂-, -CH₂-O-N=C(R¹)-, -N(R²)-CQ-Q-, -CQ-N(R²)-CQ-Q- oder -N(R²)-CQ-Q-CH₂- steht,
wobei
n für die Zahlen 0, 1 oder 2 steht,
Q für Sauerstoff oder Schwefel steht,
R¹ für Wasserstoff, Cyano, für jeweils gegebenenfalls durch Fluor, Chlor, Cyano, Methoxy oder Ethoxy substituiertes Methyl, Ethyl, n- oder i-Propyl, n-, i- oder s-Butyl, Methoxy, Ethoxy, Propoxy, Butoxy, Methylthio, Ethylthio, Propylthio, Butylthio, Methylamino, Ethylamino, Propylamino, Dimethylamino oder Diethylamino oder für jeweils gegebenenfalls durch Fluor, Chlor, Cyano, Carboxy, Methyl, Ethyl, n- oder i-Propyl, Methoxycarbonyl oder Ethoxy-carbonyl substituiertes Cyclopropyl, Cyclobutyl, Cyclopentyl oder Cyclohexyl steht, und
R² für Wasserstoff, Hydroxy, Cyano oder für jeweils gegebenenfalls durch Fluor, Chlor, Cyano, Methoxy oder Ethoxy substituiertes Methyl, Ethyl, n- oder i-Propyl, n-, i-, s- oder t-Butyl oder für jeweils gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht,
Q¹ für Sauerstoff oder Schwefel steht,
Q² für Hydroxy, Mercapto, Amino oder für jeweils gegebenenfalls durch Fluor, Chlor, Methoxy oder Ethoxy substituiertes Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i- s- oder t-Butylamino, Methoxyamino, Ethoxyamino, n- oder i-Propoxyamino, n-, i-, s- oder t-Butoxyamino, Dimethylamino, Diethylamino, Dipropylamino, Dibutylamino oder N-Methoxy-N-methyl-amino steht, sowie
Z für jeweils gegebenenfalls durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl, für jeweils gegebenenfalls durch Fluor, Chlor oder Brom substituiertes Allyl, Crotonyl, 1-Methyl-allyl, Propargyl oder 1-Methylpropargyl, für jeweils gegebenenfalls durch Fluor, Chlor, Brom, Cyano, Carboxy, Phenyl (welches gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiert ist), Methyl, Ethyl, n- oder i-Propyl, Methoxy-carbonyl oder Ethoxy-carbonyl substiuiertes Cyclopropyl, Cyclobutyl, Cyclopentyl oder Cyclohexyl, für jeweils gegebenenfalls substituiertes Phenyl, Naphthyl, Furyl, Thienyl, Oxazolyl, Isoxazolyl, Thiazolyl, Isothiazolyl, Oxadiazolyl, Thiadiazolyl, Pyridinyl, Pyrimidinyl, Pyridazinyl, Pyrazinyl oder Triazinyl steht, wobei die möglichen Substituenten vorzugsweise aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Ethyl, n- oder i-Propyl substituiertes Trimethylen (Propan-1,3-diyl), Methylendioxy oder Ethylendioxy, Cyclopropyl, Cyclobutyl, Cyclopentyl oder Cyclohexyl, sowie jeweils gegebenenfalls im Phenylteil bzw. im Heteroarylteil, einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl, Benzyloxy, Benzylthio, Phenoxymethyl bzw. Furyl, Thienyl, Oxazolyl, Isoxazolyl, Thiazolyl, Isothiazolyl, Oxadiazolyl, Thiadiazolyl, Pyridinyl, Pyrimidinyl, Pyridazinyl, Pyrazinyl oder Triazinyl.

4. Arylessigsäurederivate der Formel (I) gemäß Anspruch 1, in welcher
Ar für ortho-Phenylen, Pyridin-2,3-diyl oder Thiophen-2,3-diyl steht,
E für eine der die nachstehenden Gruppierungen steht 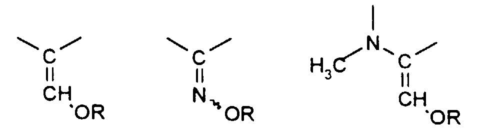 worin
R für Fluormethyl oder Difluormethyl steht,
G für Sauerstoff für eine direkte Bindung, Methylen, Ethen-1,2-diyl oder eine der nachstehenden Gruppierungen
-O-CO-, -CO-O-, -CH₂-O-, -O-CH₂-, -S(O)ₙ-, -CH₂-S(O)ₙ-, -S(O)ₙ-CH₂-, -C(R¹)=N-O-, -O-N=C(R¹)-, -C(R¹)=N-O-CH₂-, -N(R²)- oder -CH₂-O-N=C(R¹)- steht,
wobei
n für die Zahlen 0, 1 oder 2 steht,
R¹ für Wasserstoff, Methyl oder Ethyl steht und
R² für Wasserstoff, Methyl oder Ethyl steht,
Q¹ für Sauerstoff steht,
Q² für Methoxy, Ethoxy, Methylamino oder Ethylamino steht, sowie
Z für jeweils gegebenenfalls substituiertes Phenyl, Thiazolyl, Thiadiazolyl, Pyridinyl, Pyrimidinyl, Pyridazinyl, Pyrazinyl oder Triazinyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy, Benzylthio oder Phenoxymethyl, sowie ferner jeweils gegebenenfalls einfach oder mehrfach, gleich oder verschieden durch Fluor, Chlor, Brom, Methyl, Methoxy oder Trifluormethyl substituiertes Furyl, Thienyl, Pyridinyl oder Pyrimidinyl.

5. Verfahren zur Herstellung von Arylessigsäurederivaten der Formel (I) gemäß Anspruch 1, dadurch gekennzeichnet, daß man
(a) Arylglyoxylsäurederivate der allgemeinen Formel (II) 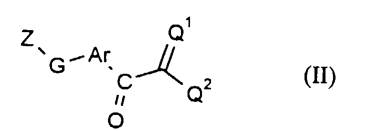 in welcher
Ar, G, Q¹, Q² und Z die oben angegebene Bedeutung haben,
mit O-Halogenalkyl-hydroxylaminen der allgemeinen Formel (III)
H₂N-O-R (III)
in welcher
R für Halogenalkyl steht,
- oder mit Hydrogenhalogenid-Addukten von Verbindungen der Formel (III) -
gegebenenfalls in Gegenwart eines Reaktionshilfsmittels und gegebenenfalls in Gegenwart eines Verdünnungsmittels umsetzt, oder wenn man
(b) 2-Hydroximino-arylessigsäurederivate der allgemeinen Formel (IV) 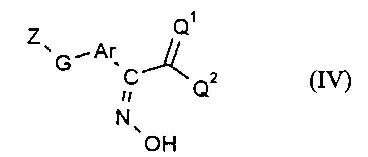 in welcher
Ar, G, Q¹, Q² und Z die oben angegebene Bedeutung haben,
mit Halogenalkanen der allgemeinen Formel (V)
X-R (V)
in welcher
R für Halogenalkyl steht und
X für Halogen steht,
gegebenenfalls in Gegenwart eines Reaktionshilfsmittels und gegebenenfalls in Gegenwart eines Verdünnungsmittels umsetzt.

6. Fungizide, gekennzeichnet durch einen Gehalt an mindestens einer Verbindung der Formel (I) gemäß Anspruch 1.

7. Verfahren zur Bekämpfung von pilzlichen Krankheiten, dadurch gekennzeichnet, daß man Verbindungen der Formel (I) gemäß Anspruch 1 auf Schadpilze und/oder deren Lebensraum einwirken läßt.

8. Verwendung von Verbindungen der Formel (I) gemäß Anspruch 1 zur Bekämpfung von pilzlichen Krankheiten.

9. Verfahren zur Herstellung von Fungiziden, dadurch gekennzeichnet, daß man Verbindungen der Formel (I) gemäß Anspruch 1 mit Streckmitteln und/oder oberflächenaktiven Mitteln vermischt.

## Claims

1. Arylacetic acid derivative of the general formula (I) 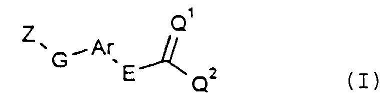 in which
Ar represents in each case optionally substituted arylene or heteroarylene,
E represents a 1-alkene-1,1-diyl group or a 2-aza-1-alkene-1,1-diyl group, each of which contains a halogenoalkoxy group in the 2-position,
G represents oxygen for a direct bond, or alkanediyl, alkenediyl or alkinediyl, each of which is optionally substituted by halogen, hydroxyl, alkyl, halogenoalkyl or cycloalkyl, or one of the following groups
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R¹)=N-O-, -C(R¹)=N-O-CH₂-, -N(R²)-, -CQ-N(R²)-, -N(R²)-CQ-, -Q-CQ-N(R²)-, -N=C(R¹)-Q-CH₂-, -CH₂-O-N=C(R¹)-, -N(R²)-CQ-Q-, -CQ-N(R²)-CQ-Q- or -N(R²)-CQ-Q-CH₂-,
where
n represents the numbers 0, 1 or 2,
Q represents oxygen or sulphur,
R¹ represents hydrogen, cyano, or in each case optionally substituted alkyl, alkoxy, alkylthio, alkylamino, dialkylamino or cycloalkyl, and
R² represents hydrogen, hydroxyl, cyano, or in each case optionally substituted alkyl, alkoxy or cycloalkyl,
Q¹ represents oxygen or sulphur,
Q² represents hydroxyl, mercapto, amino, or in each case optionally substituted alkoxy, alkylthio, alkylamino, alkoxyamino, dialkylamino or N-alkoxy-N-alkyl-amino, and
Z represents in each case optionally substituted alkyl, alkenyl, alkinyl, cycloalkyl, aryl or heteroaryl.

2. Arylacetic acid derivative of the formula (I) according to Claim 1, in which
Ar represents in each case optionally substituted phenylene or naphthylene, or represents heteroarylene having 5 or 6 ring members, of which at least one represents oxygen, sulphur or nitrogen and, if appropriate, one or two others represent nitrogen, possible substituents preferably being selected from the series which follows:
halogen, cyano, nitro, amino, hydroxyl, formyl, carboxyl, carbamoyl, thiocarbamoyl, in each case straight-chain or branched alkyl, alkoxy, alkylthio, alkylsulphinyl or alkylsulphonyl, each of which has 1 to 6 carbon atoms, in each case straight-chain or branched alkenyl, alkenyloxy or alkinyloxy, each of which has 2 to 6 carbon atoms, in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms, in each case straight-chain or branched halogenoalkenyl or halogenoalkenyloxy, each of which has 2 to 6 carbon atoms and 1 to 13 identical or different halogen atoms, in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl, each of which has 1 to 6 carbon atoms in the individual alkyl moieties, or in each case bivalent alkylene or dioxyalkylene, each of which has 1 to 6 carbon atoms and each of which is optionally monosubstituted or polysubstituted by identical or different substituents from the series consisting of halogen and/or straight-chain or branched alkyl having 1 to 4 carbon atoms and/or straight-chain or branched halogenoalkyl having 1 to 4 carbon atoms and 1 to 9 identical or different halogen atoms,
E represents one of the groups which follow 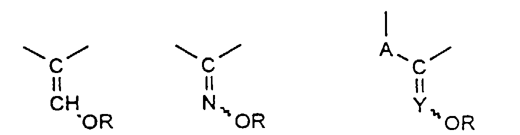 in which
R represents halogenoalkyl having 1 to 6 carbon atoms,
A represents oxygen, sulphur, imino (NH) or alkylimino (N-alkyl - the latter having 1 to 4 carbon atoms), and
Y represents nitrogen or a CH group,
G represents oxygen for a direct bond, or alkanediyl, alkenediyl or alkinediyl, each of which has up to 4 carbon atoms and each of which is optionally substituted by halgoen, hydroxyl, C₁-C₄-alkyl, C₁-C₄-halogenoalkyl or C₃-C₆-cycloalkyl, or one of the groups which follow
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R¹)=N-O-, -C(R¹)=N-O-CH₂-, -N(R²)-, -CQ-N(R²)-, -N(R²)-CQ-, -Q-CQ-N(R²)-, -N=C(R¹)-Q-CH₂-, -CH₂-O-N=C(R¹)-, -N(R²)-CQ-Q-, -CQ-N(R²)-CQ-Q- or -N(R²)-CQ-Q-CH₂-,
in which
n represents the numbers 0, 1 or 2,
Q represents oxygen or sulphur,
R¹ represents hydrogen, cyano, or alkyl, alkoxy, alkylthio, alkylamino or dialkylamino, each of which has 1 to 6 carbon atoms in the alkyl groups and is optionally substituted by halogen, cyano or C₁-C₄-alkoxy, or represents cycloalkyl having 3 to 6 carbon atoms, each of which is optionally substituted by halogen, cyano, carboxyl, C₁-C₄-alkyl or C₁-C₄-alkoxy-carbonyl, and
R² represents hydrogen, hydroxyl, cyano or alkyl having 1 to 6 carbon atoms which is optionally substituted by halogen, cyano or C₁-C₄-alkoxy, or cycloalkyl having 3 to 6 carbon atoms which is optionally substituted by halogen, cyano, carboxyl, C₁-C₄-alkyl or C₁-C₄-alkoxy-carbonyl,
Q¹ represents oxygen or sulphur,
Q² represents hydroxyl, mercapto, amino, or represents C₁-C₆-alkoxy, C₁-C₆-alkylthio, C₁-C₆-alkylamino, C₁-C₆-alkoxyamino, di-(C₁-C₄-alkyl)-amino or N-(C₁-C₄-alkoxy)-N-(C₁-C₄-alkyl)-amino, each of which is optionally substituted by halogen or C₁-C₄-alkoxy, and
Z represents alkyl having 1 to 8 carbon atoms which is optionally substituted by halogen, cyano, hydroxyl, amino, C₁-C₄-alkoxy, C₁-C₄-alkylthio, C₁-C₄-alkylsulphinyl or C₁-C₄-alkylsulphonyl (each of which is optionally substituted by halogen), or represents alkenyl or alkinyl, each of which has up to 8 carbon atoms and each of which is optionally substituted by halogen, or represents cycloalkyl having 3 to 6 carbon atoms, each of which is optionally substituted by halogen, cyano, carboxyl, phenyl (which is optionally substituted by halogen, cyano, C₁-C₄-alkyl, C₁-C₄-halogenoalkyl, C₁-C₄-alkoxy or C₁-C₄-halogenoalkoxy), C₁-C₄-alkyl or C₁-C₄-alkoxycarbonyl, or represents in each case optionally substituted phenyl, naphthyl or heteroaryl having 5 or 6 ring members, of which at least one represents oxygen, sulphur or nitrogen and, if appropriate, one or two others represent nitrogen, possible substituents preferably being selected from the series which follows: halogen, cyano, nitro, amino, hydroxyl, formyl, carboxyl, carbamoyl, thiocarbamoyl, in each case straight-chain or branched alkyl, alkoxy, alkylthio, alkylsulphinyl or alkylsulphonyl, each of which has 1 to 6 carbon atoms, in each case straight-chain or branched alkenyl or alkenyloxy, each of which has 2 to 6 carbon atoms, in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms, in each case straight-chain or branched halogenoalkenyl or halogenoalkenyloxy, each of which has 2 to 6 carbon atoms and 1 to 13 identical or different halogen atoms, in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl, each of which has 1 to 6 carbon atoms in the individual alkyl moieties, in each case bivalent alkylene or dioxyalkylene, each of which has 1 to 6 carbon atoms and each of which is optionally monosubstituted or polysubstituted by identical or different substituents from the series consisting of halogen and/or straight-chain or branched alkyl having 1 to 4 carbon atoms and/or straight-chain or branched halogenoalkyl having 1 to 4 carbon atoms and 1 to 9 identical or different halogen atoms, cycloalkyl having 3 to 6 carbon atoms, optionally substituted heterocyclyl or heterocyclyl-methyl, each of which has 3 to 7 ring members, of which in each case 1 to 3 are identical or different hetero atoms - in particular nitrogen, oxygen and/or sulphur -, and phenyl, phenoxy, phenoxymethyl, benzyl, benzyloxy, benzylthio, phenylethyl or phenylethyloxy, each of which is optionally monosubstituted or polysubstituted in the phenyl moiety by identical or different substituents from the series consisting of halogen, cyano and/or straight-chain or branched alkyl having 1 to 4 carbon atoms and/or straight-chain or branched halogenoalkyl having 1 to 4 carbon atoms and 1 to 9 identical or different halogen atoms and/or straight-chain or branched alkoxy having 1 to 4 carbon atoms and/or straight-chain or branched halogenoalkoxy having 1 to 4 carbon atoms and 1 to 9 identical or different halogen atoms.

3. Arylacetic acid derivative of the formula (I) according to Claim 1, in which
Ar represents in each case optionally substituted ortho-, meta- or para-phenylene, or represents furanediyl, thiophenediyl, pyrrolediyl, pyrazolediyl, triazolediyl, oxazolediyl, isoxazolediyl, thiazolediyl, isothiazolediyl, oxadiazolediyl, thiadiazolediyl, pyridinediyl (in particular pyridine-2,3-diyl), pyrimidinediyl, pyridazinediyl, pyrazinediyl, 1,3,4-triazinediyl or 1,2,3-triazinediyl, possible substituents being selected, in particular, from the series which follows: fluorine, chlorine, cyano, methyl, ethyl, trifluoromethyl, methoxy, ethoxy, methylthio, methylsulphinyl or methylsulphonyl,
E represents one of the groups which follows 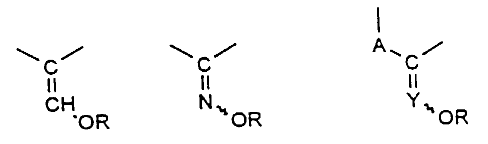 in which
R represents methyl, ethyl, n- or i-propyl or n-, i-, s- or t-butyl, each of which is substituted by fluorine and/or chlorine,
A represents oxygen, sulphur, imino (NH) or methylimino (N-methyl), and
Y represents nitrogen or a CH group,
G represents oxygen for a direct bond, or methylene, dimethylene (ethane-1,2-diyl), ethene-1,2-diyl or ethine-1,2-diyl, each of which is optionally substituted by fluorine, chlorine, hydroxyl, methyl, ethyl, n- or i-propyl, trifluoromethyl, cyclopropyl, cyclobutyl, cyclopentyl or cyclohexyl, or one of the groups which follows
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R¹)=N-O-, -C(R¹)=N-O-CH₂-, -N(R²)-, -CQ-N(R²)-, -N(R²)-CQ-, -Q-CQ-N(R²)-, -N=C(R¹)-Q-CH₂-, -CH₂-O-N=C(R¹)-, -N(R²)-CQ-Q-, -CQ-N(R²)-CQ-Q- or -N(R²)-CQ-Q-CH₂-,
in which
n represents the numbers 0, 1 or 2,
Q represents oxygen or sulphur,
R¹ represents hydrogen, cyano, or methyl, ethyl, n- or i-propyl, n-, i- or s-butyl, methoxy, ethoxy, propoxy, butoxy, methylthio, ethylthio, propylthio, butylthio, methylamino, ethylamino, propylamino, dimethylamino or diethylamino, each of which is optionally substituted by fluorine, chlorine, cyano, methoxy or ethoxy, or represents cyclopropyl, cyclobutyl, cyclopentyl or cyclohexyl, each of which is optionally substituted by fluorine, chlorine, cyano, carboxyl, methyl, ethyl, n- or i-propyl, methoxycarbonyl or ethoxycarbonyl, and
R² represents hydrogen, hydroxyl, cyano, or methyl, ethyl, n- or i-propyl or n-, i-, s- or t-butyl, each of which is optionally substituted by fluorine, chlorine, cyano, methoxy or ethoxy, or represents cyclopropyl, cyclobutyl, cyclopentyl or cyclohexyl, each of which is optionally substituted by fluorine, chlorine, cyano, carboxyl, methyl, ethyl, n- or i-propyl, methoxycarbonyl or ethoxycarbonyl,
Q¹ represents oxygen or sulphur,
Q² represents hydroxyl, mercapto, amino, or represents methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i- s- or t-butylamino, methoxyamino, ethoxyamino, n- or i-propoxyamino, n-, i-, s- or t-butoxyamino, dimethylamino, diethylamino, dipropylamino, dibutylamino or N-methoxy-N-methyl-amino, each of which is optionally substituted by fluorine, chlorine, methoxy or ethoxy, and
Z represents methyl, ethyl, n- or i-propyl or n-, i-, s- or t-butyl, each of which is optionally substituted by fluorine, chlorine, bromine, cyano, hydroxyl, amino, methoxy, ethoxy, methylthio, ethylthio, methylsulphinyl, ethylsulphinyl, methylsulphonyl or ethylsulphonyl (in each case optionally substituted by fluorine and/or chlorine), or represents allyl, crotonyl, 1-methyl-allyl, propargyl or 1-methyl-propargyl, each of which is optionally substituted by fluorine, chlorine or bromine, or represents cyclopropyl, cyclobutyl, cyclopentyl or cyclohexyl, each of which is optionally substituted by fluorine, chlorine, bromine, cyano, carboxyl, phenyl (which is optionally substituted by fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy), methyl, ethyl, n- or i-propyl, methoxycarbonyl or ethoxycarbonyl, or represents in each case optionally substituted phenyl, naphthyl, furyl, thienyl, oxazolyl, isoxazolyl, thiazolyl, isothiazolyl, oxadiazolyl, thiadiazolyl, pyridinyl, pyrimidinyl, pyridazinyl, pyrazinyl or triazinyl, possible substituents preferably being selected from the series which follows:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluoromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl; trimethylene (propane-1,3-diyl), methylenedioxy or ethylenedioxy, each of which is optionally monosubstituted or polysubstituted by identical or different substituents from the series consisting of fluorine, chlorine, methyl, ethyl or n- or i-propyl, or cyclopropyl, cyclobutyl, cyclopentyl or cyclohexyl, or phenyl, phenoxy, benzyl, benzyloxy, benzylthio, phenoxymethyl or furyl, thienyl, oxazolyl, isoxazolyl, thiazolyl, isothiazolyl, oxadiazolyl, thiadiazolyl, pyridinyl, pyrimidinyl, pyridazinyl, pyrazinyl or triazinyl each of which is optionally monosubstituted or polysubstituted in the phenyl moiety or in the heteroaryl moiety by identical or different substituents from the series consisting of fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy.

4. Arylacetic acid derivative of the formula (I) according to Claim 1, in which
Ar represents ortho-phenylene, pyridine-2,3-diyl or thiophene-2,3-diyl,
E represents one of the groups which follow 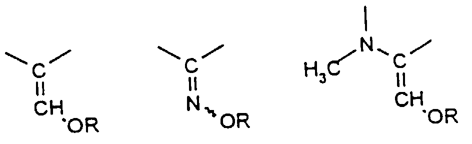 in which
R represents fluoromethyl or difluoromethyl,
G represents oxygen for a direct bond, methylene, ethene-1,2-diyl or one of the groups which follow
-O-CO-, -CO-O-, -CH₂-O-, -O-CH₂-, -S(O)ₙ-, -CH₂-S(O)ₙ-, -S(O)ₙ-CH₂-, -C(R¹)=N-O-, -O-N=C(R¹)-, -C(R¹)=N-O-CH₂-, -N(R²)- or -CH₂-O-N=C(R¹)-,
in which
n represents the numbers 0, 1 or 2,
R¹ represents hydrogen, methyl or ethyl, and
R² represents hydrogen, methyl or ethyl,
Q¹ represents oxygen,
Q² represents methoxy, ethoxy, methylamino or ethylamino, and
Z represents in each case optionally substituted phenyl, thiazolyl, thiadiazolyl, pyridinyl, pyrimidinyl, pyridazinyl, pyrazinyl or triazinyl, possible substituents preferably being selected from the series which follows:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluoromethylthio, trifluoromethylthio, trifluoromethylsulphinyl or trifluoromethylsulphonyl, methoxycarbonyl, ethoxycarbonyl, methoximinomethyl, ethoximinomethyl, methoximinoethyl, ethoximinoethyl, or methylenedioxy or ethylenedioxy, each of which is optionally monosubstituted or polysubstituted by identical or different substituents from the series consisting of fluorine, chlorine, methyl or ethyl, or phenyl, phenoxy, benzyl or benzyloxy, benzylthio or phenoxymethyl, each of which is optionally monosubstituted or polysubstituted in the phenyl moiety by identical or different substituents from the series consisting of fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy, and furthermore also furyl, thienyl, pyridinyl or pyrimidinyl, each of which is optionally monosubstituted or polysubstituted by identical or different substituents from the series consisting of fluorine, chlorine, bromine, methyl, methoxy or trifluoromethyl.

5. Process for the preparation of arylacetic acid derivatives of the formula (I) according to Claim 1, characterized in that
(a) arylglyoxylic acid derivatives of the general formula (II) 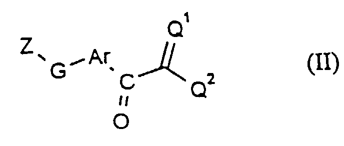 in which
Ar, G, Q¹, Q² and Z have the abovementioned meaning,
are reacted with O-halogenoalkyl-hydroxylamines of the general formula (III)
H₂N-O-R (III)
in which
R represents halogenoalkyl,
- or with hydrogen halide adducts of compounds of the formula (III) -,
if appropriate in the presence of a reaction auxiliary and if appropriate in the presence of a diluent, or when
(b) 2-hydroximino-arylacetic acid derivatives of the general formula (IV) 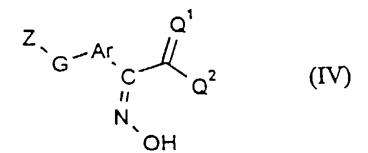 in which
Ar, G, Q¹, Q² and Z have the abovementioned meaning,
are reacted with halogenoalkanes of the general formula (V)
X-R (V)
in which
R represents halogenoalkyl and
X represents halogen,
if appropriate in the presence of a reaction auxiliary and if appropriate in the presence of a diluent.

6. Fungicide characterized in that it contains at least one compound of the formula (I) according to Claim 1.

7. Method of combating fungal diseases, characterized in that compounds of the formula (I) according to Claim 1 are allowed to act on fungal pests and/or their environment.

8. Use of a compound of the formula (I) according to Claim 1 for combating fungal diseases.

9. Process for the preparation of fungicides, characterized in that compounds of the formula (I) according to Claim 1 are mixed with extenders and/or surface-active agents.

## Revendications

1. Dérivés d'acides arylacétiques de formule générale (I), 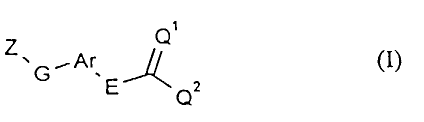 dans laquelle
Ar désigne des groupes arylène ou hétéroarylène éventuellement substitués,
E est un groupement 1-alcène-1,1-diyle ou un groupement 2-aza-1-alcène-1,1-diyle, contenant chacun en position 2 un groupe halogénalkoxy,
G représente de l'oxygène, une liaison directe, un groupe alcanediyle, alcènediyle, alcynediyle dont chacun est éventuellement substitué par un radical halogéno, hydroxy, alkyle, halogénalkyle ou cycloalkyle, ou l'un des groupements suivants :
-Q-CQ-, -CQ-Q-, -CH₂-Q-; -Q-CH₂-, -CQ-Q-CH₂-,-CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R¹)=N-O-, -C(R¹)=N-O-CH₂-, -N(R²)-, -CQ-N(R²)-, -N(R²)-CQ-, -Q-CQ-N(R²)-, -N=C(R¹)-Q-CH₂-, -CH₂-O-N=C(R¹)-, -N(R²)-CQ-Q-, -CQ-N(R²)-CQ-Q- ou -N(R²)-CQ-Q-CH₂-
où
n représente les nombres 0, 1 ou 2,
Q est de l'oxygène ou du soufre,
R¹ est de l'hydrogène, un groupe cyano ou un groupe alkyle, alkoxy, alkylthio, alkylamino, dialkylamino ou cycloalkyle, dont chacun est éventuellement substitué, et
R² est de l'hydrogène, un groupe hydroxy, cyano, ou un groupe alkyle, alkoxy ou cycloalkyle dont chacun est éventuellement substitué,
Q¹ est de l'oxygène ou du soufre,
Q² est un groupe hydroxy, mercapto, amino ou un groupe alkoxy, alkylthio, alkylamino, alkoxyamino, dialkylamino ou N-alkoxy-N-alkylamino dont chacun est éventuellement substitué, de même que
Z représente un groupe alkyle, alcényle, alcynyle, cycloalkyle, aryle ou hétéroaryle éventuellement substitué.

2. Dérivés d'acides arylacétiques de formule (I) suivant la revendication 1, dans laquelle
Ar représente un groupe phénylène ou un groupe naphtylène dont chacun est éventuellement substitué ou un groupe hétéroarylène à noyau de 5 ou 6 chaînons, dont au moins l'un représente de l'oxygène, du soufre ou de l'azote et, le cas échéant, un ou deux autres représentent de l'azote, les substituants possibles étant choisis de préférence parmi les substituants énumérés ci-après :
halogène, cyano, nitro, amino, hydroxy, formyle, carboxy, carbamoyle, thiocarbamoyle, groupes linéaires ou ramifiés alkyle, alkoxy, alkylthio, alkylsulfinyle ou alkylsulfonyle ayant chacun 1 à 6 atomes de carbone, groupes linéaires ou ramifiés alcényle, alcényloxy ou alcynyloxy ayant chacun 2 à 6 atomes de carbone, groupes linaires ou ramifiés halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents, groupes linéaires ou ramifiés halogénalcényle ou halogénalcényloxy ayant chacun 2 à 6 atomes de carbone et 1 à 13 atomes d'halogènes identiques ou différents, groupes linéaires ou ramifiés alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles, groupes alkylène ou dioxyalkylène ayant chacun 1 à 6 atomes de carbone, attachés chacun par deux liaisons et portant chacun, le cas échéant, un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents,
E représente les groupements suivants 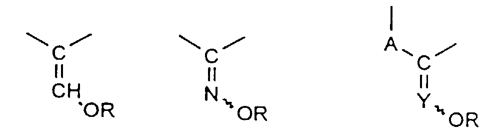 dans lesquels
R est un groupe halogénalkyle ayant 1 à 6 atomes de carbone,
A est de l'oxygène, du soufre, un groupe imino (NH) ou alkylimino (N-alkyle à groupe alkyle ayant 1 à 4 atomes de carbone), et
Y est de l'oxygène ou un groupement CH,
G représente de l'oxygène, une liaison directe, des groupes alcanediyle, alcènediyle, alcynediyle ayant chacun jusqu'à 4 atomes de crabone et substitués chacun par un radical halogéno, hydroxy, alkyle en C₁ à C₄, halogénalkyle en C₁ à C₄ ou cycloalkyle en C₃ à C₆, ou l'un des groupements suivants :
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R¹)=N-O-, -C(R¹)=N-O-CH₂-, -N(R²)-, -CQ-N(R²)-, -N(R²)-CQ-, -Q-CQ-N(R²)-, -N=C(R¹)-Q-CH₂-, -CH₂-O-N=C(R¹)-, -N(R²)-CQ-Q-, -CQ-N(R²)-CQ-Q- ou -N(R²)-CQ-Q-CH₂-
où
n représente les nombres 0, 1 ou 2,
Q est de l'oxygène ou du soufre,
R¹ est de l'hydrogène, un groupe cyano, un groupe alkyle, alkoxy, alkylthio, alkylamino ou dialkylamino, dont chacun a 1 à 6 atomes de carbone dans les parties alkyle et chacun est substitué, le cas échéant par un radical halogéno, cyano ou alkoxy en C₁ à C₄, ou un groupe cycloalkyle de 3 à 6 atomes de carbone éventuellement substitué par un radical halogéno, cyano, carboxy, alkyle en C₁ à C₄ ou alkoxy en C₁ à C₄, et
R² est de l'hydrogène, un groupe hydroxy, cyano, ou un groupe alkyle de 1 à 6 atomes de carbone éventuellement substitué par un radical halogéno, cyano ou alkoxy en C₁ à C₄ ou un groupe cycloalkyle de 3 à 6 atomes de carbone éventuellement substitué par un radical halogéno, cyano, carboxy, alkyle en C₁ à C₄ ou (alkoxy en C₁ à C₄)-carbonyle,
Q¹ est de l'oxygène ou du soufre,
Q² est un groupe hydroxy, mercapto, amino ou un groupe alkoxy en C₁ à C₆, alkylthio en C₁ à C₆, alkylamino en C₁ à C₆, alkoxyamino en C₁ à C₆, di(alkyle en C₁ à C₄)amino ou N-(alkoxy en C₁ à C₄)-N-(alkyle en C₁ à C₄)amino portant chacun, le cas échéant, un substituant halogéno ou alkoxy en C₁ à C₄, de même que
Z représente un groupe alkyle ayant 1 à 8 atomes de carbone éventuellement substitué par un radical halogéno, cyano, hydroxy, amino, alkoxy en C₁ à C₄, alkylthio en C₁ à C₄, alkylsulfinyle en C₁ à C₄ ou alkylsulfonyle en C₁ à C₄ (chacun étant substitué le cas échéant par un halogène), un groupe alcényle ou alcynyle ayant chacun jusqu'à 8 atomes de carbone et portant chacun, le cas échéant, un substituant halogéno, un groupe cycloalkyle ayant 3 à 6 atomes de carbone éventuellement substitué par un radical halogéno, cyano, carboxy, phényle (qui est substitué le cas échéant par un radical halogéno, cyano, alkyle en C₁ à C₄, halogénalkyle en C₁ à C₄, alkoxy en C₁ à C₄ ou halogénalkoxy en C₁ à C₄), alkyle en C₁ à C₄ ou (alkoxy en C₁ à C₄)-carbonyle, un groupe éventuellement substitué phényle, naphtyle ou hétéroaryle à noyau de 5 ou 6 chaînons, dont l'un au moins est de l'oxygène, du soufre ou de l'azote et, le cas échéant, un ou deux autres représentent de l'azote, les substituants possibles étant choisis de préférence parmi ceux qui sont énumérés ci-après :
halogène, cyano, nitro, amino, hydroxy, formyle, carboxy, carbamoyle, thiocarbamoyle, groupe linéaire ou ramifié alkyle, alkoxy, alkylthio, alkylsulfinyle ou alkylsulfonyle ayant dans chaque cas 1 à 6 atomes de carbone, groupe linéaire ou ramifié alcényle ou alcényloxy ayant chacun 2 à 6 atomes de carbone, groupe linéaire ou ramifié halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents, groupe linéaire ou ramifié halogénalcényle ou halogénalcényloxy ayant chacun 2 à 6 atomes de carbone et 1 à 13 atomes d'halogènes identiques ou différents, groupe linéaire ou ramifié alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles, groupe alkylène ou dioxyalkylène ayant chacun 1 à 6 atomes de carbone, attaché chacun par deux liaisons et étant substitué chacun, le cas échéant, une ou plusieurs fois identiques ou différentes par un halogène et/ou un radical alkyle linéaire ou ramifié ayant 1 à 4 atomes de carbone et/ou un radical halogénalkyle linéaire ou ramifié ayant 1 à 4 atomes de carbone et 1 à 9 atomes d'halogènes identiques ou différents, cycloalkyle ayant 3 à 6 atomes de carbone, hétérocyclyle ou hétérocyclyl-méthyle éventuellement substitué ayant chacun un noyau de 3 à 7 chaînons, dont 1 à 3, dans chaque cas, sont des hétéroatomes identiques ou différents - notamment azote, oxygène et/ou soufre -, de même que phényle, phénoxy, phénoxyméthyle, benzyle, benzyloxy, benzylthio, phényléthyle ou phényléthyloxy portant chacun, le cas échéant, dans la partie phényle un ou plusieurs substituants, identiques ou différents, halogéno, cyano et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents et/ou alkoxy linéaire ou ramifié ayant 1 à 4 atomes de carbone et/ou halogénalkoxy linéaire ou ramifié ayant 1 à 4 atomes de carbone et 1 à 9 atomes d'halogènes identiques ou différents.

3. Dérivés d'acides arylacétiques de formule (I) suivant la revendication 1, dans laquelle
Ar désigne un groupe ortho-, méta- ou paraphénylène éventuellement substitué, un groupe furannediyle, thiophènediyle, pyrrolediyle, pyrazolediyle, triazolediyle, oxazolediyle, isoxazolediyle, thiazolediyle, isothiazolediyle, oxadiazolediyle, thiadiazolediyle, pyridinediyle (notamment pyridine-2,3-diyle), pyrimidinediyle, pyridazinediyle, pyrazinediyle, 1,3,4-triazinediyle ou 1,2,3-triazinediyle, les substituants possibles étant choisis notamment parmi ceux qui sont énumérés ci-après : fluor, chlore, cyano, méthyle, éthyle, trifluorométhyle, méthoxy, éthoxy, méthylthio, méthylsulfinyle ou méthylsulfonyle,
E représente l'un des groupements suivants : 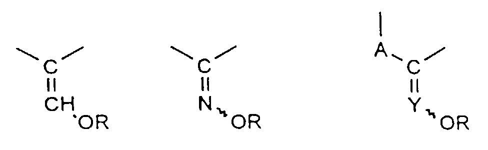 où
R représente un groupe méthyle, éthyle, n-propyle, isopropyle, n-butyle, isobutyle, sec.-butyle ou tertio-butyle dont chacun est éventuellement substitué par du fluor et/ou du chlore,
A est de l'oxygène, du soufre, un groupe imino (NH) ou méthylimino (N-méthyle), et
Y est de l'azote ou un groupement CH,
G est de l'oxygène, une liaison directe, un groupe méthylène, diméthylène (éthane-1,2-diyle), éthène-1,2-diyle, éthyne-2-diyle portant chacun, le cas échéant, un substituant fluoro, chloro, hydroxy, méthyle, éthyle, n-propyle, isopropyle, trifluorométhyle, cyclopropyle, cyclobutyle, cyclopentyle ou cyclohexyle, ou l'un des groupements suivants :
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R¹)=N-O-, -C(R¹)=N-O-CH₂-, -N(R²)-, -CQ-N(R²)-, -N(R²)-CQ-, -Q-CQ-N(R²)-, -N=C(R¹)-Q-CH₂-, -CH₂-O-N=C(R¹)-, -N(R²)-CQ-Q-, -CQ-N(R²)-CQ-Q- ou -N(R²)-CQ-Q-CH₂-
où
n représente les nombres 0, 1 ou 2,
Q est de l'oxygène ou du soufre,
R¹ est de l'hydrogène, un groupe cyano, un groupe méthyle, éthyle, n-propyle, isopropyle, n-butyle, isobutyle, sec.-butyle, méthoxy, éthoxy, propoxy, butoxy, méthylthio, éthylthio, propylthio, butylthio, méthylamino, éthylamino, propylamino, diméthylamino ou diéthylamino portant chacun, le cas échéant, un substituant fluoro, chloro, cyano, méthoxy ou éthoxy, ou un groupe cyclopropyle, cyclobutyle, cyclopentyle ou cyclohexyle portant chacun, le cas échéant, un substituant fluoro, chloro, cyano, carboxy, méthyle, éthyle, n-propyle, isopropyle, méthoxycarbonyle ou éthoxycarbonyle, et
R² est de l'hydrogène, un groupe hydroxy, cyano ou un groupe méthyle, éthyle, n-propyle, isopropyle, n-butyle, isobutyle, sec.-butyle ou tertio-butyle portant chacun, le cas échéant, un substituant fluoro, chloro, cyano, méthoxy ou éthoxy, ou un groupe cyclopropyle, cyclobutyle, cyclopentyle ou cyclohexyle portant chacun, le cas échéant, un substituant fluoro, chloro, cyano, carboxy, méthyle, éthyle, n-propyle, isopropyle, méthoxycarbonyle ou éthoxycarbonyle,
Q¹ est de l'oxygène ou du soufre,
Q² est un groupe hydroxy, mercapto, amino ou un groupe méthoxy, éthoxy, n-propoxy, isopropoxy, n-butoxy, isobutoxy, sec.-butoxy, tertio-butoxy, méthylthio, éthylthio, n-propylthio, isopropylthio, n-butylthio, isobutylthio, sec.-butylthio, tertio-butylthio, méthylamino, éthylamino, n-propylamino, isopropylamino, n-butylamino, isobutylamino, sec.-butylamino, tertio-butylamino, méthoxyamino, éthoxyamino, n-propoxyamino, isopropoxyamino, n-butoxyamino, isobutoxyamino, sec.-butoxyamino, tertio-butoxyamino, diméthylamino, diéthylamino, dipropylamino, dibutylamino ou N-méthoxy-N-méthylamino dont chacun est substitué, le cas échéant, par du fluor, du chlore, un radical méthoxy ou éthoxy, de même que
Z représente un groupe méthyle, éthyle, n-propyle, isopropyle, n-butyle, isobutyle, sec.-butyle ou tertio-butyle dont chacun est substitué le cas échéant par du fluor, du chlore, du brome, un radical cyano, hydroxy, amino, méthoxy, éthoxy, méthylthio, éthylthio, méthylsulfinyle, éthylsulfinyle, méthylsulfonyle, éthylsulfonyle (chacun de ces radicaux étant substitué le cas échéant par du fluor et/ou du chlore),
un groupe allyle, crotonyle, 1-méthylallyle, propargyle ou 1-méthylpropargyle, dont chacun est substitué le cas échéant par du fluor, du chlore ou du brome, un groupe cyclopropyle, cyclobutyle, cyclopentyle ou cyclohexyle dont chacun est substitué le cas échéant par du fluor, du chlore, du brome, un radical cyano, carboxy, phényle (substitué, le cas échéant par du fluor, du chlore, du brome, un radical cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy), méthyle, éthyle, n-propyle, isopropyle, méthoxycarbonyle ou éthoxycarbonyle, un groupe phényle, naphtyle, furyle, thiényle, oxazolyle, isoxazolyle, thiazolyle, isothiazolyle, oxadiazolyle, thiadiazolyle, pyridinyle, pyrimidinyle, pyridazinyle, pyrazinyle ou triazinyle dont chacun est éventuellement substitué, les substituants possibles étant de préférence choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, trifluorométhylsulfinyle ou trifluorométhylsulfonyle, méthylthio, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle ou éthoximinoéthyle ; triméthylène (propane-1,3-diyle), méthylènedioxy ou éthylènedioxy, cyclopropyle, cyclobutyle, cyclopentyle ou cyclohexyle portant chacun, le cas échéant, un ou plusieurs substituants, identiques ou différents, fluoro, chloro, méthyle, éthyle, n-propyle ou isopropyle, ainsi que, le cas échéant, phényle, phénoxy, benzyle, benzyloxy, benzylthio, phénoxyméthyle ou furyle, thiényle, oxazolyle, isoxazolyle, thiazolyle, isothiazolyle, oxadiazolyle, thiadiazolyle, pyridinyle, pyrimidinyle, pyridazinyle, pyrazinyle ou triazinyle dont chacun porte, le cas échéant, dans la partie phényle ou dans la partie hétéroaryle un ou plusieurs substituants, identiques ou différents, fluoro, chloro, bromo, cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy.

4. Dérivés d'acides arylacétiques de formule (I) suivant la revendication 1, dans laquelle
Ar est un groupe ortho-phénylène, pyridine-2,3-diyle ou thiophène-2,3-diyle,
E est l'un des groupements suivants : 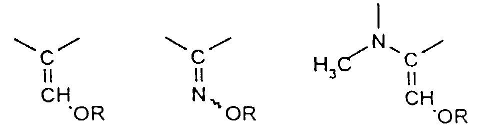 où
R est un groupe fluorométhyle ou difluorométhyle,
G est de l'oxygène, une liaison directe, un groupe méthylène, éthène-1,2-diyle ou l'un des groupements suivants :
-O-CO-, -CO-O-, -CH₂-O-, -O-CH₂-, -S(O)ₙ-, -CH₂-S(O)ₙ-, -S(O)ₙ-CH₂-, -C(R¹)=N-O-, -O-N=C(R¹)-, -C(R¹)=N-O-CH₂-, -N(R²)- ou -CH₂-O-N=C(R¹)-
où
n représente les nombres 0, 1 ou 2,
R¹ est de l'hydrogène, un groupe méthyle ou éthyle et
R² est de l'hydrogène, un groupe méthyle ou éthyle,
Q¹ est de l'oxygène,
Q² est un groupe méthoxy, éthoxy, méthylamino ou éthylamino, de même que
Z représente un groupe phényle, thiazolyle, thiadiazolyle, pyridinyle, pyrimidinyle, pyridazinyle, pyrazinyle ou triazinyle dont chacun est éventuellement substitué, les substituants possibles étant choisis de préférence parmi ceux qui sont énumérés ci-après :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, trifluorométhylsulfinyle, trifluorométhylsulfonyle, méthoxycarbonyle, éthoxycarbonyle, méthoximinométhyle, éthoximinométhyle, méthoximinoéthyle, éthoximinoéthyle ; méthylènedioxy ou éthylènedioxy portant chacun, le cas échéant, un ou plusieurs substituants, identiques ou différents, fluoro, chloro, méthyle ou éthyle ; ainsi que phényle, phénoxy, benzyle ou benzyloxy, benzylthio ou phénoxyméthyle portant chacun, le cas échéant, dans la partie phényle un ou plusieurs substituants, identiques ou différents, fluoro, chloro, bromo, cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy, de même que, en outre, furyle, thiényle, pyridinyle ou pyrimidinyle portant chacun, le cas échéant, un ou plusieurs substituants, identiques ou différents, fluoro, chloro, bromo, méthyle, méthoxy ou trifluorométhyle.

5. Procédé de production de dérivés d'esters arylacétiques de formule (I) suivant la revendication 1, caractérisé en ce que
(a) on fait réagir des dérivés d'acide arylglyoxylique de formule générale (II) 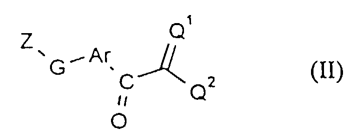 dans laquelle
Ar, G, Q¹, Q² et Z ont la définition indiquée ci-dessus, avec des O-halogénalkylhydroxylamines de formule générale (III)
H₂N-O-R (III)
dans laquelle
R est un groupe halogénalkyle,
- ou avec des produits d'addition d'halogénures d'hydrogène de composés de formule (III) -
le cas échéant en présence d'une substance auxiliaire de réaction et en la présence éventuelle d'un diluant, ou bien
(b) on fait réagir des dérivés d'acides 2-hydroximinoarylacétiques de formule générale (IV) 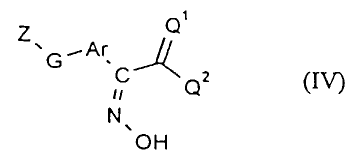 dans laquelle
Ar, G, Q¹, Q² et Z ont la définition indiquée ci-dessus, avec des halogénalcanes de formule générale (V)
X-R (V)
dans laquelle
R est un groupe halogénalkyle et
X est un halogène,
le cas échéant en présence d'une substance auxiliaire de réaction et en la présence éventuelle d'un diluant.

6. Fongicides, caractérisés par une teneur en au moins un composé de formule (I) suivant la revendication 1.

7. Procédé pour combattre des maladies dues à des champignons, caractérisé en ce qu'on fait agir des composés de formule (I) suivant la revendication 1 sur les champignons parasites et/ou sur leur milieu.

8. Utilisation de composés de formule (I) suivant la revendication 1 pour combattre des maladies dues à des champignons.

9. Procédé de préparation de compositions fongicides, caractérisé en ce qu'on mélange des composés de formule (I) suivant la revendication 1 avec des diluants et/ou des agents tensioactifs.
